# EUROPEAN PATENT APPLICATION

(11) **EP 4 636 087 A1**
(43) Date of publication of application: **22.10.2025**
(21) Application number: 23894581.0
(22) Date of filing: 21.11.2023
(51) Int. Cl.: C12N 15/13, A61K 39/395, A61P 1/00, A61P 1/04, C07K 16/18, C12N 1/15, C12N 1/19, C12N 1/21, C12N 5/10, C12N 15/63

(54) **ANTI-GPA33 ANTIBODY AND UTILIZATION THEREOF**

(30) Priority: 22.11.2022 JP 2022186944
(71) Applicant: Kagoshima University, Kagoshima-shi, Kagoshima 890-8580 (JP); National University Corporation Tottori University, Tottori-shi, Tottori 680-8550 (JP); Tokyo University of Pharmacy & Life Sciences, Hachioji-shi, Tokyo 192-0392 (JP)
(72) Inventor: ITO, Yuji, Kagoshima-shi, Kagoshima 890-8580 (JP); KAZUKI, Yasuhiro, Yonago-shi, Tottori 683-8503 (JP); TOMIZUKA, Kazuma, Hachioji-shi, Tokyo 192-0392 (JP)
(74) Representative: Vossius & Partner Patentanwälte Rechtsanwälte mbB
(86) International application number: PCT/JP2023/041789
(87) International publication number: WO 2024/111576

(57) **Abstract**

The present invention provides a human antibody or an antigen-binding portion thereof that specifically binds to human GPA33 and mouse GPA33.

## Description

### [Technical Field]

The present invention relates to a novel anti-GPA33 antibody and utilization thereof.

### [Background Art]

Inflammatory bowel disease is an intractable disease of unknown causes, and the number of patients is rapidly increasing especially in Asian area including Japan.

The main method for treating inflammatory bowel disease has conventionally been an approach based on non-specific immunosuppression, such as corticosteroids, thiopurine preparations, and calcineurin inhibitors. In 1993, the effectiveness of anti-TNFα antibody preparations was reported for the first time in the world in patients with Crohn's disease. However, these immunosuppressants reduce systemic immune function in patients, and infectious diseases as a side effect have become a major problem.

On the other hand, an antibody-drug conjugate (ADC) is attracting attention as means for selectively delivering low-molecular-weight compounds and proteins as payloads to target cells and target tissues, and attracting attention as one of the effective options in the treatment approach for diseases centering on cancer.

GPA33 is under investigation as a target for cancer treatment because its significant expression has been confirmed in certain tumors (95% of colon cancer (including both primary site and metastatic site), about half (58%) of gastric cancer, half (50%) of pancreatic cancer, and so on) (Non Patent Literature 1). In fact, a bispecific antibody against GPA33 and CD3 has been reported as an anti-cancer drug that utilizes GPA33 (Non Patent Literature 2).

As described, GPA33 has been investigated as a target for cancer treatment. However, there have been no reports to date relating to an anti-GPA33 antibody aiming at selective drug delivery to intestinal tissues, targeting intestinal diseases other than cancer.

### [Citation List]

### [Non Patent Literature]

[Non Patent Literature 1]
   Murer et al., MAbs. 2020 Jan-Dec; 12(1):1714371
[Non Patent Literature 2]
   Moore et al., Mol Cancer Ther. 2018 Aug; 17(8):1761-1772.

### [Summary of Invention]

### [Technical Problem]

The present invention aims to provide a novel therapeutic agent for bowel diseases.

### [Solution to Problem]

In order to solve the aforementioned problems, the present inventors attempted to bind an agent to the antibody that migrates intestine-specifically, thereby allowing the agent to act locally. For this purpose, they searched for antibodies superior in migration to the intestine, and found that antibodies against GPA33 are extremely superior in migration to the intestine. Furthermore, in order to promote the development of antibody drugs, the present inventors searched for human antibodies that specifically bind to both human GPA33 and mouse GPA33. Concretely, the present inventors succeeded in isolating a human antibody that specifically binds to both human and mouse GPA33 (human anti-human/mouse GPA33 antibody) from a human single-chain Fv antibody library constructed based on the mRNA of B cells derived from the spleen of a complete human antibody-producing animal immunized with a mixture of human GPA33 and mouse GPA33, and confirmed that the antibody accumulates extremely efficiently in the intestine of mouse.

The present inventors also confirmed that the activity of the anti-GPA33 antibody they created was maintained even after introduction of an agent molecule thereinto.

In addition, the present inventors confirmed that a fusion of scFV of the anti-GPA33 antibody they created with an activation domain of R-spondin, and a fusion of scFV of the anti-GPA33 antibody with an activation domain of R-spondin and Fc region of an antibody, promote Wnt/β-catenin signaling specifically in cells expressing GPA33, can promote growth of mouse small intestinal organoid, and can treat mouse models suffering from inflammatory bowel disease (IBD).

That is, the present invention provides the following.
[1] A human antibody or an antigen-binding portion thereof that specifically binds to human GPA33 and mouse GPA33.
[2] The antibody or an antigen-binding portion thereof of [1], which comprises a light chain variable region and a heavy chain variable region, wherein
   (1) the light chain variable region comprises CDR1 comprising the amino acid sequence shown in SEQ ID NO: 1, CDR2 comprising the amino acid sequence alanine-alanine-serine, and the amino acid sequence shown in SEQ ID NO: 2, and the heavy chain variable region comprises CDR1 comprising the amino acid sequence shown in SEQ ID NO: 3, CDR2 comprising the amino acid sequence shown in SEQ ID NO: 4, and CDR3 comprising the amino acid sequence shown in SEQ ID NO: 5;
   (2) the light chain variable region comprises CDR1 comprising the amino acid sequence shown in SEQ ID NO: 6, CDR2 comprising the amino acid sequence valine-alanine-serine, and the amino acid sequence shown in SEQ ID NO: 7, and the heavy chain variable region comprises CDR1 comprising the amino acid sequence shown in SEQ ID NO: 8, CDR2 comprising the amino acid sequence shown in SEQ ID NO: 4, and CDR3 comprising the amino acid sequence shown in SEQ ID NO: 5; or,
   (3) the light chain variable region comprises CDR1 comprising the amino acid sequence shown in SEQ ID NO: 1, CDR2 comprising the amino acid sequence isoleucine-alanine-serine, and the amino acid sequence shown in SEQ ID NO: 9, and the heavy chain variable region comprises CDR1 comprising the amino acid sequence shown in SEQ ID NO: 10, CDR2 comprising the amino acid sequence shown in SEQ ID NO: 11, and CDR3 comprising the amino acid sequence shown in SEQ ID NO: 12.
[3] The antibody or an antigen-binding portion thereof of [1], which comprises a light chain variable region and a heavy chain variable region, wherein
   (4) the light chain variable region comprises the amino acid sequence shown in SEQ ID NO: 13, and the heavy chain variable region comprises the amino acid sequence shown in SEQ ID NO: 14;
   (5) the light chain variable region comprises the amino acid sequence shown in SEQ ID NO: 15, and the heavy chain variable region comprises the amino acid sequence shown in SEQ ID NO: 16; or
   (6) the light chain variable region comprises the amino acid sequence shown in SEQ ID NO: 17, and the heavy chain variable region comprises the amino acid sequence shown in SEQ ID NO: 18.
[4] A conjugate of the antibody or an antigen-binding portion thereof of any of [1] to [3] and a functional molecule.
[5] The conjugate of [4], wherein the functional molecule is a drug for examination or for treating diseases.
[6] The conjugate of [5], wherein the drug is fused to the antibody or an antigen-binding portion thereof, or bound to a constant region thereof.
[7] An intestinal tissue targeting agent comprising the antibody or an antigen-binding portion thereof of any of [1] to [3].
[8] An agent for examining an intestinal tissue or treating a bowel disease, comprising the conjugate of [5] or [6].
[9] A polynucleotide encoding the antibody or an antigen-binding portion thereof of any of [1] to [3].
[10] A vector comprising the polynucleotide of [9].
[11] A transformant comprising the vector of [10].
[12] The antibody or an antigen-binding portion thereof of [1], wherein the heavy chain variable region (VH) is selected from the group consisting of the following (a1) to (a38):
   (a1) VH in which the amino acid sequences of CDR1 to CDR3 respectively comprise the amino acid sequences shown in SEQ ID NOs: 8, 197 and 217
   (a2) VH in which the amino acid sequences of CDR1 to CDR3 respectively comprise the amino acid sequences shown in SEQ ID NOs: 180, 198 and 218
   (a3) VH in which the amino acid sequences of CDR1 to CDR3 respectively comprise the amino acid sequences shown in SEQ ID NOs: 3, 199 and 217
   (a4) VH in which the amino acid sequences of CDR1 to CDR3 respectively comprise the amino acid sequences shown in SEQ ID NOs: 3, 200 and 219
   (a5) VH in which the amino acid sequences of CDR1 to CDR3 respectively comprise the amino acid sequences shown in SEQ ID NOs: 181, 200 and 219
   (a6) VH in which the amino acid sequences of CDR1 to CDR3 respectively comprise the amino acid sequences shown in SEQ ID NOs: 8, 199 and 5
   (a7) VH in which the amino acid sequences of CDR1 to CDR3 respectively comprise the amino acid sequences shown in SEQ ID NOs: 3, 201 and 217
   (a8) VH in which the amino acid sequences of CDR1 to CDR3 respectively comprise the amino acid sequences shown in SEQ ID NOs: 8, 199 and 217
   (a9) VH in which the amino acid sequences of CDR1 to CDR3 respectively comprise the amino acid sequences shown in SEQ ID NOs: 181, 200 and 5
   (a10) VH in which the amino acid sequences of CDR1 to CDR3 respectively comprise the amino acid sequences shown in SEQ ID NOs: 182, 202 and 220
   (a11) VH in which the amino acid sequences of CDR1 to CDR3 respectively comprise the amino acid sequences shown in SEQ ID NOs: 183, 203 and 221
   (a12) VH in which the amino acid sequences of CDR1 to CDR3 respectively comprise the amino acid sequences shown in SEQ ID NOs: 8, 4 and 5
   (a13) VH in which the amino acid sequences of CDR1 to CDR3 respectively comprise the amino acid sequences shown in SEQ ID NOs: 3, 4 and 219
   (a14) VH in which the amino acid sequences of CDR1 to CDR3 respectively comprise the amino acid sequences shown in SEQ ID NOs: 181, 204 and 222
   (a15) VH in which the amino acid sequences of CDR1 to CDR3 respectively comprise the amino acid sequences shown in SEQ ID NOs: 181, 204 and 223
   (a16) VH in which the amino acid sequences of CDR1 to CDR3 respectively comprise the amino acid sequences shown in SEQ ID NOs: 183, 203 and 224
   (a17) VH in which the amino acid sequences of CDR1 to CDR3 respectively comprise the amino acid sequences shown in SEQ ID NOs: 184, 205 and 225
   (a18) VH in which the amino acid sequences of CDR1 to CDR3 respectively comprise the amino acid sequences shown in SEQ ID NOs: 185, 206 and 226
   (a19) VH in which the amino acid sequences of CDR1 to CDR3 respectively comprise the amino acid sequences shown in SEQ ID NOs: 183, 203 and 227
   (a20) VH in which the amino acid sequences of CDR1 to CDR3 respectively comprise the amino acid sequences shown in SEQ ID NOs: 186, 207 and 228
   (a21) VH in which the amino acid sequences of CDR1 to CDR3 respectively comprise the amino acid sequences shown in SEQ ID NOs: 187, 208 and 229
   (a22) VH in which the amino acid sequences of CDR1 to CDR3 respectively comprise the amino acid sequences shown in SEQ ID NOs: 184, 209 and 230
   (a23) VH in which the amino acid sequences of CDR1 to CDR3 respectively comprise the amino acid sequences shown in SEQ ID NOs: 3, 4 and 5
   (a24) VH in which the amino acid sequences of CDR1 to CDR3 respectively comprise the amino acid sequences shown in SEQ ID NOs: 8, 4 and 231
   (a25) VH in which the amino acid sequences of CDR1 to CDR3 respectively comprise the amino acid sequences shown in SEQ ID NOs: 188, 4 and 5
   (a26) VH in which the amino acid sequences of CDR1 to CDR3 respectively comprise the amino acid sequences shown in SEQ ID NOs: 189, 210 and 232
   (a27) VH in which the amino acid sequences of CDR1 to CDR3 respectively comprise the amino acid sequences shown in SEQ ID NOs: 8, 211 and 5
   (a28) VH in which the amino acid sequences of CDR1 to CDR3 respectively comprise the amino acid sequences shown in SEQ ID NOs: 190, 202 and 233
   (a29) VH in which the amino acid sequences of CDR1 to CDR3 respectively comprise the amino acid sequences shown in SEQ ID NOs: 184, 205 and 234
   (a30) VH in which the amino acid sequences of CDR1 to CDR3 respectively comprise the amino acid sequences shown in SEQ ID NOs: 182, 203 and 235
   (a31) VH in which the amino acid sequences of CDR1 to CDR3 respectively comprise the amino acid sequences shown in SEQ ID NOs: 191, 212 and 236
   (a32) VH in which the amino acid sequences of CDR1 to CDR3 respectively comprise the amino acid sequences shown in SEQ ID NOs: 192, 208 and 229
   (a33) VH in which the amino acid sequences of CDR1 to CDR3 respectively comprise the amino acid sequences shown in SEQ ID NOs: 184, 205 and 237
   (a34) VH in which the amino acid sequences of CDR1 to CDR3 respectively comprise the amino acid sequences shown in SEQ ID NOs: 193, 213 and 238
   (a35) VH in which the amino acid sequences of CDR1 to CDR3 respectively comprise the amino acid sequences shown in SEQ ID NOs: 194, 214 and 239
   (a36) VH in which the amino acid sequences of CDR1 to CDR3 respectively comprise the amino acid sequences shown in SEQ ID NOs: 195, 215 and 240
   (a37) VH in which the amino acid sequences of CDR1 to CDR3 respectively comprise the amino acid sequences shown in SEQ ID NOs: 196, 216 and 241
   (a38) VH in which the amino acid sequences of CDR1 to CDR3 respectively comprise the amino acid sequences shown in SEQ ID NOs: 10, 11 and 12.
[13] The antigen-binding portion of [12], which is a VHH.
[14] A conjugate of the antibody or an antigen-binding portion thereof of [12] or [13] and a functional molecule.
[15] The conjugate of [14], wherein the functional molecule is a drug for examination or for treating a disease.
[16] The conjugate of [15], wherein the drug is fused to the antibody or an antigen-binding portion thereof, or bound to a constant region thereof.
[17] An intestinal tissue targeting agent comprising the antibody or an antigen-binding portion thereof of [12] or [13].
[18] An agent for examining an intestinal tissue or treating a bowel disease, comprising the conjugate of [15] or [16].
[19] A polynucleotide encoding the antibody or an antigen-binding portion thereof of [12] or [13].
[20] A vector comprising the polynucleotide of [19].
[21] A transformant comprising the vector of [20].

In another embodiment, the present invention provides the following.
[1'] A human antibody or an antigen-binding portion thereof that specifically binds to human GPA33 and mouse GPA33.
[2'] The antibody or an antigen-binding portion thereof of [1'], which comprises a light chain variable region and a heavy chain variable region, wherein
   (1) the light chain variable region comprises CDR1 comprising the amino acid sequence shown in SEQ ID NO: 1, CDR2 comprising the amino acid sequence alanine-alanine-serine, and the amino acid sequence shown in SEQ ID NO: 2, and the heavy chain variable region comprises CDR1 comprising the amino acid sequence shown in SEQ ID NO: 3, CDR2 comprising the amino acid sequence shown in SEQ ID NO: 4, and CDR3 comprising the amino acid sequence shown in SEQ ID NO: 5;
   (2) the light chain variable region comprises CDR1 comprising the amino acid sequence shown in SEQ ID NO: 6, CDR2 comprising the amino acid sequence valine-alanine-serine, and the amino acid sequence shown in SEQ ID NO: 7, and the heavy chain variable region comprises CDR1 comprising the amino acid sequence shown in SEQ ID NO: 8, CDR2 comprising the amino acid sequence shown in SEQ ID NO: 4, and CDR3 comprising the amino acid sequence shown in SEQ ID NO: 5; or,
   (3) the light chain variable region comprises CDR1 comprising the amino acid sequence shown in SEQ ID NO: 1, CDR2 comprising the amino acid sequence isoleucine-alanine-serine, and the amino acid sequence shown in SEQ ID NO: 9, and the heavy chain variable region comprises CDR1 comprising the amino acid sequence shown in SEQ ID NO: 10, CDR2 comprising the amino acid sequence shown in SEQ ID NO: 11, and CDR3 comprising the amino acid sequence shown in SEQ ID NO: 12.
[3'] The antibody or an antigen-binding portion thereof of [1'], which comprises a light chain variable region and a heavy chain variable region, wherein
   (4) the light chain variable region comprises the amino acid sequence shown in SEQ ID NO: 13, and the heavy chain variable region comprises the amino acid sequence shown in SEQ ID NO: 14;
   (5) the light chain variable region comprises the amino acid sequence shown in SEQ ID NO: 15, and the heavy chain variable region comprises the amino acid sequence shown in SEQ ID NO: 16; or
   (6) the light chain variable region comprises the amino acid sequence shown in SEQ ID NO: 17, and the heavy chain variable region comprises the amino acid sequence shown in SEQ ID NO: 18.
[4'] The antibody or an antigen-binding portion thereof of [1], wherein the heavy chain variable region (VH) is selected from the group consisting of the following (a1) to (a38):
   (a1) VH in which the amino acid sequences of CDR1 to CDR3 respectively comprise the amino acid sequences shown in SEQ ID NOs: 8, 197 and 217
   (a2) VH in which the amino acid sequences of CDR1 to CDR3 respectively comprise the amino acid sequences shown in SEQ ID NOs: 180, 198 and 218
   (a3) VH in which the amino acid sequences of CDR1 to CDR3 respectively comprise the amino acid sequences shown in SEQ ID NOs: 3, 199 and 217
   (a4) VH in which the amino acid sequences of CDR1 to CDR3 respectively comprise the amino acid sequences shown in SEQ ID NOs: 3, 200 and 219
   (a5) VH in which the amino acid sequences of CDR1 to CDR3 respectively comprise the amino acid sequences shown in SEQ ID NOs: 181, 200 and 219
   (a6) VH in which the amino acid sequences of CDR1 to CDR3 respectively comprise the amino acid sequences shown in SEQ ID NOs: 8, 199 and 5
   (a7) VH in which the amino acid sequences of CDR1 to CDR3 respectively comprise the amino acid sequences shown in SEQ ID NOs: 3, 201 and 217
   (a8) VH in which the amino acid sequences of CDR1 to CDR3 respectively comprise the amino acid sequences shown in SEQ ID NOs: 8, 199 and 217
   (a9) VH in which the amino acid sequences of CDR1 to CDR3 respectively comprise the amino acid sequences shown in SEQ ID NOs: 181, 200 and 5
   (a10) VH in which the amino acid sequences of CDR1 to CDR3 respectively comprise the amino acid sequences shown in SEQ ID NOs: 182, 202 and 220
   (a11) VH in which the amino acid sequences of CDR1 to CDR3 respectively comprise the amino acid sequences shown in SEQ ID NOs: 183, 203 and 221
   (a12) VH in which the amino acid sequences of CDR1 to CDR3 respectively comprise the amino acid sequences shown in SEQ ID NOs: 8, 4 and 5
   (a13) VH in which the amino acid sequences of CDR1 to CDR3 respectively comprise the amino acid sequences shown in SEQ ID NOs: 3, 4 and 219
   (a14) VH in which the amino acid sequences of CDR1 to CDR3 respectively comprise the amino acid sequences shown in SEQ ID NOs: 181, 204 and 222
   (a15) VH in which the amino acid sequences of CDR1 to CDR3 respectively comprise the amino acid sequences shown in SEQ ID NOs: 181, 204 and 223
   (a16) VH in which the amino acid sequences of CDR1 to CDR3 respectively comprise the amino acid sequences shown in SEQ ID NOs: 183, 203 and 224
   (a17) VH in which the amino acid sequences of CDR1 to CDR3 respectively comprise the amino acid sequences shown in SEQ ID NOs: 184, 205 and 225
   (a18) VH in which the amino acid sequences of CDR1 to CDR3 respectively comprise the amino acid sequences shown in SEQ ID NOs: 185, 206 and 226
   (a19) VH in which the amino acid sequences of CDR1 to CDR3 respectively comprise the amino acid sequences shown in SEQ ID NOs: 183, 203 and 227
   (a20) VH in which the amino acid sequences of CDR1 to CDR3 respectively comprise the amino acid sequences shown in SEQ ID NOs: 186, 207 and 228
   (a21) VH in which the amino acid sequences of CDR1 to CDR3 respectively comprise the amino acid sequences shown in SEQ ID NOs: 187, 208 and 229
   (a22) VH in which the amino acid sequences of CDR1 to CDR3 respectively comprise the amino acid sequences shown in SEQ ID NOs: 184, 209 and 230
   (a23) VH in which the amino acid sequences of CDR1 to CDR3 respectively comprise the amino acid sequences shown in SEQ ID NOs: 3, 4 and 5
   (a24) VH in which the amino acid sequences of CDR1 to CDR3 respectively comprise the amino acid sequences shown in SEQ ID NOs: 8, 4 and 231
   (a25) VH in which the amino acid sequences of CDR1 to CDR3 respectively comprise the amino acid sequences shown in SEQ ID NOs: 188, 4 and 5
   (a26) VH in which the amino acid sequences of CDR1 to CDR3 respectively comprise the amino acid sequences shown in SEQ ID NOs: 189, 210 and 232
   (a27) VH in which the amino acid sequences of CDR1 to CDR3 respectively comprise the amino acid sequences shown in SEQ ID NOs: 8, 211 and 5
   (a28) VH in which the amino acid sequences of CDR1 to CDR3 respectively comprise the amino acid sequences shown in SEQ ID NOs: 190, 202 and 233
   (a29) VH in which the amino acid sequences of CDR1 to CDR3 respectively comprise the amino acid sequences shown in SEQ ID NOs: 184, 205 and 234
   (a30) VH in which the amino acid sequences of CDR1 to CDR3 respectively comprise the amino acid sequences shown in SEQ ID NOs: 182, 203 and 235
   (a31) VH in which the amino acid sequences of CDR1 to CDR3 respectively comprise the amino acid sequences shown in SEQ ID NOs: 191, 212 and 236
   (a32) VH in which the amino acid sequences of CDR1 to CDR3 respectively comprise the amino acid sequences shown in SEQ ID NOs: 192, 208 and 229
   (a33) VH in which the amino acid sequences of CDR1 to CDR3 respectively comprise the amino acid sequences shown in SEQ ID NOs: 184, 205 and 237
   (a34) VH in which the amino acid sequences of CDR1 to CDR3 respectively comprise the amino acid sequences shown in SEQ ID NOs: 193, 213 and 238
   (a35) VH in which the amino acid sequences of CDR1 to CDR3 respectively comprise the amino acid sequences shown in SEQ ID NOs: 194, 214 and 239
   (a36) VH in which the amino acid sequences of CDR1 to CDR3 respectively comprise the amino acid sequences shown in SEQ ID NOs: 195, 215 and 240
   (a37) VH in which the amino acid sequences of CDR1 to CDR3 respectively comprise the amino acid sequences shown in SEQ ID NOs: 196, 216 and 241
   (a38) VH in which the amino acid sequences of CDR1 to CDR3 respectively comprise the amino acid sequences shown in SEQ ID NOs: 10, 11 and 12.
[5'] The antigen-binding portion of [4'], which is a VHH.
[6'] A conjugate of the antibody or an antigen-binding portion thereof of any of [1'] to [5'] and a functional molecule.
[7'] The conjugate of [6'], wherein the functional molecule is a drug for examination or for treating a disease.
[8'] The conjugate of [7'], wherein the drug is fused to the antibody or an antigen-binding portion thereof, or bound to a constant region thereof.
[9'] The conjugate of [8'], wherein the drug is R-spondin 1, 2, 3 or 4, or a partial fragment thereof.
[10'] An intestinal tissue targeting agent comprising the antibody or an antigen-binding portion thereof of any of [1'] to [5'].
[11'] An agent for examining an intestinal tissue or treating a bowel disease, comprising the conjugate of any of [6'] to [9'].
[12'] A polynucleotide encoding the antibody or an antigen-binding portion thereof of any of [1'] to [5'].
[13'] A vector comprising the polynucleotide of [11'].
[14'] A transformant comprising the vector of [12'].
[15'] The transformant of [14'], which is a mammalian cell that expresses the antibody or an antigen-binding portion thereof of any of [1'] to [5'] on a cell surface thereof.
[16'] An agent for promoting regeneration of and/or repairing an intestinal tissue, comprising the transformant of [15'].
[17'] A method for examining an intestinal tissue in a target or treating a bowel disease in a target, comprising administering the conjugate of any of [6'] to [9'] to the target.
[18'] A method for promoting regeneration of and/or repairing an intestinal tissue in a target, comprising administering the transformant of [15'] to the target.
[19'] Use of the conjugate of any of [6'] to [9'], in the production of a reagent for examining an intestinal tissue or a medicament for treating a bowel disease.
[20'] Use of the transformant of [15'], in the production of a medicament for promoting regeneration and/or repairing of an intestinal tissue.
[21'] The conjugate of any of [6'] to [9'], for use in examining an intestinal tissue, or treating a bowel disease.
[22'] Use of the transformant of [15'], for use in promoting regeneration of and/or repairing an intestinal tissue.

### [Advantageous Effects of Invention]

The antibody of the present invention specifically recognizes GPA33, which is highly expressed in intestinal tissue. Therefore, the antibody of the present invention accumulates very efficiently in intestinal tissue. The present invention thus enables drug delivery targeting intestinal tissue, and can realize treatment of intestinal disease with high therapeutic effect and reduced side effects.

### [Brief Description of Drawings]

[Fig. 1]

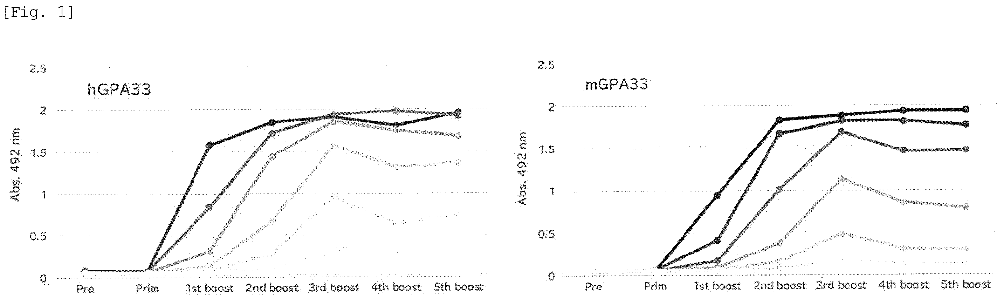

   Fig. 1 is a diagram showing the results of the antibody titers against anti-human GPA33 antibody and anti-mouse GPA33 antibody, in the serum derived from complete human antibody-producing animals immunized with human GPA33 and mouse GPA33, as evaluated by ELISA.
[Fig. 2]

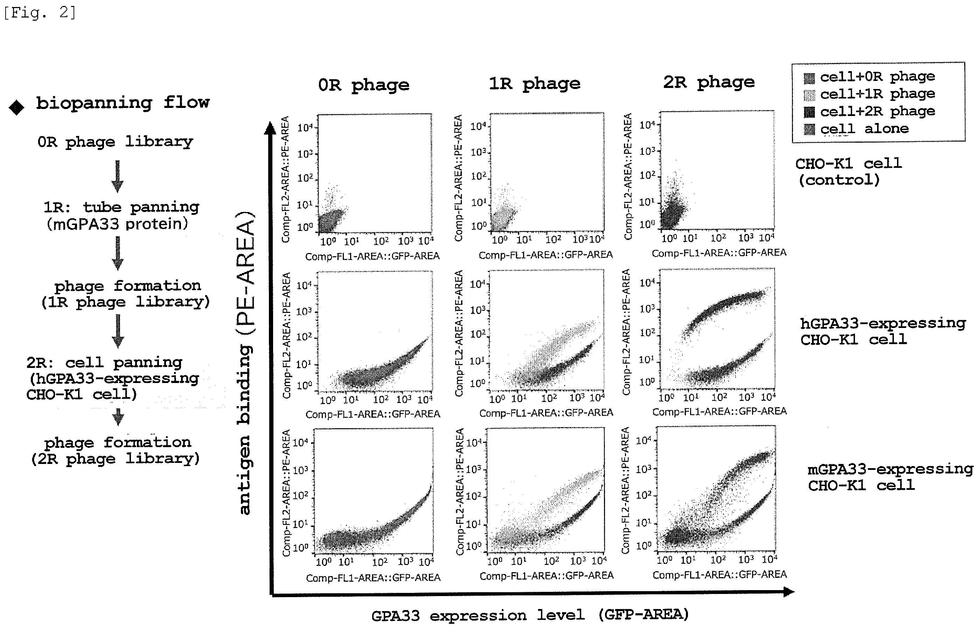

   Fig. 2 is a diagram showing the flow (left) and the results (right) of the isolation method in the enrichment of GPA33-specific antibody clones from GPA33 immune scFv antibody phage library.
[Fig. 3]

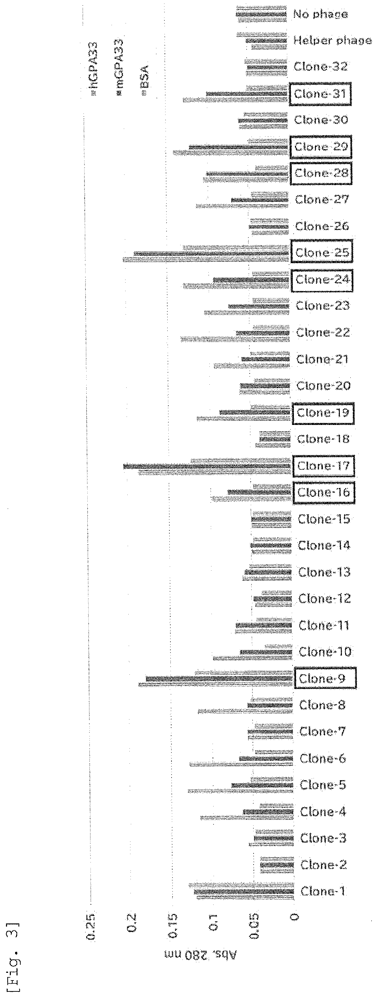

   Fig. 3 is a diagram showing the results of the binding activity of 32 phages, obtained from library stock 2R, to human and mouse GPA33 recombinant proteins, as analyzed by ELISA.
[Fig. 4]

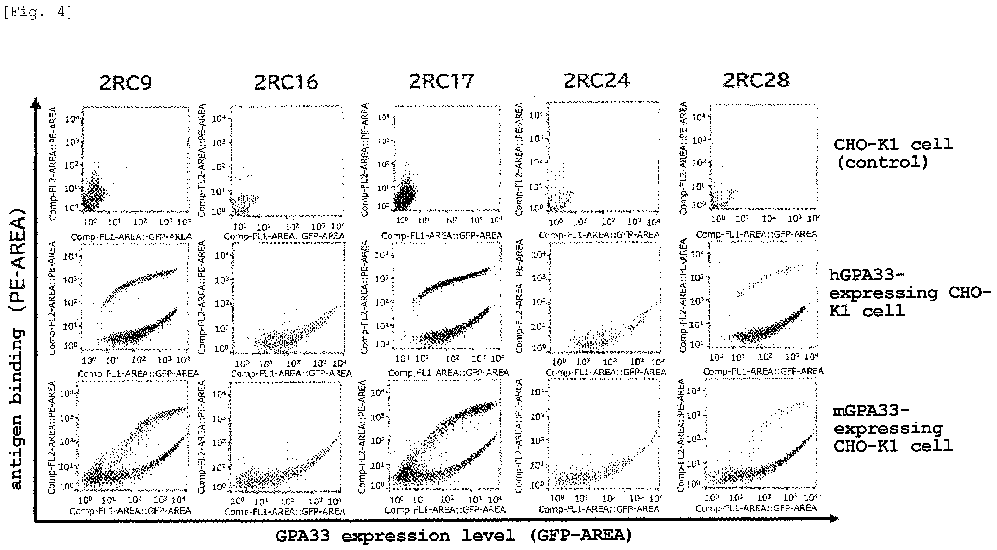

   Fig. 4 is a diagram showing the results of the binding activity of five different cloned phages, with cross-reactivity confirmed by ELISA, to GPA33, as evaluated by FACS.
[Fig. 5]

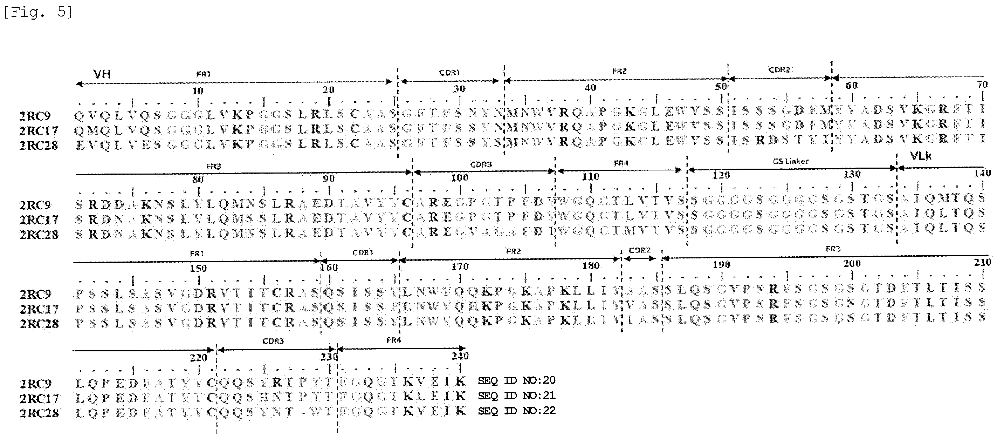

   Fig. 5 is a diagram showing the amino acid sequences of single-chain Fv of clones showing cross-binding activity against human and mouse GPA33. 2RC9: SEQ ID NO: 20, 2RC17: SEQ ID NO: 21, 2RC28: SEQ ID NO: 22.
[Fig. 6]

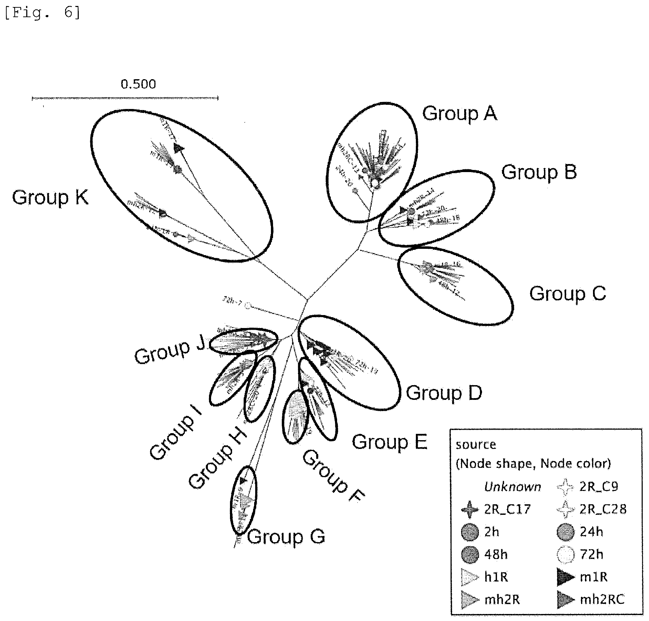

   Fig. 6 is a diagram showing a molecular phylogenetic tree of the VH sequences with the top 40 amplification rates, among the VH sequences amplified by each panning.
[Fig. 7]

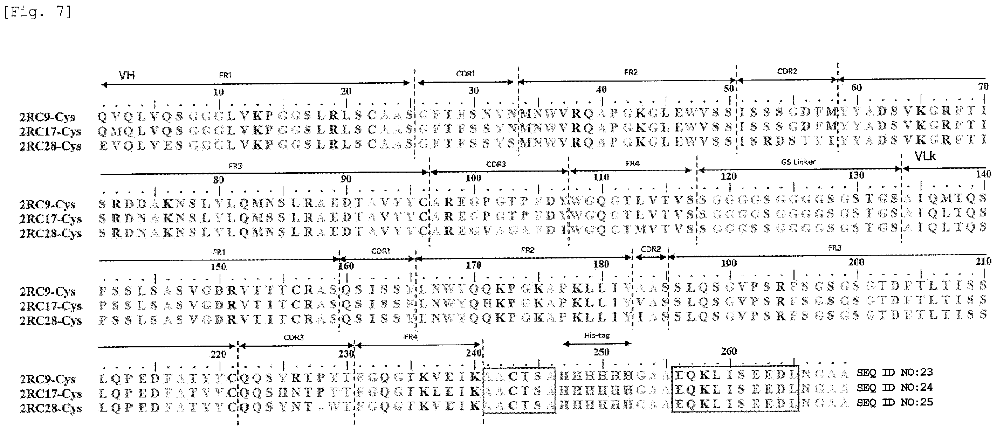

   Fig. 7 is a diagram showing the amino acid sequences of three types of anti-GPA33 scFVs (2RC9-Cys (SEQ ID NO: 23), 2RC17-Cys (SEQ ID NO: 24), 2RC28-Cys (SEQ ID NO: 25)) with Cys introduced at the C-terminal side.
[Fig. 8]

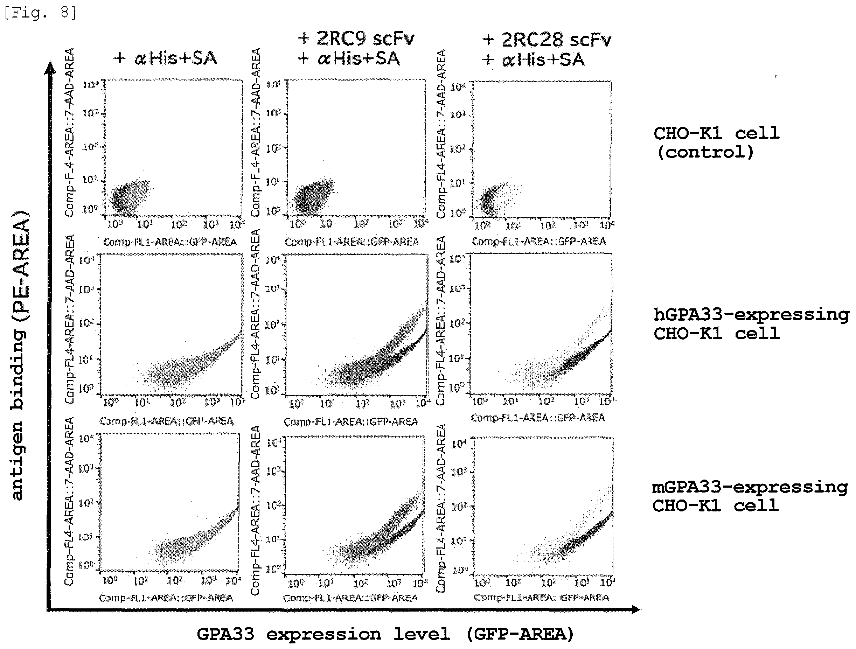

   Fig. 8 is a diagram showing the results of FACS analysis of GPA33-expressing cells with two types of anti-GPA33 single-chain Fv expressed in Escherichia coli.
[Fig. 9]

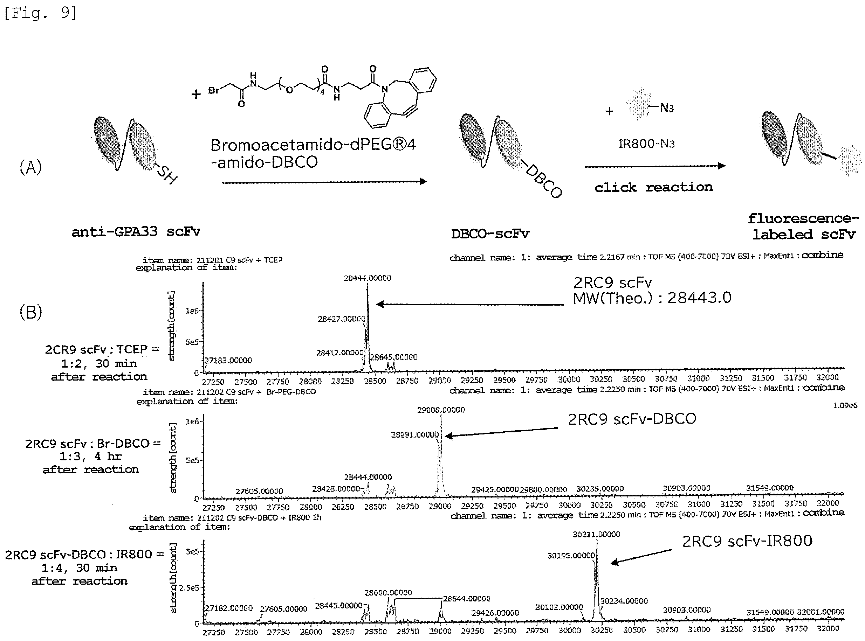

   Fig. 9(A) is a diagram showing the fluorescence labeling scheme of anti-GPA33 single-chain Fv expressed in Escherichia coli, and Fig. 9(B) is a diagram showing the results of LC-MS analysis of the reduction, DBCO labeling and fluorescence (IR-800) labeling reactions.
[Fig. 10]

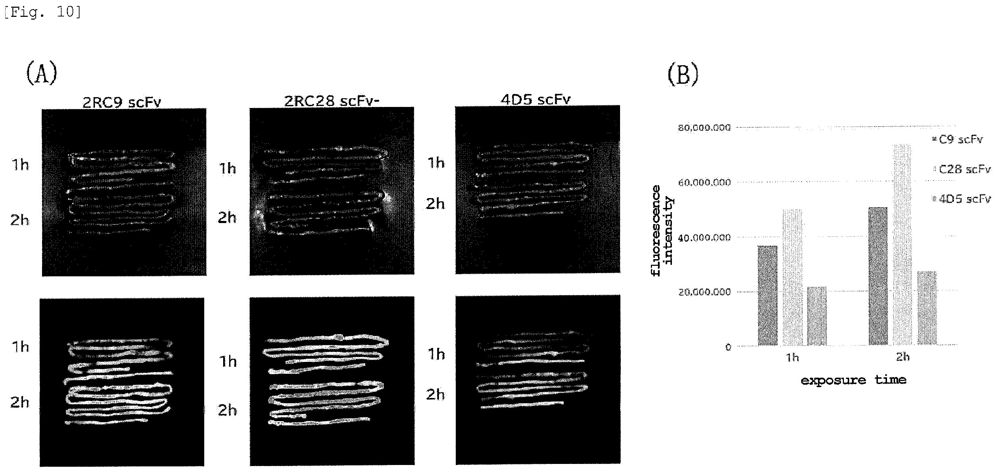

   Fig. 10(A) is a diagram showing the results of intestinal fluorescence imaging of mice after administration of fluorescence-labeled anti-GPA33 scFv, and Fig. 10(B) is a diagram showing the quantification results of the fluorescence transferred to the intestine.
[Fig. 11]

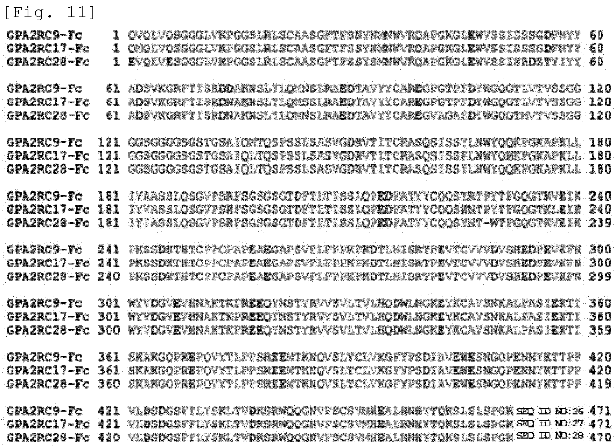

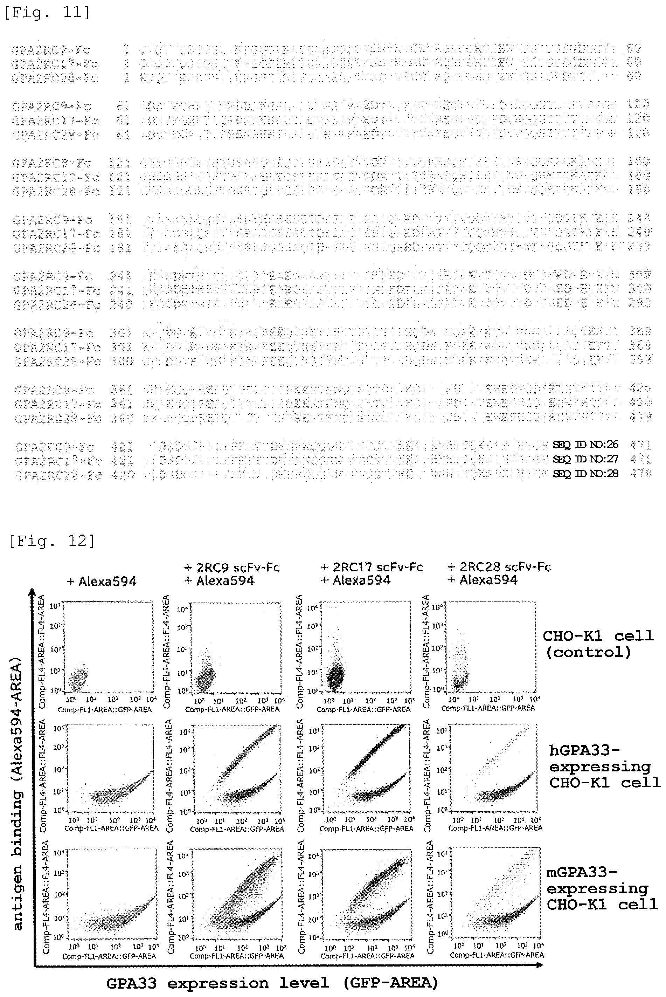

   Fig. 11 is a diagram showing the amino acid sequences of the three types of single-chain Fv-Fc (GPA2RC9-Fc (SEQ ID NO: 26), GPA2RC17-Fc (SEQ ID NO: 27), and GPA2RC28-Fc (SEQ ID NO: 28)) prepared in Example 7.
[Fig. 12]
   Fig. 12 is a diagram showing the binding ability of the three types of single-chain Fv-Fc prepared in Example 7 to human or mouse GPA33-expressing cells, as analyzed by FACS.
[Fig. 13]

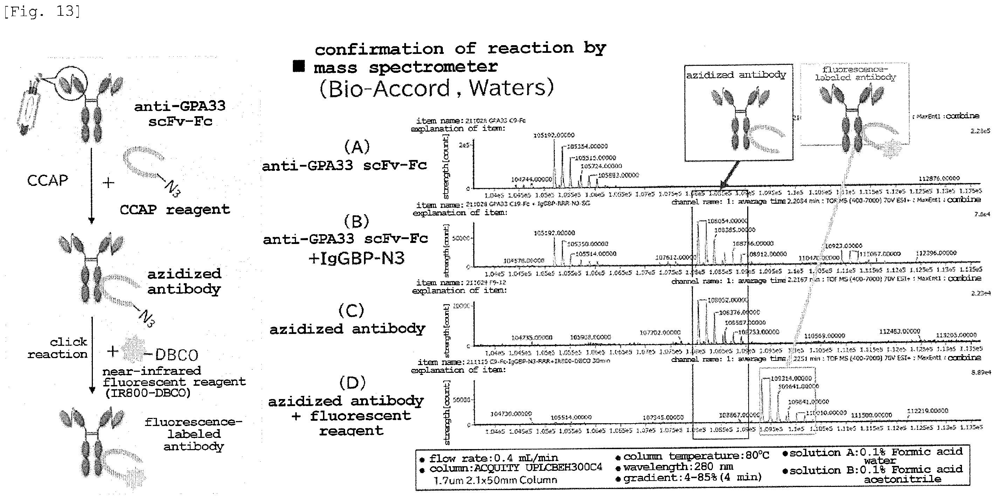

   Fig. 13 is a diagram showing the outline of the fluorescence labeling process of anti-GPA33 single-chain Fv-Fc by the CCPA method, and the confirmation results of the reaction of fluorescence-labeled anti-GPA33 single-chain Fv-Fc ((A) to (D)).
[Fig. 14]

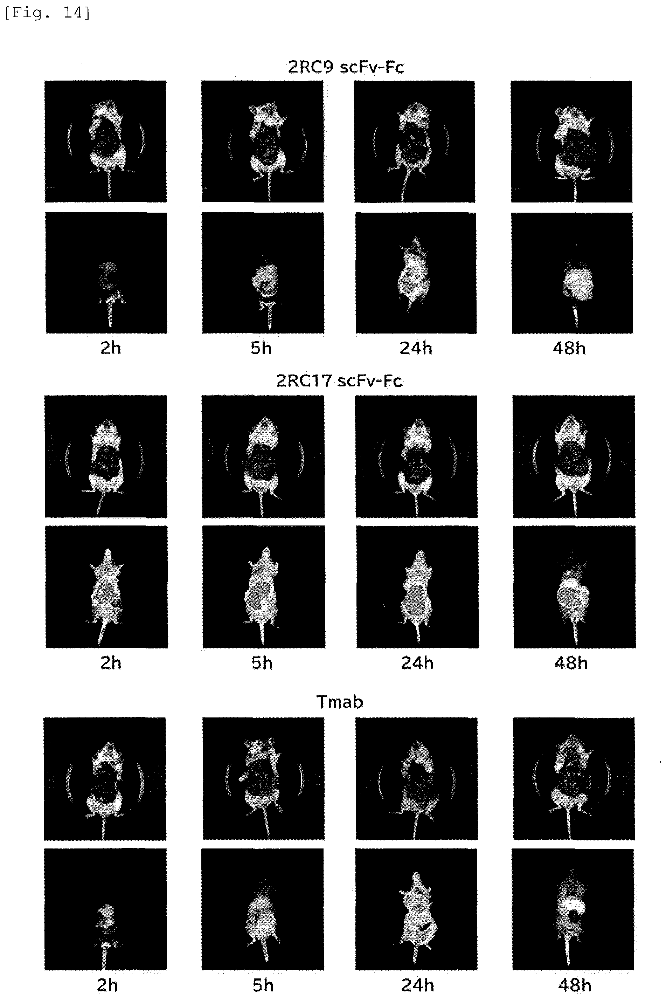

   Fig. 14 shows fluorescence imaging of mouse internal organs at elapsed times after administration of fluorescence-labeled anti-GPA33 single-chain Fv-Fc antibody.
[Fig. 15]

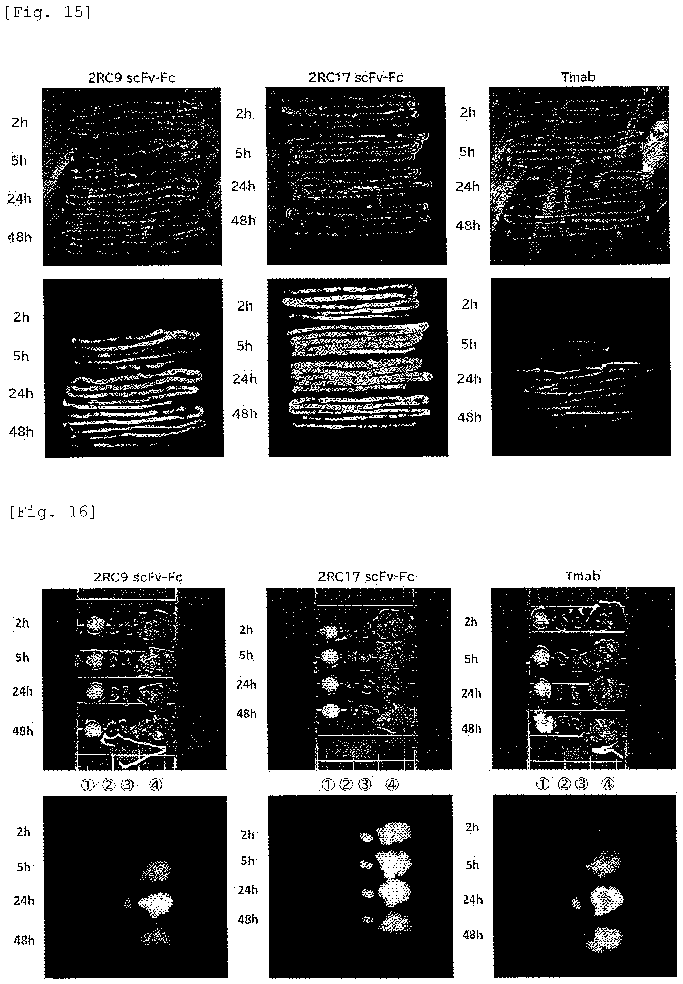

   Fig. 15 shows fluorescence imaging of mouse intestine at elapsed times after administration of fluorescence-labeled anti-GPA33 single-chain Fv-Fc antibody.
[Fig. 16]
   Fig. 16 shows fluorescence imaging of isolated organ ((1) brain, (2) heart, (3) kidney, (4) liver) of mouse at elapsed times after administration of the fluorescence-labeled anti-GPA33 single-chain Fv-Fc antibody.
[Fig. 17]

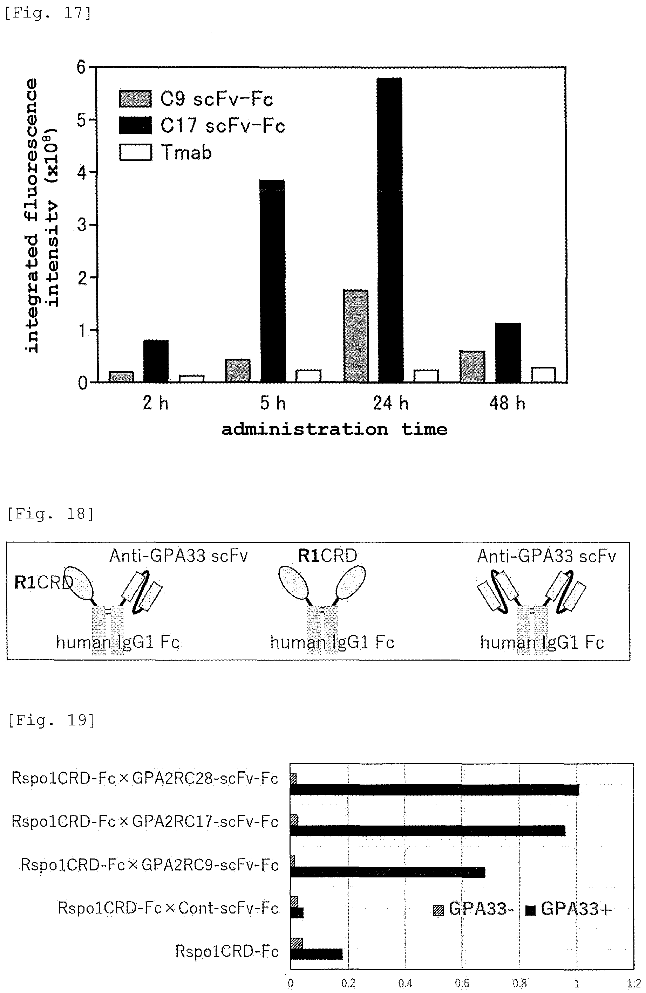

   Fig. 17 is a diagram showing the quantification results of the accumulated amount of each anti-GPA33 scFv-Fc in the intestine.
[Fig. 18]
   Fig. 18 is a diagram showing the structures of the heterodimer of anti-GPA33 scFv-Fc and R-spondin 1 CRD fragment-Fc, the R-spondin 1 CRD fragment-Fc homodimer, and the anti-GPA33 scFv-Fc homodimer.
[Fig. 19]
   Fig. 19 is a diagram showing the measurement results of the Wnt/β-catenin signal promoting activity of culture supernatant containing the heterodimer of anti-GPA33 scFv-Fc and human R-spondin 1 CRD fragment-Fc.
[Fig. 20]

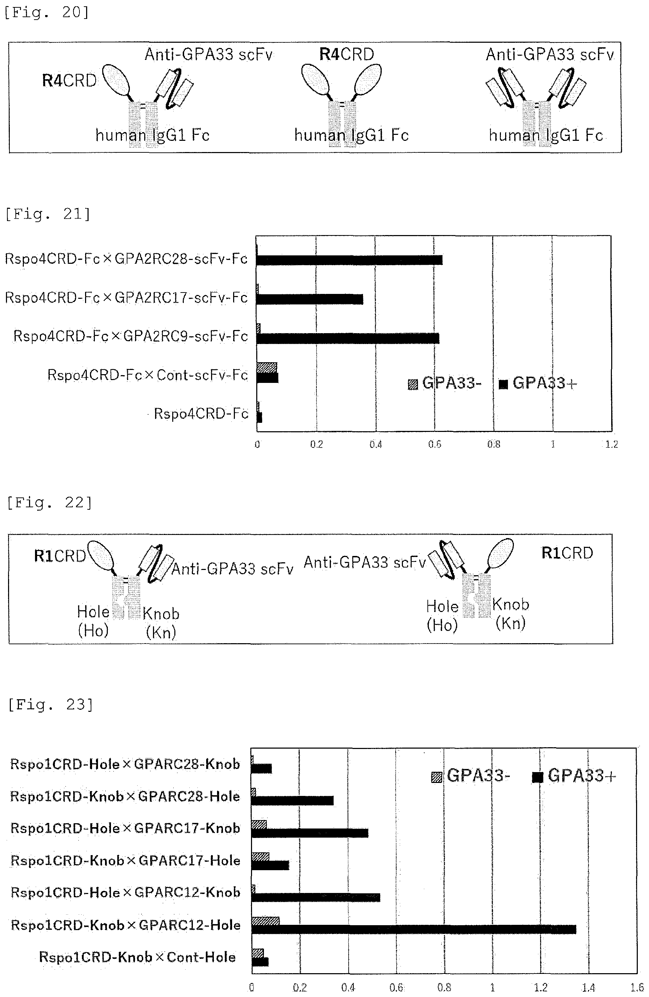

   Fig. 20 is a diagram showing the structures of the heterodimer of anti-GPA33 scFv-Fc and R-spondin4 CRD fragment-Fc, the R-spondin4 CRD fragment-Fc homodimer, and the anti-GPA33 scFv-Fc homodimer.
[Fig. 21]
   Fig. 21 is a diagram showing the measurement results of the Wnt/β-catenin signal promoting activity of culture supernatant containing the heterodimer of anti-GPA33 scFv-Fc and human R-spondin 1 CRD fragment-Fc.
[Fig. 22]
   Fig. 22 is a diagram showing the structures of anti-GPA33-scFv-Fc-Knob×R1CRD-Fc-Hole and anti-GPA33-scFv-Fc-Hole×R1CRD-Fc-Knob.
[Fig. 23]
   Fig. 23 is a diagram showing the measurement results of the Wnt/β-catenin signal promoting activity of culture supernatant containing the heterodimer of Knobs into Holes type anti-GPA33 scFv-Fc/human R-spondin 1 CRD fragment-Fc.
[Fig. 24]

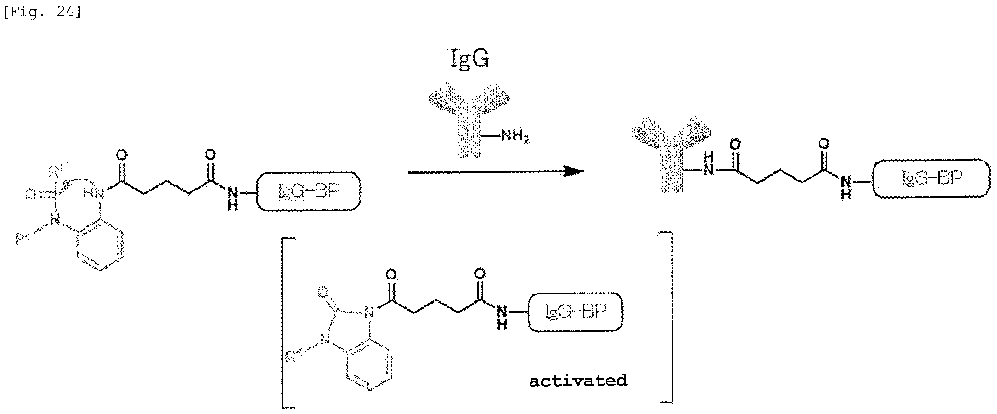

   Fig. 24 is a schematic diagram showing the reaction of modifying an antibody with IgG-BP using a compound used in the tCAP method.
[Fig. 25]

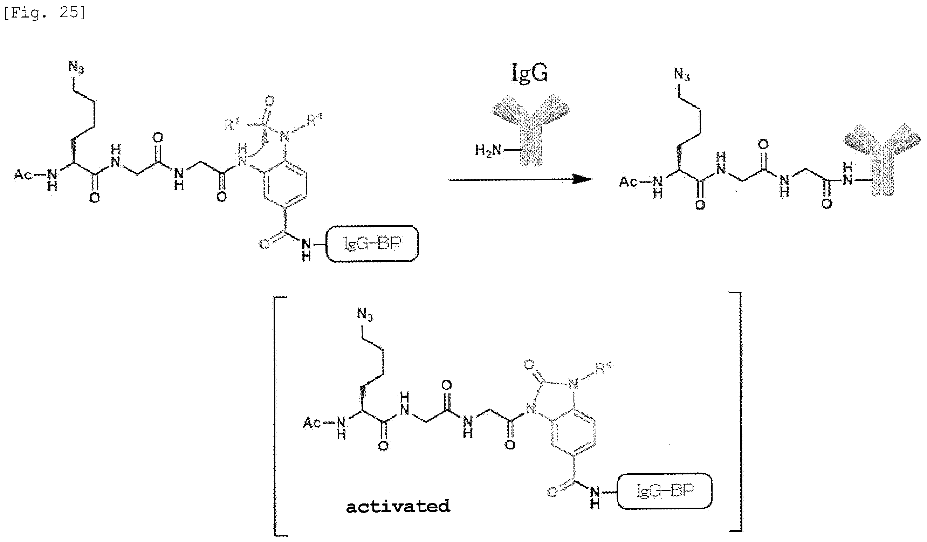

   Fig. 25 is a schematic diagram showing the reaction of modifying an antibody with a compound used in the tCAP method.
[Fig. 26]

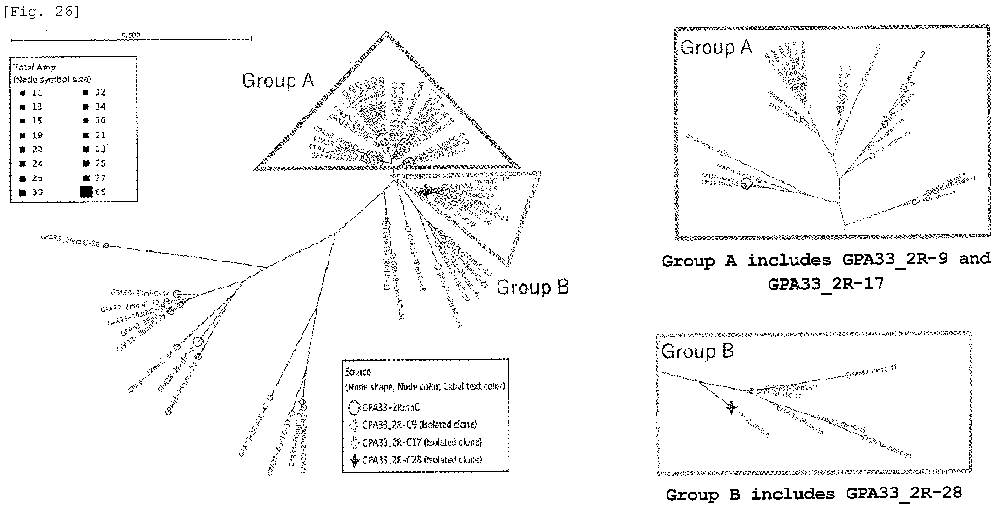

   Fig. 26 is a diagram showing a molecular phylogenetic tree created based on the VH sequences with the top 50 amplification rates, among the phage VH sequences obtained after 2R cell panning.
[Fig. 27]

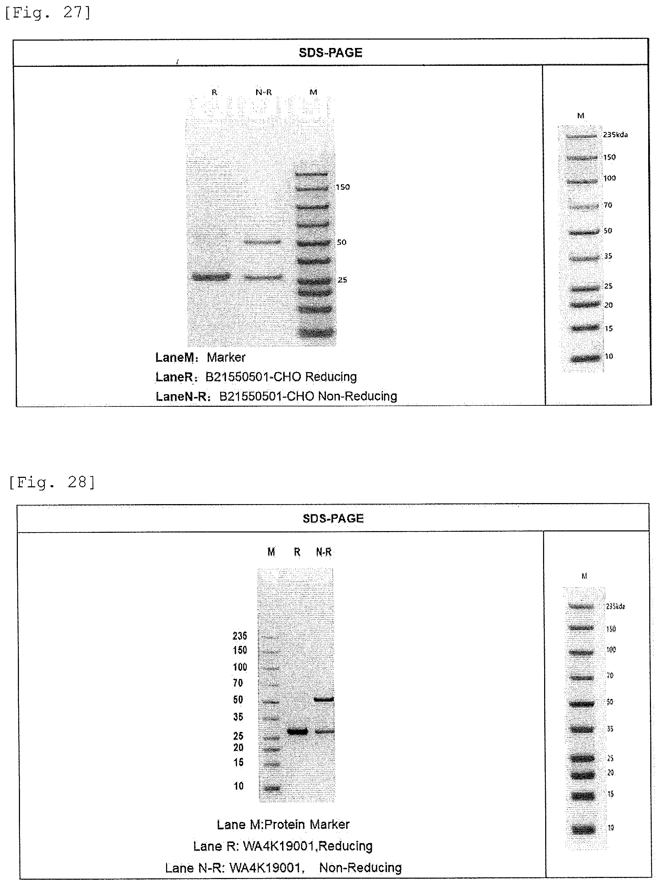

   Fig. 27 is a diagram showing the results of SDS-PAGE after expression and purification of G17(GPA33_2R_C17)_scFv-His in CHO cells.
[Fig. 28]
   Fig. 28 is a diagram showing the results of SDS-PAGE after expression and purification of G9(GPA33_2R_C9)_scFv-His in CHO cells.
[Fig. 29]

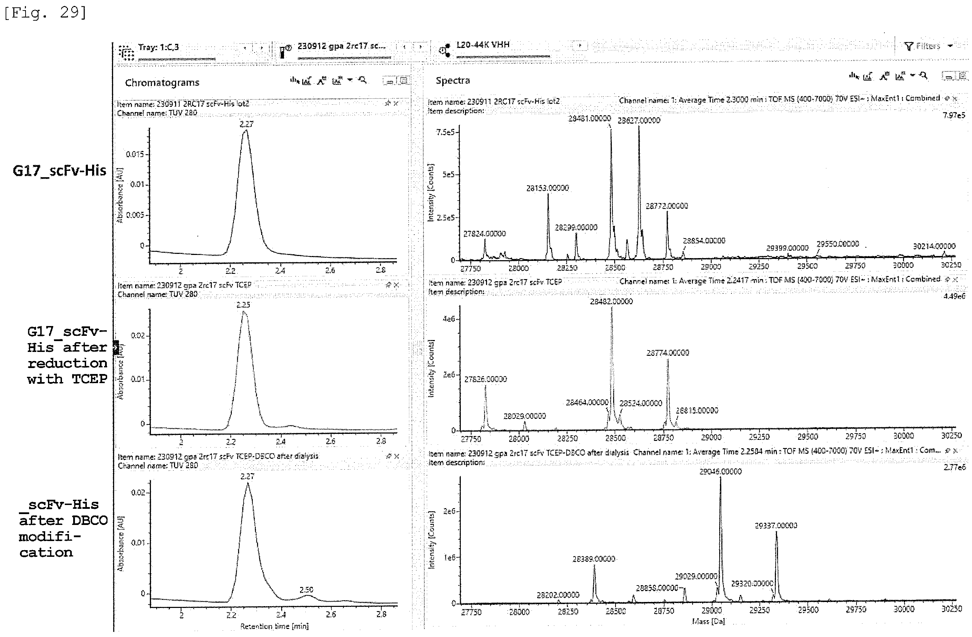

   Fig. 29 is a diagram showing the results of LC-MS analysis of reduction with TCEP and DBCO modification with Bromoacetyl-PEG4-DBCO reagent of G17_scFv-His.
[Fig. 30]

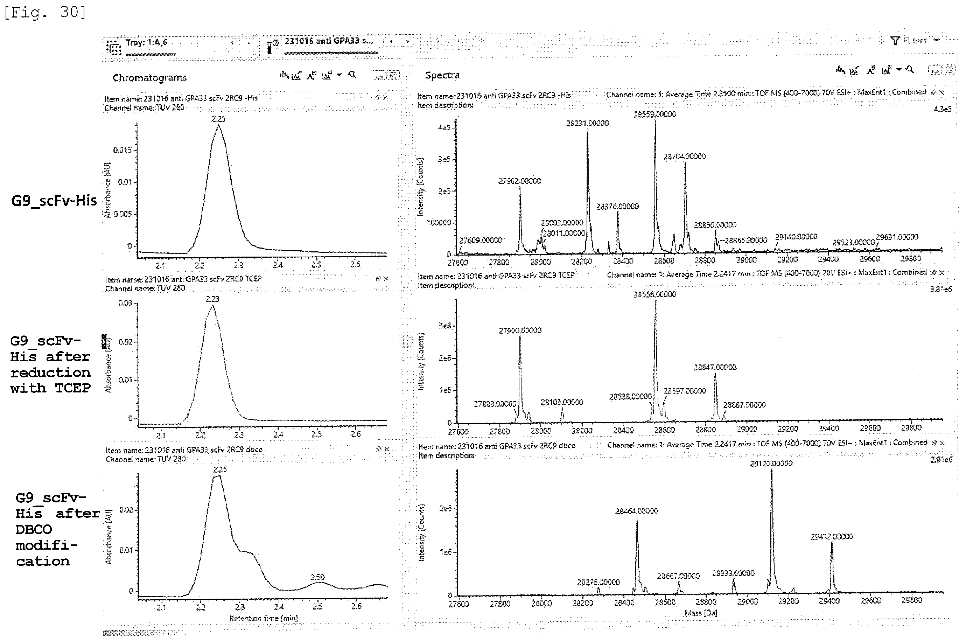

   Fig. 30 is a diagram showing the results of LC-MS analysis of G9_scFv-His after reduction with TCEP and G9_scFv-His after DBCO modification with Bromoacetyl-PEG4-DBCO reagent.
[Fig. 31]

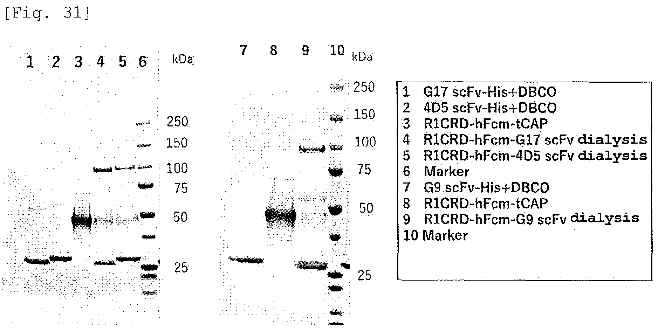

   Fig. 31 is a diagram showing SDS-PAGE after the click reaction of azidized R1CRD-Fcm with DBCO-G17 or DBCO-G9.
[Fig. 32]

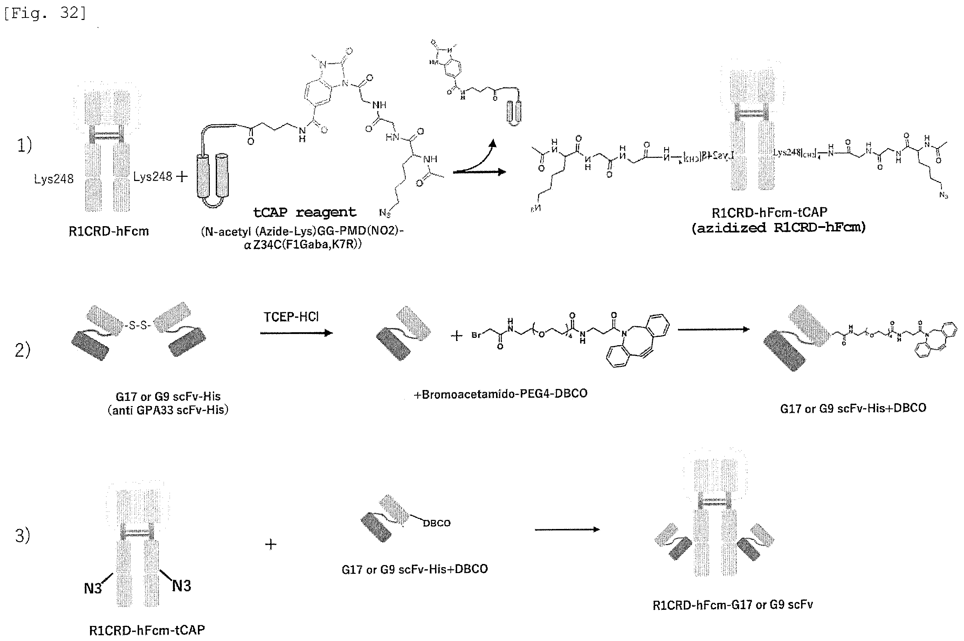

   Fig. 32 is a diagram showing the outline of the production of a conjugate between R1CRD-Fcm and a DBCO- anti-GPA33 antibody scFv fragment by the tCAP method.
[Fig. 33]

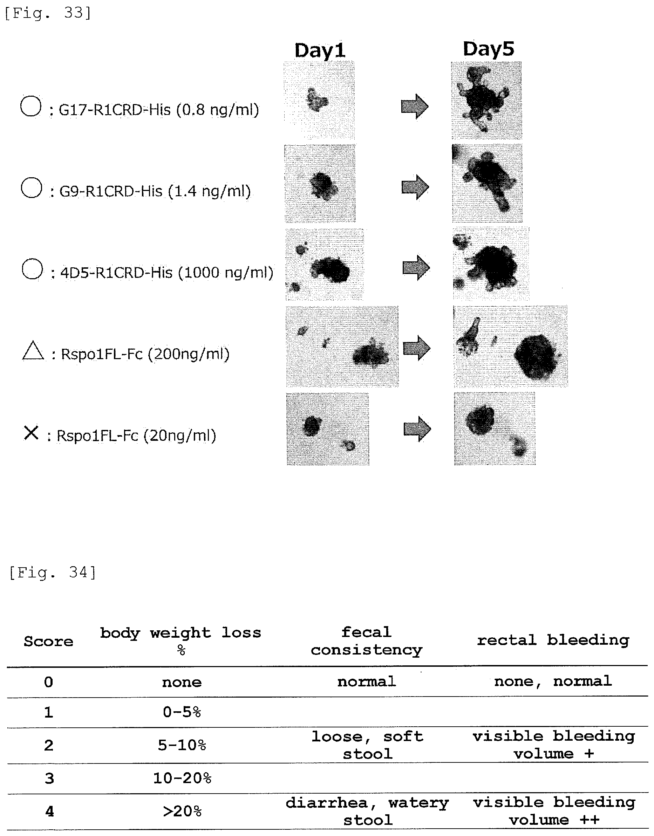

   Fig. 33 is a microphotograph showing typical organoid morphology when mouse small intestinal organoids were cultured using an anti-GPA33 antibody scFv-Rspo fragment fusion or an anti-GPA33 antibody scFv-Rspo fragment-Fc conjugate.
[Fig. 34]
   Fig. 34 is a diagram showing the scoring criteria (body weight loss, fecal consistency, rectal bleeding) used in Example 23.
[Fig. 35]

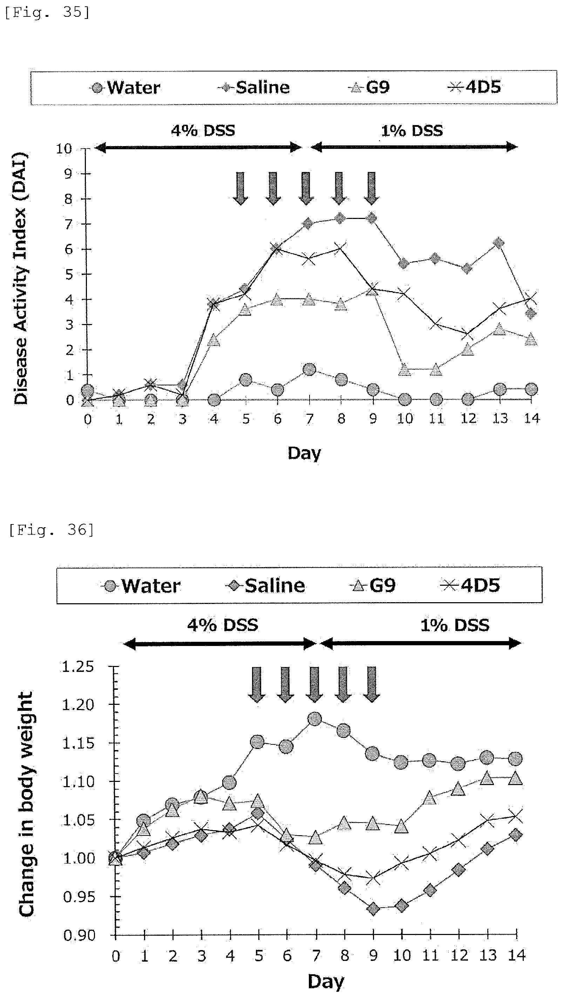

   Fig. 35 is a diagram showing the average Disease Activity Index (DAI) score of each animal.
[Fig. 36]
   Fig. 36 is a diagram showing the average change in the body weight of each animal.
[Fig. 37]

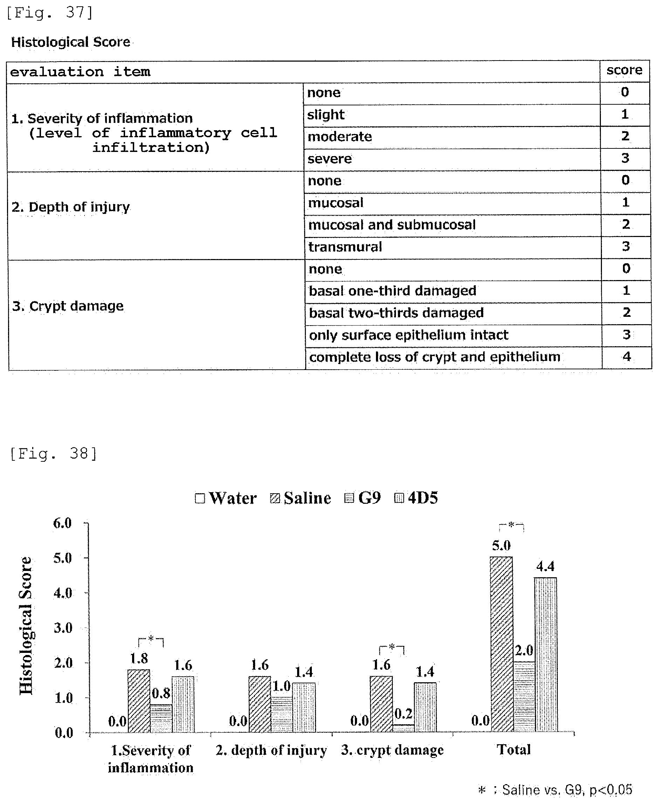

   Fig. 37 is a diagram showing the scoring criteria (Histological score) used in Example 23.
[Fig. 38]
   Fig. 38 is a diagram showing the histological score of each group in Example 23.
[Fig. 39]

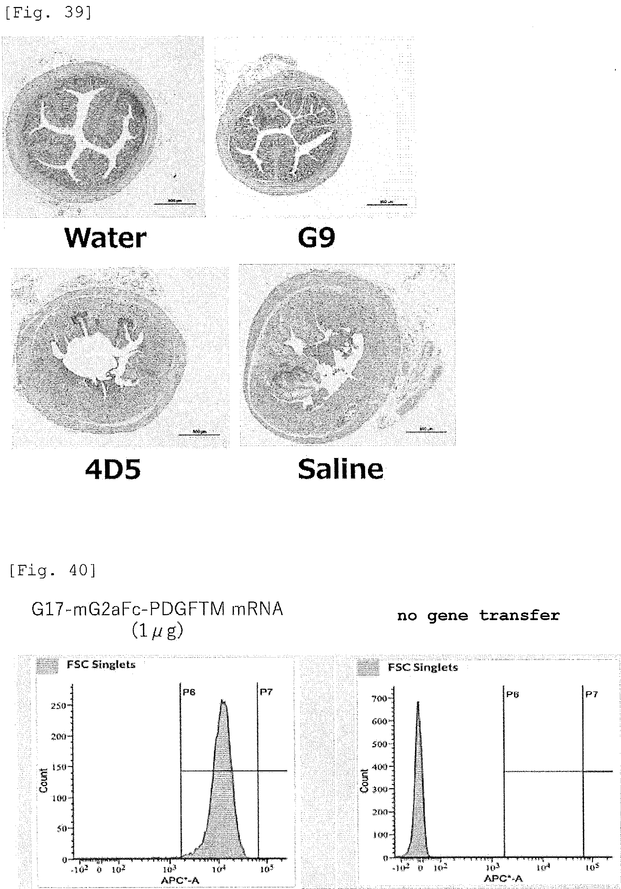

   Fig. 39 is a microphotograph of typical colon tissue of a DSS-induced inflammatory bowel disease model treated with an anti-GPA33 antibody scFv-Rspo fragment fusion, and the like.
[Fig. 40]
   Fig. 40 is a FACS analysis diagram showing the production of human mesenchymal stem cells expressing anti-GPA33 antibody scFv-Fc on the membrane surface.

### [Description of Embodiments]

The present invention is described in detail below.

The present invention provides a human antibody or an antigen-binding portion thereof that specifically binds to human GPA33 and mouse GPA33 (hereinafter sometimes referred to as "the antibody, etc. of the present invention").

In the present specification, GPA33 is a transmembrane glycoprotein belonging to the CTX family, and is a known protein also called "A33", "cell surface A33 antigen", "glycoprotein A33 (transmembrane)", "glycoprotein A33", or the like. Human and mouse GPA33 are proteins consisting of 319 amino acids, and the amino acid sequences and gene sequences thereof are recorded in databases (in NCBI, human gene (Gene ID:10223), human protein (NP_005805.1), mouse gene (Gene ID: 59290), mouse protein (NCBI NP_067623.1)).

The "human GPA33" means that the amino acid sequence or nucleic acid sequence of human GPA33 has an amino acid sequence or nucleic acid sequence that is identical or substantially identical to the amino acid sequence or nucleic acid sequence of GPA33 naturally expressed in human. "Substantially identical" means that the amino acid sequence or nucleic acid sequence of interest has 70% or more (preferably 80% or more, more preferably 90% or more, further preferably 95% or more, most preferably 99% or more) identity with the amino acid sequence or nucleic acid sequence of GPA33 naturally expressed in human, and has the function of human GPA33. (The same interpretation also applies to mouse GPA33.)

In the present specification, "antibody" refers to a glycoprotein or an antigen-binding portion thereof that includes at least two heavy chains (H) and two light chains (L) linked by disulfide bonds. Each heavy chain is composed of a heavy chain variable region (abbreviated as V_{H} herein) and a heavy chain constant region. The heavy chain constant region is composed of three domains C_{H}1, C_{H}2 and C_{H}3. Each light chain is composed of a light chain variable region (abbreviated as V_{L} herein) and a light chain constant region. The light chain constant region is composed of one domain C_{L}. The V_{H} and V_{L} regions are further subdivided into highly variable regions called complementarity determining regions (CDRs), in which more conserved regions called framework regions (FRs) are scattered. V_{H} and V_{L} are each composed of three CDRs and four FRs, and arranged from amino terminus to carboxy terminus in the following order, i.e., FR1, CDR1, FR2, CDR2, FR3, CDR3, FR4. The variable regions of the heavy and light chains contain binding domains that interact with antigens. The constant region of antibody can mediate the binding of immunoglobulin to a host tissue or factor, including various cells of the immune system (e.g., effector cells) and the first component (C1q) of old complement system.

In the present specification, the "antigen-binding portion" of an antibody refers to one or more fragments of an antibody that retain the ability to specifically bind to an antigen (e.g., human GPA33). It has been clarified that the antigen-binding function of an antibody is performed by a fragment of a full-length antibody. Examples of the binding fragment included in the term "antigen-binding portion" of an antibody include (i) a Fab fragment, which is a monovalent fragment composed of V_{L}, V_{H}, C_{L} and C_{H1} domains; (ii) a F(ab')₂ fragment, which is a bivalent fragment containing two Fab fragments linked by disulfide bridges in the hinge region; (iii) a Fab' fragment, which is an original Fab having a portion of the hinge region (see FUNDAMENTAL IMMUNOLOGY (Paul ed., 3.sup.rd ed. 1993)); (iv) an Fd fragment composed of V_{H} and C_{H1} domains; (v) an Fv fragment composed of V_{L} and V_{H} domains of a single arm of an antibody; (vi) a dAb fragment composed of V_{H} domain (Wardra, (1989) Nature 341:544-546); (vii) an isolated complementarity determining region (CDR); and (viii) a variable domain of heavy chain of heavy chain antibody (VHH antibody) composed only of heavy chains. Furthermore, although the two domains V_{L} and V_{H} of an Fv fragment are encoded by separate genes, they can be linked by recombinant techniques using a synthetic linker that can produce them as a single protein chain, in which chain the V_{L} and V_{H} regions as a pair form a monovalent molecule (known as a single-chain Fv (scFv); see, e.g., Bird et al., (1988) Science 242:423-426; and Huston et al., (1988), Proc. Natl. Acad. Sci. USA 85:5879-5883). Such single-chain antibody is also encompassed in the "antigen-binding portion" of an antibody. These antibody fragments can be obtained using conventional techniques known to those of ordinary skill in the art, and the fragments are screened for usefulness as in the case of unmodified antibodies.

In the present specification, that an antibody or antigen-binding portion "specifically binds" to an antigen X means that the K_{D} value of the binding affinity of the antibody or antigen-binding portion to antigen X in an antigen-antibody reaction is 1x10⁻⁷ M or less. Particularly, that an antibody or an immunoreactive fragment thereof "specifically binds" to an antigen X means that the antibody or an antigen-binding portion thereof binds to antigen X with substantially higher affinity than the affinity to other amino acid sequences or steric structures. As used herein, "substantially higher affinity" means a high affinity at which a specific amino acid sequence or steric structure can be detected and distinguished from other amino acid sequences or steric structures by a desired measurement device or method. For example, substantially high affinity may mean a binding strength of 2 times or more, 5 times or more, or 10 times or more. Specific binding does not mean binding with substantially high affinity only to a single antigen; in the case of cross-reactivity as described below, it means binding with substantially high affinity to multiple antigens.

The antibody, etc. of the present invention is preferably a monoclonal antibody (or a portion of a monoclonal antibody). The "monoclonal antibody" refers to a preparation of antibody molecules having a single molecular composition. A monoclonal antibody composition exhibits a single binding specificity and affinity for a portion of an antigen called a specific epitope. The antigen-binding portion is preferably monoclonal and includes, for example, a monoclonal VHH antibody and the like.

The antibody, etc. of the present invention is a human antibody (or a portion of a human antibody). A "human antibody" refers to an antibody having variable regions, derived from human germ line immunoglobulin sequences, in both the framework and CDR region. When the antibody further contains a constant region, the constant region is also derived from a human germ line immunoglobulin sequence. In the present specification, "human antibody" also includes embodiments containing amino acid residues not encoded by human germ line immunoglobulin sequences (e.g., mutation introduced by in vitro random or site-directed mutagenesis or in vivo somatic mutation). However, in the present specification, the term "human antibody" is not intended to include an antibody in which CDR sequence derived from the germ line of an animal species other than human, such as mouse, is fused onto a human framework sequence.

In the present specification, the human antibody includes "artificial human antibody". Artificial human antibody can be produced using a general gene recombinant method (e.g., Rothe, C. et al. J. Mol. Biol. 376:1182-1200, 2008).

The antibody, etc. of the present invention is preferably isolated or purified. Being "isolated or purified" means that an operation to remove components other than the target component from a naturally occurring state has been performed. The purity of the isolated or purified antibody of the present invention (the ratio of the weight of the antibody of the present invention to the total protein weight) is generally 50% or more, preferably 70% or more, more preferably 90% or more, most preferably 95% or more (e.g., substantially 100%).

The antibody, etc. of the present invention specifically binds to human GPA33 and also has cross-reactivity with mouse GPA33. "Cross-reactivity" means that the antibody specifically binds to two or more types of antigens and concretely means that an antibody that specifically binds to human GPA33 also specifically binds to mouse GPA33 through an antigen-antibody reaction.

As the antibody, etc. of the present invention, the antibodies or antigen-binding portions described in the following (1) to (3) can be mentioned.
(1) An antibody or an antigen-binding portion thereof, containing a light chain variable region and a heavy chain variable region, wherein the light chain variable region contains CDR1 containing the amino acid sequence shown in SEQ ID NO: 1, CDR2 containing the amino acid sequence alanine-alanine-serine, and the amino acid sequence shown in SEQ ID NO: 2, and the heavy chain variable region contains CDR1 containing the amino acid sequence shown in SEQ ID NO: 3, CDR2 containing the amino acid sequence shown in SEQ ID NO: 4, and CDR3 containing the amino acid sequence shown in SEQ ID NO: 5.
(2) An antibody or an antigen-binding portion thereof, containing a light chain variable region and a heavy chain variable region, wherein the light chain variable region contains CDR1 containing the amino acid sequence shown in SEQ ID NO: 6, CDR2 containing the amino acid sequence valine-alanine-serine, and the amino acid sequence shown in SEQ ID NO: 7, and the heavy chain variable region contains CDR1 containing the amino acid sequence shown in SEQ ID NO: 8, CDR2 containing the amino acid sequence shown in SEQ ID NO: 4, and CDR3 containing the amino acid sequence shown in SEQ ID NO: 5.
(3) An antibody or an antigen-binding portion thereof, wherein the light chain variable region contains CDR1 containing the amino acid sequence shown in SEQ ID NO: 1, CDR2 containing the amino acid sequence isoleucine-alanine-serine, and the amino acid sequence shown in SEQ ID NO: 9, and the heavy chain variable region contains CDR1 containing the amino acid sequence shown in SEQ ID NO: 10, CDR2 containing the amino acid sequence shown in SEQ ID NO: 11, and CDR3 containing the amino acid sequence shown in SEQ ID NO: 12.

In one preferred embodiment, the antibody, etc. of the present invention include the antibodies or antigen-binding portions described in the following (4) to (6).
(4) An antibody or an antigen-binding portion, containing a light chain variable region and a heavy chain variable region, wherein the light chain variable region contains the amino acid sequence shown in SEQ ID NO: 13, and the aforementioned heavy chain variable region contains the amino acid sequence shown in SEQ ID NO: 14.
(5) An antibody or an antigen-binding portion, containing a light chain variable region and a heavy chain variable region, wherein the light chain variable region contains the amino acid sequence shown in SEQ ID NO: 15, and the aforementioned heavy chain variable region contains the amino acid sequence shown in SEQ ID NO: 16.
(6) An antibody or an antigen-binding portion, wherein the aforementioned light chain variable region contains the amino acid sequence shown in SEQ ID NO: 17, and the heavy chain variable region contains the amino acid sequence shown in SEQ ID NO: 18.
(7) An antibody or an antigen-binding portion thereof, wherein CDR1 to CDR3 (CDRH1 to CDRH3) of a heavy chain variable region each contain, in that order, amino acid sequences selected from the following:
   (a1) the amino acid sequences shown in SEQ ID NOs: 8, 197 and 217
   (a2) the amino acid sequences shown in SEQ ID NOs: 180, 198 and 218
   (a3) the amino acid sequences shown in SEQ ID NOs: 3, 199 and 217
   (a4) the amino acid sequences shown in SEQ ID NOs: 3, 200 and 219
   (a5) the amino acid sequences shown in SEQ ID NOs: 181, 200 and 219
   (a6) the amino acid sequences shown in SEQ ID NOs: 8, 199 and 5
   (a7) the amino acid sequences shown in SEQ ID NOs: 3, 201 and 217
   (a8) the amino acid sequences shown in SEQ ID NOs: 8, 199 and 217
   (a9) the amino acid sequences shown in SEQ ID NOs: 181, 200 and 5
   (a10) the amino acid sequences shown in SEQ ID NOs: 182, 202 and 220
   (a11) the amino acid sequences shown in SEQ ID NOs: 183, 203 and 221
   (a12) the amino acid sequences shown in SEQ ID NOs: 8, 4 and 5
   (a13) the amino acid sequences shown in SEQ ID NOs: 3, 4 and 219
   (a14) the amino acid sequences shown in SEQ ID NOs: 181, 204 and 222
   (a15) the amino acid sequences shown in SEQ ID NOs: 181, 204 and 223
   (a16) the amino acid sequences shown in SEQ ID NOs: 183, 203 and 224
   (a17) the amino acid sequences shown in SEQ ID NOs: 184, 205 and 225
   (a18) the amino acid sequences shown in SEQ ID NOs: 185, 206 and 226
   (a19) the amino acid sequences shown in SEQ ID NOs: 183, 203 and 227
   (a20) the amino acid sequences shown in SEQ ID NOs: 186, 207 and 228
   (a21) the amino acid sequences shown in SEQ ID NOs: 187, 208 and 229
   (a22) the amino acid sequences shown in SEQ ID NOs: 184, 209 and 230
   (a23) the amino acid sequences shown in SEQ ID NOs: 3, 4 and 5
   (a24) the amino acid sequences shown in SEQ ID NOs: 8, 4 and 231
   (a25) the amino acid sequences shown in SEQ ID NOs: 188, 4 and 5
   (a26) the amino acid sequences shown in SEQ ID NOs: 189, 210 and 232
   (a27) the amino acid sequences shown in SEQ ID NOs: 8, 211 and 5
   (a28) the amino acid sequences shown in SEQ ID NOs: 190, 202 and 233
   (a29) the amino acid sequences shown in SEQ ID NOs: 184, 205 and 234
   (a30) the amino acid sequences shown in SEQ ID NOs: 182, 203 and 235
   (a31) the amino acid sequences shown in SEQ ID NOs: 191, 212 and 236
   (a32) the amino acid sequences shown in SEQ ID NOs: 192, 208 and 229
   (a33) the amino acid sequences shown in SEQ ID NOs: 184, 205 and 237
   (a34) the amino acid sequences shown in SEQ ID NOs: 193, 213 and 238
   (a35) the amino acid sequences shown in SEQ ID NOs: 194, 214 and 239
   (a36) the amino acid sequences shown in SEQ ID NOs: 195, 215 and 240
   (a37) the amino acid sequences shown in SEQ ID NOs: 196, 216 and 241
   (a38) the amino acid sequences shown in SEQ ID NOs: 10, 11 and 12
   (a39) the amino acid sequences shown in SEQ ID NOs: 3, 4 and 5
   (a40) the amino acid sequences shown in SEQ ID NOs: 8, 4 and 5
   (a41) the amino acid sequences shown in SEQ ID NOs: 10, 11 and 12.
(8) An antibody or an antigen-binding portion thereof, wherein the heavy chain variable region includes an amino acid sequence selected from SEQ ID NOs: 23 - 25 and 242 - 291.
(9) An antibody or an antigen-binding portion thereof, containing a CDR or CDRs (preferably CDR1 to CDR3) in a heavy chain variable region consisting of an amino acid sequence selected from SEQ ID NOs: 23 - 25 and 242 - 291.
(10) The antibody or an antigen-binding portion thereof described in (7) or (8), which is VHH.

The CDR sequences in the variable regions can be determined by selecting and applying, according to the purpose, an appropriate method from among the known methods already reported. For example, the CDR sequences can be determined by the numbering system of Kabat et al. (Kabat, E. et al., U.S. Department of Health and Human Services, (1983) and subsequent editions), the numbering system of Chothia et al. (Chothia and Lesk, J. Mol. Biol., 196:901-917 (1987)), the numbering system of Honegger et al. (Honegger, A et al., J. Mol. Biol. 309:657-670 (2001)), the contact definition method (MacCallum et al., J Mol Biol, 262 (5):732-745 (1996)), the numbering system according to the IMGT database (http://www.imgt.org/), or by alignment to a known sequence database. Therefore, the antibody of the present invention may be an antibody or antigen-binding portion in which the CDR sequence in the light chain variable region is the CDR sequence in the amino acid sequence shown in SEQ ID NO: 13 and the CDR sequence in the heavy chain variable region is the CDR sequence in the amino acid sequence shown in SEQ ID NO: 14; an antibody or antigen-binding portion in which the CDR sequence in the light chain variable region is the CDR sequence in the amino acid sequence shown in SEQ ID NO: 15 and the CDR sequence in the heavy chain variable region is the CDR sequence in the amino acid sequence shown in SEQ ID NO: 16; or an antibody or antigen-binding portion in which the CDR sequence in the light chain variable region is the CDR sequence in the amino acid sequence shown in SEQ ID NO: 17 and the CDR sequence in the heavy chain variable region is the CDR sequence in the amino acid sequence shown in SEQ ID NO: 18.

In the antibody, etc. of the present invention, as a framework region (FR) of the antibody etc. to be linked to the CDR, one in which the CDR forms a good antigen-binding site is selected. The FR used in the antibody of the present invention is not particularly limited as long as it is FR of a human antibody. The FR of a human antibody may have a natural sequence, or one or more amino acids in the framework region having the natural sequence may be substituted, deleted, added, and/or inserted, etc., as necessary, so that the CDR forms an appropriate antigen-binding site. For example, a mutant FR sequence having desired properties can be selected by measuring and evaluating the antigen-binding activity of an antibody using an FR with substituted amino acids (Sato, K. et al., Cancer Res. (1993) 53, 851-856).

In the present specification, when a specific amino acid sequence is contained as the CDR or when a specific amino acid sequence is contained as the variable region, the CDR or variable region preferably consists of a specific amino acid sequence thereof.

When the antibody, etc. of the present invention has a constant region, the constant region to be used is not particularly limited, and any constant region may be used. Preferred examples of the constant region to be used in the antibody of the present invention include the constant region of a human antibody (such as a constant region derived from IgG1, IgG2, IgG3, IgG4, IgA, or IgM). For example, Cγ1, Cy2, Cγ3, Cy4, Cµ, Cδ, Cα1, Cα2, and Cε can be used for the H chain, and Cκ and Cλ can be used for the L chain. In one embodiment, the constant region used in the antibody, etc. of the present invention contains the amino acid sequence shown in SEQ ID NO: 19.

In one embodiment, examples of the antibody, etc. of the present invention include the following:
three anti-GPA33 scFvs shown in Fig. 5:
   2RC9: SEQ ID NO: 20
   2RC17: SEQ ID NO: 21
   2RC28: SEQ ID NO: 22
three anti-GPA33 scFvs having additional sequences on the C-terminal side shown in Fig. 7:
   2RC9-Cys: SEQ ID NO: 23
   2RC17-Cys: SEQ ID NO: 24
   2RC28-Cys: SEQ ID NO: 25
three anti-GPA33 scFv-Fcs shown in Fig. 11:
   GPA2RC9-Fc: SEQ ID NO: 26
   GPA2RC17-Fc: SEQ ID NO: 27
   GPA2RC28-Fc: SEQ ID NO: 28

In another embodiment of the present invention, the antibody, etc. of the present invention may be VHH (or nanobody). A method for producing a VHH having a desired amino acid sequence is known in the pertinent technique field. Those of ordinary skill in the art can produce a VHH having the same binding properties as the original scFv by replacing FR1(VH), FR2(VH), FR3(VH), and FR4(VH) of the single-chain heavy chain variable region (VH(scFv)) with FR1(VHH), FR2(VHH), FR3(VHH), and FR4(VHH), respectively (converting VH into VHH).

Examples of the VHH that can be used as the antibody, etc. of the present invention include, but are not limited to, those shown in the following paragraphs, and may have one or more (e.g., 1, 2, 3, 4, 5, 6, 7, 8, 9, 10, or more) amino acids added, deleted, substituted, or inserted, as long as the desired binding activity is maintained.
(1) An antibody fragment in which the amino acid sequences of CDR1 to CDR3 of VHH respectively contain the amino acid sequences shown in SEQ ID NOs: 8, 197 and 217
(2) an antibody fragment in which the amino acid sequences of CDR1 to CDR3 of VHH respectively contain the amino acid sequences shown in SEQ ID NOs: 180, 198 and 218
(3) an antibody fragment in which the amino acid sequences of CDR1 to CDR3 of VHH respectively contain the amino acid sequences shown in SEQ ID NOs: 3, 199 and 217
(4) an antibody fragment in which the amino acid sequences of CDR1 to CDR3 of VHH respectively contain the amino acid sequences shown in SEQ ID NOs: 3, 200 and 219
(5) an antibody fragment in which the amino acid sequences of CDR1 to CDR3 of VHH respectively contain the amino acid sequences shown in SEQ ID NOs: 181, 200 and 219
(6) an antibody fragment in which the amino acid sequences of CDR1 to CDR3 of VHH respectively contain the amino acid sequences shown in SEQ ID NOs: 8, 197 and 217
(7) an antibody fragment in which the amino acid sequences of CDR1 to CDR3 of VHH respectively contain the amino acid sequences shown in SEQ ID NOs: 8, 199 and 5
(8) an antibody fragment in which the amino acid sequences of CDR1 to CDR3 of VHH respectively contain the amino acid sequences shown in SEQ ID NOs: 3, 201 and 217
(9) an antibody fragment in which the amino acid sequences of CDR1 to CDR3 of VHH respectively contain the amino acid sequences shown in SEQ ID NOs: 8, 199 and 217
(10) an antibody fragment in which the amino acid sequences of CDR1 to CDR3 of VHH respectively contain the amino acid sequences shown in SEQ ID NOs: 181, 200 and 5
(11) an antibody fragment in which the amino acid sequences of CDR1 to CDR3 of VHH respectively contain the amino acid sequences shown in SEQ ID NOs: 182, 202 and 220
(12) an antibody fragment in which the amino acid sequences of CDR1 to CDR3 of VHH respectively contain the amino acid sequences shown in SEQ ID NOs: 181, 200 and 219
(13) an antibody fragment in which the amino acid sequences of CDR1 to CDR3 of VHH respectively contain the amino acid sequences shown in SEQ ID NOs: 8, 197 and 217
(14) an antibody fragment in which the amino acid sequences of CDR1 to CDR3 of VHH respectively contain the amino acid sequences shown in SEQ ID NOs: 183, 203 and 221
(15) an antibody fragment in which the amino acid sequences of CDR1 to CDR3 of VHH respectively contain the amino acid sequences shown in SEQ ID NOs: 8, 4 and 5
(16) an antibody fragment in which the amino acid sequences of CDR1 to CDR3 of VHH respectively contain the amino acid sequences shown in SEQ ID NOs: 3, 4 and 219
(17) an antibody fragment in which the amino acid sequences of CDR1 to CDR3 of VHH respectively contain the amino acid sequences shown in SEQ ID NOs: 181, 204 and 222
(18) an antibody fragment in which the amino acid sequences of CDR1 to CDR3 of VHH respectively contain the amino acid sequences shown in SEQ ID NOs: 181, 204 and 222
(19) an antibody fragment in which the amino acid sequences of CDR1 to CDR3 of VHH respectively contain the amino acid sequences shown in SEQ ID NOs: 181, 204 and 223
(20) an antibody fragment in which the amino acid sequences of CDR1 to CDR3 of VHH respectively contain the amino acid sequences shown in SEQ ID NOs: 183, 203 and 224
(21) an antibody fragment in which the amino acid sequences of CDR1 to CDR3 of VHH respectively contain the amino acid sequences shown in SEQ ID NOs: 184, 205 and 225
(22) an antibody fragment in which the amino acid sequences of CDR1 to CDR3 of VHH respectively contain the amino acid sequences shown in SEQ ID NOs: 181, 204 and 222
(23) an antibody fragment in which the amino acid sequences of CDR1 to CDR3 of VHH respectively contain the amino acid sequences shown in SEQ ID NOs: 185, 206 and 226
(24) an antibody fragment in which the amino acid sequences of CDR1 to CDR3 of VHH respectively contain the amino acid sequences shown in SEQ ID NOs: 181, 204 and 222
(25) an antibody fragment in which the amino acid sequences of CDR1 to CDR3 of VHH respectively contain the amino acid sequences shown in SEQ ID NOs: 8, 4 and 5
(26) an antibody fragment in which the amino acid sequences of CDR1 to CDR3 of VHH respectively contain the amino acid sequences shown in SEQ ID NOs: 181, 204 and 222
(27) an antibody fragment in which the amino acid sequences of CDR1 to CDR3 of VHH respectively contain the amino acid sequences shown in SEQ ID NOs: 183, 203 and 227
(28) an antibody fragment in which the amino acid sequences of CDR1 to CDR3 of VHH respectively contain the amino acid sequences shown in SEQ ID NOs: 186, 207 and 228
(29) an antibody fragment in which the amino acid sequences of CDR1 to CDR3 of VHH respectively contain the amino acid sequences shown in SEQ ID NOs: 187, 208 and 229
(30) an antibody fragment in which the amino acid sequences of CDR1 to CDR3 of VHH respectively contain the amino acid sequences shown in SEQ ID NOs: 8, 4 and 5
(31) an antibody fragment in which the amino acid sequences of CDR1 to CDR3 of VHH respectively contain the amino acid sequences shown in SEQ ID NOs: 8, 4 and 5
(32) an antibody fragment in which the amino acid sequences of CDR1 to CDR3 of VHH respectively contain the amino acid sequences shown in SEQ ID NOs: 184, 209 and 230
(33) an antibody fragment in which the amino acid sequences of CDR1 to CDR3 of VHH respectively contain the amino acid sequences shown in SEQ ID NOs: 8, 4 and 5
(34) an antibody fragment in which the amino acid sequences of CDR1 to CDR3 of VHH respectively contain the amino acid sequences shown in SEQ ID NOs: 3, 4 and 5
(35) an antibody fragment in which the amino acid sequences of CDR1 to CDR3 of VHH respectively contain the amino acid sequences shown in SEQ ID NOs: 8, 4 and 231
(36) an antibody fragment in which the amino acid sequences of CDR1 to CDR3 of VHH respectively contain the amino acid sequences shown in SEQ ID NOs: 188, 4 and 5
(37) an antibody fragment in which the amino acid sequences of CDR1 to CDR3 of VHH respectively contain the amino acid sequences shown in SEQ ID NOs: 189, 210 and 232
(38) an antibody fragment in which the amino acid sequences of CDR1 to CDR3 of VHH respectively contain the amino acid sequences shown in SEQ ID NOs: 8, 4 and 5
(39) an antibody fragment in which the amino acid sequences of CDR1 to CDR3 of VHH respectively contain the amino acid sequences shown in SEQ ID NOs: 8, 211 and 5
(40) an antibody fragment in which the amino acid sequences of CDR1 to CDR3 of VHH respectively contain the amino acid sequences shown in SEQ ID NOs: 190, 202 and 233
(41) an antibody fragment in which the amino acid sequences of CDR1 to CDR3 of VHH respectively contain the amino acid sequences shown in SEQ ID NOs: 3, 4 and 5
(42) an antibody fragment in which the amino acid sequences of CDR1 to CDR3 of VHH respectively contain the amino acid sequences shown in SEQ ID NOs: 184, 205 and 234
(43) an antibody fragment in which the amino acid sequences of CDR1 to CDR3 of VHH respectively contain the amino acid sequences shown in SEQ ID NOs: 182, 203 and 235
(44) an antibody fragment in which the amino acid sequences of CDR1 to CDR3 of VHH respectively contain the amino acid sequences shown in SEQ ID NOs: 191, 212 and 236
(45) an antibody fragment in which the amino acid sequences of CDR1 to CDR3 of VHH respectively contain the amino acid sequences shown in SEQ ID NOs: 192, 208 and 229
(46) an antibody fragment in which the amino acid sequences of CDR1 to CDR3 of VHH respectively contain the amino acid sequences shown in SEQ ID NOs: 184, 205 and 237
(47) an antibody fragment in which the amino acid sequences of CDR1 to CDR3 of VHH respectively contain the amino acid sequences shown in SEQ ID NOs: 193, 213 and 238
(48) an antibody fragment in which the amino acid sequences of CDR1 to CDR3 of VHH respectively contain the amino acid sequences shown in SEQ ID NOs: 194, 214 and 239
(49) an antibody fragment in which the amino acid sequences of CDR1 to CDR3 of VHH respectively contain the amino acid sequences shown in SEQ ID NOs: 195, 215 and 240
(50) an antibody fragment in which the amino acid sequences of CDR1 to CDR3 of VHH respectively contain the amino acid sequences shown in SEQ ID NOs: 196, 216 and 241,
(51) an antibody fragment in which the amino acid sequences of CDR1 to CDR3 of VHH respectively contain the amino acid sequences shown in SEQ ID NOs: 10, 11 and 12.

In one preferred embodiment, VHH is the following :
(a1) an antibody fragment in which the amino acid sequences of CDR1 to CDR3 of VHH respectively contain the amino acid sequences shown in SEQ ID NOs: 8, 197 and 217
(a2) an antibody fragment in which the amino acid sequences of CDR1 to CDR3 of VHH respectively contain the amino acid sequences shown in SEQ ID NOs: 180, 198 and 218
(a3) an antibody fragment in which the amino acid sequences of CDR1 to CDR3 of VHH respectively contain the amino acid sequences shown in SEQ ID NOs: 3, 199 and 217
(a4) an antibody fragment in which the amino acid sequences of CDR1 to CDR3 of VHH respectively contain the amino acid sequences shown in SEQ ID NOs: 3, 200 and 219
(a5) an antibody fragment in which the amino acid sequences of CDR1 to CDR3 of VHH respectively contain the amino acid sequences shown in SEQ ID NOs: 181, 200 and 219
(a6) an antibody fragment in which the amino acid sequences of CDR1 to CDR3 of VHH respectively contain the amino acid sequences shown in SEQ ID NOs: 8, 199 and 5
(a7) an antibody fragment in which the amino acid sequences of CDR1 to CDR3 of VHH respectively contain the amino acid sequences shown in SEQ ID NOs: 3, 201 and 217
(a8) an antibody fragment in which the amino acid sequences of CDR1 to CDR3 of VHH respectively contain the amino acid sequences shown in SEQ ID NOs: 8, 199 and 217
(a9) an antibody fragment in which the amino acid sequences of CDR1 to CDR3 of VHH respectively contain the amino acid sequences shown in SEQ ID NOs: 181, 200 and 5
(a10) an antibody fragment in which the amino acid sequences of CDR1 to CDR3 of VHH respectively contain the amino acid sequences shown in SEQ ID NOs: 182, 202 and 220
(a11) an antibody fragment in which the amino acid sequences of CDR1 to CDR3 of VHH respectively contain the amino acid sequences shown in SEQ ID NOs: 183, 203 and 221
(a12) an antibody fragment in which the amino acid sequences of CDR1 to CDR3 of VHH respectively contain the amino acid sequences shown in SEQ ID NOs: 8, 4 and 5
(a13) an antibody fragment in which the amino acid sequences of CDR1 to CDR3 of VHH respectively contain the amino acid sequences shown in SEQ ID NOs: 3, 4 and 219
(a14) an antibody fragment in which the amino acid sequences of CDR1 to CDR3 of VHH respectively contain the amino acid sequences shown in SEQ ID NOs: 181, 204 and 222
(a15) an antibody fragment in which the amino acid sequences of CDR1 to CDR3 of VHH respectively contain the amino acid sequences shown in SEQ ID NOs: 181, 204 and 223
(a16) an antibody fragment in which the amino acid sequences of CDR1 to CDR3 of VHH respectively contain the amino acid sequences shown in SEQ ID NOs: 183, 203 and 224
(a17) an antibody fragment in which the amino acid sequences of CDR1 to CDR3 of VHH respectively contain the amino acid sequences shown in SEQ ID NOs: 184, 205 and 225
(a18) an antibody fragment in which the amino acid sequences of CDR1 to CDR3 of VHH respectively contain the amino acid sequences shown in SEQ ID NOs: 185, 206 and 226
(a19) an antibody fragment in which the amino acid sequences of CDR1 to CDR3 of VHH respectively contain the amino acid sequences shown in SEQ ID NOs: 183, 203 and 227
(a20) an antibody fragment in which the amino acid sequences of CDR1 to CDR3 of VHH respectively contain the amino acid sequences shown in SEQ ID NOs: 186, 207 and 228
(a21) an antibody fragment in which the amino acid sequences of CDR1 to CDR3 of VHH respectively contain the amino acid sequences shown in SEQ ID NOs: 187, 208 and 229
(a22) an antibody fragment in which the amino acid sequences of CDR1 to CDR3 of VHH respectively contain the amino acid sequences shown in SEQ ID NOs: 184, 209 and 230
(a23) an antibody fragment in which the amino acid sequences of CDR1 to CDR3 of VHH respectively contain the amino acid sequences shown in SEQ ID NOs: 3, 4 and 5
(a24) an antibody fragment in which the amino acid sequences of CDR1 to CDR3 of VHH respectively contain the amino acid sequences shown in SEQ ID NOs: 8, 4 and 231
(a25) an antibody fragment in which the amino acid sequences of CDR1 to CDR3 of VHH respectively contain the amino acid sequences shown in SEQ ID NOs: 188, 4 and 5
(a26) an antibody fragment in which the amino acid sequences of CDR1 to CDR3 of VHH respectively contain the amino acid sequences shown in SEQ ID NOs: 189, 210 and 232
(a27) an antibody fragment in which the amino acid sequences of CDR1 to CDR3 of VHH respectively contain the amino acid sequences shown in SEQ ID NOs: 8, 211 and 5
(a28) an antibody fragment in which the amino acid sequences of CDR1 to CDR3 of VHH respectively contain the amino acid sequences shown in SEQ ID NOs: 190, 202 and 233
(a29) an antibody fragment in which the amino acid sequences of CDR1 to CDR3 of VHH respectively contain the amino acid sequences shown in SEQ ID NOs: 184, 205 and 234
(a30) an antibody fragment in which the amino acid sequences of CDR1 to CDR3 of VHH respectively contain the amino acid sequences shown in SEQ ID NOs: 182, 203 and 235
(a31) an antibody fragment in which the amino acid sequences of CDR1 to CDR3 of VHH respectively contain the amino acid sequences shown in SEQ ID NOs: 191, 212 and 236
(a32) an antibody fragment in which the amino acid sequences of CDR1 to CDR3 of VHH respectively contain the amino acid sequences shown in SEQ ID NOs: 192, 208 and 229
(a33) an antibody fragment in which the amino acid sequences of CDR1 to CDR3 of VHH respectively contain the amino acid sequences shown in SEQ ID NOs: 184, 205 and 237
(a34) an antibody fragment in which the amino acid sequences of CDR1 to CDR3 of VHH respectively contain the amino acid sequences shown in SEQ ID NOs: 193, 213 and 238
(a35) an antibody fragment in which the amino acid sequences of CDR1 to CDR3 of VHH respectively contain the amino acid sequences shown in SEQ ID NOs: 194, 214 and 239
(a36) an antibody fragment in which the amino acid sequences of CDR1 to CDR3 of VHH respectively contain the amino acid sequences shown in SEQ ID NOs: 195, 215 and 240
(a37) an antibody fragment in which the amino acid sequences of CDR1 to CDR3 of VHH respectively contain the amino acid sequences shown in SEQ ID NOs: 196, 216 and 241
(a38) an antibody fragment in which the amino acid sequences of CDR1 to CDR3 of VHH respectively contain the amino acid sequences shown in SEQ ID NOs: 10, 11 and 12.

In one embodiment of the present invention, the present invention provides an intestinal tissue targeting agent (hereinafter sometimes referred to as the "intestinal tissue targeting agent of the present invention") that contains the antibody, etc. of the present invention. The antibody, etc. of the present invention specifically bind to GPA33, which is highly expressed in intestinal tissues such as small intestine and large intestine, and therefore selectively accumulate in intestinal tissues. By utilizing this property, they can be used as agents that target intestinal tissues. As long as the intestinal tissue targeting agent of the present invention contains the antibody, etc. of the present invention as an active ingredient, other components are not particularly limited and may contain any components. Furthermore, the intestinal tissue targeting agent of the present invention may be used for any purpose in testing research and/or disease treatment.

In one embodiment of the present invention, the antibody, etc. of the present invention may be a conjugate fused with a functional molecule (hereinafter sometimes referred to as the "conjugate of the present invention"). In the present specification, conjugate means that the antibody of the present invention and another molecule are bound by a covalent bond or other bond via a linker or the like as necessary to constitute one type of active ingredient.

The functional molecule used in the present invention is not particularly limited as long as it does not inhibit the binding activity of the antibody, etc. of the present invention to human and mouse GPA33. For example, in order to easily purify the antibody, etc. of the present invention expressed in Escherichia coli, an affinity tag (His tag, FLAG tag, etc.) can be fused to the antibody, etc. of the present invention. As a method for producing such fusion protein, a method known per se can be used. For example, a method including linking a polynucleotide encoding the antibody, etc. of the present invention to a polynucleotide encoding another peptide or polypeptide in a frame-matching manner, introducing same into an expression vector, and expressing same in a host can be exemplified.

As described above, the antibody, etc. of the present invention specifically bind to GPA33, which is highly expressed in intestinal tissues such as the small intestine and large intestine, and therefore selectively accumulate in intestinal tissues. By utilizing such properties, in another aspect, the antibody, etc. of the present invention can be fused with functional molecules for examination or drugs for treating diseases to selectively and efficiently deliver such molecules to intestinal tissue. Thus, the conjugate of the present invention can be used as an agent for examining intestinal tissues or treating intestinal diseases, or an agent for promoting regeneration of and/or repairing intestinal tissues. In the present specification, the intestine includes the small intestine (duodenum, jejunum, ileum) and the large intestine (cecum, colon, rectum). In the present specification, "intestinal disease" means a disease characterized by a lesion in a localized intestine, and examples thereof include inflammatory bowel diseases such as Crohn's disease, ulcerative colitis, drug-induced enteritis, infectious enteritis, ischemic enteritis, and specific inflammatory bowel disease, irritable bowel syndrome, appendicitis, intestinal tuberculosis, intestinal obstruction, colon polyps, colonic polyposis, diverticulosis, chronic constipation, chronic diarrhea, malabsorption syndrome, and the like. In the present specification, regeneration of intestinal tissue means that a new intestinal tissue can be formed at the site where intestinal tissue was lost, and promotion of regeneration of intestinal tissue means that the formation of such new intestinal tissue is caused, the amount is increased, or formation is performed in a short period of time. In addition, repair of intestinal tissue means that a damaged intestinal tissue returns to a normal intestinal tissue.

Examples of the functional molecules for examination include, but are not limited to, radioactive substance, fluorescent substance, luminescent substance, enzyme, and the like. In addition, examples of drugs for treating diseases when targeting cancer include anticancer drugs such as alkylating agent, nitrosourea, metabolic antagonist, antiviral agent, antibiotic, plant alkaloid, topoisomerase inhibitor, tubulin polymerization inhibitor, hormone therapy agent, hormone antagonist, aromatase inhibitor, P-glycoprotein inhibitor, platinum complex derivative, M-phase inhibitor, and kinase inhibitor, and when targeting inflammatory bowel disease include, but are not limited to, agents for treating inflammatory bowel diseases such as 5-aminosalicylic acid preparation, steroid, immunosuppressant, TNF inhibitor, integrin inhibitor, and intestinal cell proliferation promoter.

In addition, examples of the agent for treating each inflammatory bowel disease include, but are not limited to, 5-aminosalicylic acid preparations such as salazosulfapyridine, sulfasalazine, and mesalazine; steroids such as betamethasone, betamethasone sodium phosphate, betamethasone phosphate, and prednisolone; immunosuppressants such as azathioprine, tacrolimus, 6-mercaptopurine, and cyclosporine; TNF inhibitors such as infliximab, adalimumab, certolizumab, and pegol; integrin inhibitors such as natalizumab; intestinal cell proliferation promoters such as R-spondin 1, 2, 3, or 4, or partial fragments thereof (partial fragments having equivalent intestinal cell proliferation promoting activity as wild-type R-spondin 1, 2, 3, or 4, for example, R-spondin CRD fragment), and active portions thereof.

Methods for preparing conjugates of such functional molecule and antibody (or antigen-binding portion) have already been established in this technique field (see, for example, J Natl Cancer Inst. 2019 Jun 1; 111(6):538-549., Molecules. 2021 May 15; 26(10):2943., US5057313, US5156840, and the like).

Alternatively, in one preferred embodiment of the present invention, a conjugate of a functional molecule and an antibody (or antigen-binding portion) may be prepared by the "CCAP (Chemical conjugation by affinity peptide) method".

The CCAP method is an antibody site-specific modification technique developed by the present inventors, and is a technique for site-specifically modifying an IgG antibody based on a peptide that specifically binds to the Fc region of an IgG antibody (sometimes referred to as "IgG-binding peptide", "IgG-BP" or "IgBP", etc. in the present specification). According to the CCAP method, an IgG antibody to which a functional molecule is linked can be prepared extremely conveniently while maintaining the original antigen-binding activity. Those of ordinary skill in the art can appropriately perform the CCAP method by referring to Kishimoto, S., et al. (2019) Bioconjugate Chemistry, 30, 698-702, JP-B-6872181, WO2018/230257, WO2021/080008, and the like.

When the CCAP method is used for preparing the conjugate of the present invention, there is no particular limitation on the IgBP that can be used. IgBP is preferably a peptide that binds to a site selected from Lys248, Lys246, Lys338, Lys288, Lys290, Lys360, Lys414, and Lys439 and/or adjacent region thereof according to Eu numbering in Fc, preferably Lys248 and/or adjacent region thereof, or a peptide that binds to the binding region of Protein A. For example, IgBP may be a partial peptide of Protein A or a variant thereof having Fc binding ability. Specific examples of such peptides are described in International Patent Publications WO2008/054030, WO2013/027796, WO2016/186206, and WO2018/230257, and Kyohei Muguruma et al., ACS Omega (2019); 4(11):14390-14397. They can be appropriately prepared according to the methods described in each document.

Alternatively, in one preferred embodiment of the present invention, a conjugate of a functional molecule and an antibody (or antigen-binding portion) can also be prepared using the tCAP method. The tCAP method is outlined below. The tCAP method is an improved technology of the CCAP method, and terms such as "IgBP" have the same meaning.

A conjugate prepared by the tCAP method has a functional molecule bound monovalently or divalently to Lys in the Fc region of IgG, via an atomic group containing Lys-Gly-Gly. For example, the amino group of the side chain of Lys contained in the aforementioned atomic group may be substituted by a functional molecule.

The tCAP method is a method for reacting a compound represented by the following formula I or a salt thereof with IgG: 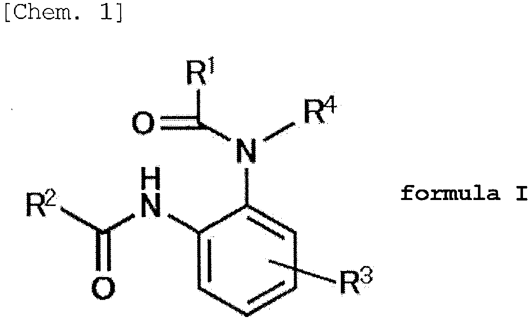 (in the formula I, R¹ is a substituent that affords pKa 4 - 14 for R¹-H,
one of R² and R³ has an IgG-binding peptide that specifically binds to the Fc region of IgG,
when R² has the aforementioned IgG-binding peptide, R³ is absent or is selected from the group consisting of a substituted or unsubstituted alkyl group having 1 to 8 carbon atoms, a substituted or unsubstituted alkenyl group having 2 to 8 carbon atoms, a substituted or unsubstituted alkynyl group having 2 to 8 carbon atoms, a nitro group, a halogen, and a carboxamide group,
when R³ has the aforementioned IgG-binding peptide, R² is selected from the group consisting of a substitution or unsubstituted alkyl group having 1 to 8 carbon atoms, a substituted or unsubstituted alkenyl group having 2 to 8 carbon atoms, a substituted or unsubstituted alkynyl group having 2 to 8 carbon atoms, a substituted or unsubstituted peptide chain having 2 to 50 amino acid residues, a substituted or unsubstituted polymer chain having a degree of polymerization of 2 to 50, and combinations thereof, and
R⁴ is H or a substituted or unsubstituted alkyl group having 1 to 8 carbon atoms).

The compound shown in formula I or a salt thereof may preferably be a compound selected from the following (1) to (6) or a salt thereof:
(1) R¹ is a nitrophenoxy group.
(2) R² has the aforementioned IgG-binding peptide, and R³ is absent.
(3) R² is shown in the following formula II, with the aforementioned IgG-binding peptide as R^{2a}. 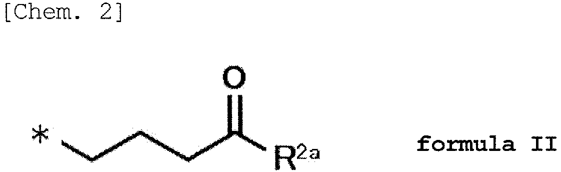
(in the formula II, * indicates a carbon of the carbonyl group of the formula I.)
(4) When R² has the aforementioned IgG-binding peptide, the IgG-binding peptide is modified with a functional molecule.
(5) R³ has the aforementioned IgG-binding peptide, and R² has a functional molecule.
(6) R³ is represented by any one of the following formulas III, IV, and V, with the aforementioned IgG-binding peptide as R^{3a}. 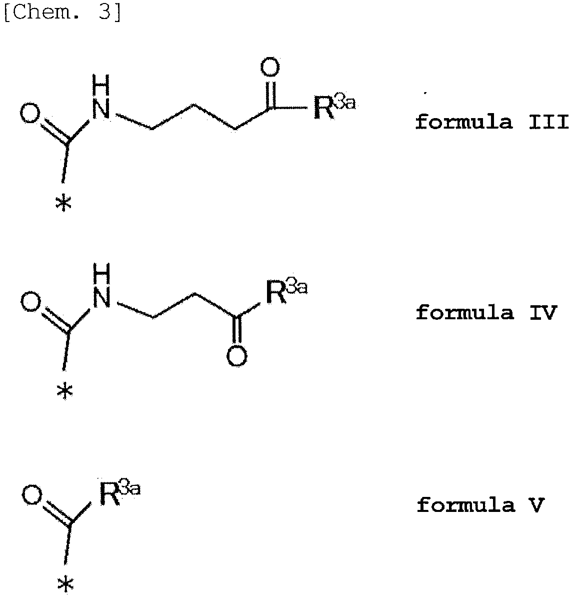
(in the formula III, the formula IV, and the formula V, * indicates a carbon of the benzene ring in the formula I.)

In the tCAP method, the amino acid sequence of the IgG-binding peptide is, for example, any one of the amino acid sequences shown in SEQ ID NOs: 1 to 151. The amino acid sequence of the aforementioned IgG-binding peptide may be any one of the amino acid sequences shown in SEQ ID NOs: 116 to 151.

In one embodiment, in the formula I, R¹ is a substituent that affords an acid dissociation constant (pKa) of 4 to 14, 4 to 12, or 4 to 10, preferably 5 to 9, for R¹-H. The pKa here is pKa at room temperature (e.g., 25°C). The above-mentioned compound becomes an activated intermediate by elimination of R¹. The pKa of R¹-H determines the ease of elimination, i.e., the ease of transfer to an activated intermediate. When the pKa is high, the transition becomes difficult, and when the pKa is low, the transition becomes easy, but when the pKa is too low, the compound is unstable and may be hydrolyzed. Preferably, R¹-H is a phenol. Examples of R¹-H include salicylic acid, phenol, 4-fluorophenol, 4-nitrophenol, 2,6-dichlorophenol, N-hydroxysuccinimide, 2,3,5,6-tetrafluorophenol, pentafluorophenol, aminophenol, and dinitrophenol. Preferably, R¹-H is 4-nitrophenol and R¹ is a nitrophenoxy group.

One of R² and R³ has IgG-BP that specifically binds to the Fc region of IgG. "IgG" may be IgG of mammals, for example, primates such as human, chimpanzee and the like, experimental animals such as rat, mouse, rabbit and the like, domestic animals such as swine, bovine, horse, sheep, goat and the like, and pet animals such as dog, cat and the like, and human IgG (IgG1, IgG2, IgG3 or IgG4) is preferred. As IgG, human IgG1, IgG2, or IgG4, or rabbit IgG is preferred and human IgG1, IgG2, or IgG4 is particularly preferred. "Fc region of IgG" typically refers to a C-terminal fragment of IgG obtained by treating IgG with protease papain.

When R² has an IgG-BP, R³ is absent or selected from the group consisting of a substituted or unsubstituted alkyl group having 1 to 8 carbon atoms, a substituted or unsubstituted alkenyl group having 2 to 8 carbon atoms, a substituted or unsubstituted alkynyl group having 2 to 8 carbon atoms, a nitro group, a halogen, and a carboxamide group. Preferably, when R² has IgG-BP, R³ is absent.

The "alkyl group" means a saturated hydrocarbon group. Alkyl group includes cyclic (including fused bicyclic) alkyl groups that satisfy the carbon number requirement, straight-chain and branched-chain alkyl groups, and straight-chain or branched-chain alkyl groups substituted by a cyclic alkyl group. Examples of the alkyl group include methyl group, ethyl group, n-propyl group, iso-propyl group, cyclopropyl group, n-butyl group, iso-butyl group, sec-butyl group, t-butyl group, n-pentyl group, 1,1-dimethylpropyl group, 1,2-dimethylpropyl group, 2,2-dimethylpropyl group, 1-ethylpropyl group, 2-ethylpropyl group, n-hexyl group, cyclohexyl group, cyclooctyl group, and 1-methyl-2-ethylpropyl group.

The "alkenyl" means a hydrocarbon group having at least one double bond. Alkenyl includes both straight-chain alkenyl group and branched-chain alkenyl group. Examples of the alkenyl include ethenyl group, n-propenyl group, iso-propenyl group, n-butenyl group, iso-butenyl group, sec-butenyl group, t-butenyl group, n-pentenyl group, 1,1-dimethylpropenyl group, 1,2-dimethylpropenyl group, 2,2-dimethylpropenyl group, 1-ethylpropenyl group, 2-ethylpropenyl group, n-hexenyl group and 1-methyl-2-ethylpropenyl group.

The "alkynyl" means a hydrocarbon group having at least one triple bond. Alkynyl includes both straight-chain alkynyl group and branched-chain alkynyl group. Examples of the alkynyl include ethynyl group, n-propynyl group, iso-propynyl group, n-butynyl group, iso-butynyl group, sec-butynyl group, t-butynyl group, n-pentynyl group and the like.

When R² has IgG-BP, R² may be IgG-BP. R² may also have a linker, and R² may be *-(Linker)-(IgG-BP) wherein * is the carbon of the carbonyl group in the formula I. The linker is not particularly limited as long as the compound maintains specific binding ability to the Fc region, and, for example, selected from the group consisting of -NH-, -O-, -S-, -C(=O)-NH-, -NH-C(=O)-, -O-, -C(=O)-O-, -O-C(=O)-, -S-, -C(=O)-, polyoxyalkylene group, amino acid residue, peptide chain, polyethylene glycol (PEG) chain and combinations thereof.

Preferably, R² is shown in the formula II, with IgG-BP as R^{2a}.

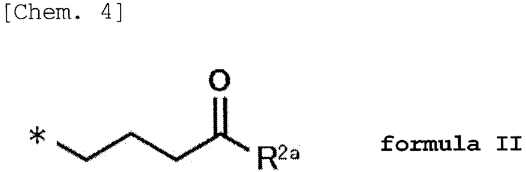

The IgG-BP in R² is bound to the carbon of the carbonyl group of the formula I or a linker via the main chain or the side chain of any amino acid residue. Preferably, the IgG-BP in R² is bound to the carbon of the carbonyl group of the formula I or a linker via an amino group of the main chain N-terminus or an amino group of a side chain.

The IgG-BP in R² may be modified with a functional molecule.

In a reaction in which IgG is modified with a compound whose R² has IgG-BP, the compound has an active ester precursor shown in the formula I, and therefore, IgG-BP can be added to a given amino acid residue, particularly a Lys residue, of IgG via an activated compound under neutral conditions, preferably weakly alkaline conditions (Fig. 24).

When R³ has IgG-BP, R² is selected from the group consisting of a substituted or unsubstituted alkyl group having 1 to 8 carbon atoms, a substituted or unsubstituted alkenyl group having 2 to 8 carbon atoms, a substituted or unsubstituted alkynyl group having 2 to 8 carbon atoms, a substituted or unsubstituted peptide chain having 2 to 50 amino acid residues, a substituted or unsubstituted polymer chain having a degree of polymerization of 2 to 50, and combinations thereof. The number of amino acid residues in the peptide chain is preferably 2 to 30, 2 to 20, 2 to 10, or 2 to 5, more preferably 3. A concrete peptide chain is Lys-Gly-Gly. The polymer chain is not particularly limited and may be any known polymer chain, for example, PEG and the like. The substituted peptide chain includes those in which a substituent is bonded to the main chain or side chain. The substituted polymer chain includes those in which a substituent is bonded to the main chain or side chain.

When R³ has IgG-BP, R³ may be IgG-BP. R³ may also have a linker, like the above-mentioned R².

The IgG-BP in R³ is bonded to the carbon of the benzene ring in the formula I or to a linker via the main chain or a side chain of any amino acid residue. Preferably, IgG-BP in R³ is bound to the carbon of the carbonyl group in the formula I or to a linker via an amino group in the main chain N-terminus or an amino group of a side chain.

Preferably, R³ is shown by any of the formula III, the formula IV and the formula V, with IgG-BP as R^{3a}. In the formulas III, IV, and V, * indicates a carbon of the benzene ring in the formula I.

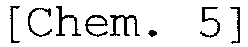

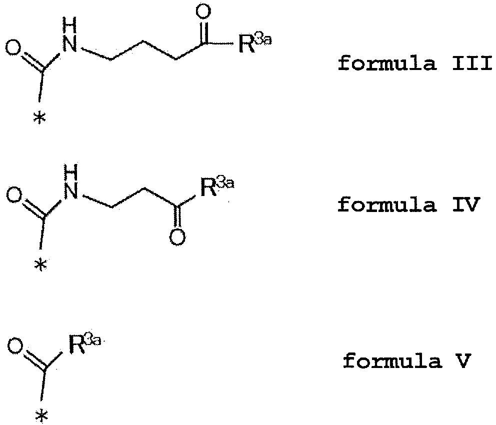

When R³ has an IgG-binding peptide, R² may have a functional molecule. The functional molecule in R² is bound to, for example, an alkyl group, an alkenyl group, an alkynyl group, a peptide chain, or a polymer chain. When R² has, for example, Lys-Gly-Gly as a peptide chain, the amino group of the side chain of Lys in the peptide chain may be substituted by a functional molecule.

A reaction in which IgG is modified with a compound in which R³ has IgG-BP is shown in Fig. 25. Since the compound has an active ester precursor shown in the formula I, it binds to IgG via an activated compound. Unlike the case in which R² has IgG-BP, the compound in which R³ has IgG-BP has a substituent containing R² added to a given amino acid residue of IgG, and R³ containing IgG-BP is not added to IgG. Therefore, when R³ has IgG-BP, R² preferably has the aforementioned functional molecule. In this way, IgG can be easily modified with a functional molecule.

IgG-BP is preferably a peptide that binds to a site selected from Lys248, Lys246, Lys338, Lys288, Lys290, Lys360, Lys414, and Lys439 or adjacent region thereof according to Eu numbering in Fc, preferably Lys248 or adjacent region thereof, or that binds to the binding region of Protein A. For example, IgG-BP may be a partial peptide of Protein A or a variant thereof having Fc binding ability. Specific examples of such peptides are described in WO 2008/054030, WO 2013/027796, the above-mentioned Patent Literatures 1 - 3 and the like. IgG-BP can be appropriately prepared according to a known peptide synthesis method, for example, solid-phase peptide synthesis method, or the methods described in each document.

More specifically, R² and R³ are shown in the following (i) to (iii).
(i) Substituent containing IgG-BP which is represented by the following formula (PI):
   NH₂-(Linker)-(X¹₁₋₃)-C-(X²)-(X³)-(X⁴)-(X⁵)-G-(X⁶)-L-(X⁷)-W-C-(X⁸₁₋₃)-(Linker) formula (PI)
   [in formula (PI), each (Linker) is independently the same or different linker, or is absent, and the linker is RRRGS, EEGGS, (GSGGS)₁₋₃ or (PEG)₁₋₁₀, preferably (PEG)₁₋₈ or (PEG)₂₋₁₀, more preferably (PEG)₄,
      X¹ in the number of 1 to 3, X², X³, X⁴, X⁵, X⁶, X⁷, and X⁸ in the number of 1 to 3 are each independently the same or different amino acid residues,
      each of X¹, X², X³ and each X⁸ are each independently the same or different any amino acid residues other than C,
      X⁴ is H, R, S, or D,
      X⁵ is one amino acid residue selected from K, C, D, E, R, V, F, L, 2-aminosuberic acid, Dpr, Orn, AcOrn, AcDab, Dab, Nle, Nva, Tle, Ala(t-Bu), and Cha,
      X⁶ is E, N, R, or D, and
      X⁷ is I or V.
      Dab, Tle and Cha mean 2,4-diaminobutanoic acid, tert-leucine and β-cyclohexyl-L-alanine, respectively. Ac and t-Bu mean an acetyl group and a tert-butyl group, respectively.]

In the formula (PI), for the purpose of binding, an amino group may be bound to the end (-COOH) of the C-terminal amino acid residue of the formula (PI) to form a (-C(=O)NH₂) group. In the formula (PI), the amino group at the N-terminus may be acetylated. In this case, a Lys residue may be introduced into an appropriate position near the N-terminus in the Linker.

IgG-BP contained in the substituent represented by the formula (PI) preferably includes the following peptides:
[1] X¹₁₋₃ is an amino acid sequence shown by (S, G, F, or absent)-(D, G, A, S, P, Hcy, or absent)-(S, D, T, N, E, or R).
[2] X¹₁₋₃ is D, GPD, R, GPR, SPD, GDD, GPS, SDD, RGN, G-Hcy-D, RGP, or GPD.
[3] X¹₁₋₃ is D or GPD.
[4] X² is A, S, or T.
[5] X² is A or T.
[6] X² is A.
[7] X³ is Y or W.
[8] X³ is Y.
[9] X⁴ is H.
[10] X⁵ is one amino acid residue selected from A, R, K, C, D, E, L, 2-aminosuberic acid, Dpr, R, F, 2-aminosuberic acid, Dpr, AcOrn, AcDab, Dab, Nle, Nva, Ala(t-Bu), and Cha.
[11] X⁵ is K, R, AcOrn.
[12] X⁵ is one amino acid residue selected from V, Dab, F, R, L, Nva, Nle, Ala(t-Bu), and Cha.
[13] X⁵ is one amino acid residue selected from F, R, L, Nva, Nle, Ala(t-Bu), and Cha.
[14] X⁵ is one amino acid residue selected from L, Ala(t-Bu), and Cha.
[15] X⁶ is E or N.
[16] X⁶ is E.
[17] X⁷ is V.
[18] X⁸₁₋₃ is (S, T, or D)-(H, G, Y, T, N, D, F, Hcy, or absent)-(Y, F, H, M, or absent).
[19] X⁸₁₋₃ is T, TFH, S, SFH, THH, TFY, TYH, or T-Hcy-H.
[20] X⁸₁₋₃ is T or TFH.

IgG-BP contained in the substituent represented by the formula (PI) may be any one or a combination of two or more of the above conditions, and may be, for example, a peptide satisfying the conditions described below: [8] and [9]; [8] and [17]; [9] and [17]; [8], [9] and [17]; or a combination of these and any one of [10] to [14].

More specifically, the following peptides can be mentioned as IgG-BP (X⁵ is the same as above; it may have an NH₂-(Linker)- group at the N-terminus; it may have an -NH₂ group or -(Linker)-NH₂ group at the C-terminus):
1) DCAYHX⁵GELVWCT (SEQ ID NO: 29)
2) GPDCAYHX⁵GELVWCTFH (SEQ ID NO: 30)
3) RCAYHX⁵GELVWCS (SEQ ID NO: 31)
4) GPRCAYHX⁵GELVWCSFH (SEQ ID NO: 32)
5) SPDCAYHX⁵GELVWCTFH (SEQ ID NO: 33)
6) GDDCAYHX⁵GELVWCTFH (SEQ ID NO: 34)
7) GPSCAYHX⁵GELVWCTFH (SEQ ID NO: 35)
8) GPDCAYHX⁵GELVWCSFH (SEQ ID NO: 36)
9) GPDCAYHX⁵GELVWCTHH (SEQ ID NO: 37)
10) GPDCAYHX⁵GELVWCTFY (SEQ ID NO: 38)
11) SPDCAYHX⁵GELVWCTFY (SEQ ID NO: 39)
12) SDDCAYHX⁵GELVWCTFY (SEQ ID NO: 40)
13) RGNCAYHX⁵GQLVWCTYH (SEQ ID NO: 41)
14) G-Hcy-DCAYHX⁵GELVWCT-Hcy-H (SEQ ID NO: 42)
15) RRGPDCAYHX⁵GELVWCTFH (SEQ ID NO: 43)
16) DCTYHX⁵GNLVWCT (SEQ ID NO: 44)
17) DCAYHX⁵GNLVWCT (SEQ ID NO: 45)
18) DCTYHX⁵GELVWCT (SEQ ID NO: 46)
19) DCAWHX⁵GELVWCT (SEQ ID NO: 47)
20) DCTYTX⁵GNLVWCT (SEQ ID NO: 48)
21) DCAYTX⁵GNLVWCT (SEQ ID NO: 49)
22) DCSYTX⁵GNLVWCT (SEQ ID NO: 50)
23) DCTWTX⁵GNLVWCT (SEQ ID NO: 51)
24) DCTYHX⁵GNLVWCT (SEQ ID NO: 52)
25) DCTYRX⁵GNLVWCT (SEQ ID NO: 53)
26) DCTYSX⁵GNLVWCT (SEQ ID NO: 54)
27) DCTYTX⁵GNLVWCT (SEQ ID NO: 55)
28) DCTYTX⁵GELVWCT (SEQ ID NO: 56)
29) DCTYTX⁵GRLVWCT (SEQ ID NO: 57)
30) DCTYTX⁵GDLVWCT (SEQ ID NO: 58)
31) DCTYTX⁵GNLIWCT (SEQ ID NO: 59)
32) DCAYHRGELVWCT (SEQ ID NO: 60)
33) GPDCAYHRGELVWCTFH (SEQ ID NO: 61)
34) RCAYHRGELVWCS (SEQ ID NO: 62)
35) GPRCAYHRGELVWCSFH (SEQ ID NO: 63)
36) SPDCAYHRGELVWCTFH (SEQ ID NO: 64)
37) GDDCAYHRGELVWCTFH (SEQ ID NO: 65)
38) GPSCAYHRGELVWCTFH (SEQ ID NO: 66)
39) GPDCAYHRGELVWCSFH (SEQ ID NO: 67)
40) GPDCAYHRGELVWCTHH (SEQ ID NO: 68)
41) GPDCAYHRGELVWCTFY (SEQ ID NO: 69)
42) SPDCAYHRGELVWCTFY (SEQ ID NO: 70)
43) SDDCAYHRGELVWCTFY (SEQ ID NO: 71)
44) DCTYHRGNLVWCT (SEQ ID NO: 72)
45) DCAYHRGNLVWCT (SEQ ID NO: 73)
46) DCTYHRGELVWCT (SEQ ID NO: 74)
47) DCAWHRGELVWCT (SEQ ID NO: 75)
48) DCTYTNGNLVWCT (SEQ ID NO: 76)
49) DCAYTNGNLVWCT (SEQ ID NO: 77)
50) DCSYTNGNLVWCT (SEQ ID NO: 78)
51) DCTWTNGNLVWCT (SEQ ID NO: 79)
52) DCTYHNGNLVWCT (SEQ ID NO: 80)
53) DCTYRNGNLVWCT (SEQ ID NO: 81)
54) DCTYSNGNLVWCT (SEQ ID NO: 82)
55) DCTYTRGNLVWCT (SEQ ID NO: 83)
56) DCTYTNGELVWCT (SEQ ID NO: 84)
57) DCTYTNGRLVWCT (SEQ ID NO: 85)
58) DCTYTNGDLVWCT (SEQ ID NO: 86)
59) DCTYTNGNLIWCT (SEQ ID NO: 87)

Examples of R² and R³ include substituents having the following structures.
60)GSGGS-GPDCAYHRGELVWCTFH-NH₂ (SEQ ID NO: 88)
(PEG)₄-GPDCAYHRGELVWCTFH-NH₂ (SEQ ID NO: 61)
61)GSGGS-DCAYHRGELVWCT-NH₂ (SEQ ID NO: 89)
(PEG)₄-DCAYHRGELVWCT-NH₂ (SEQ ID NO: 60)

In the present specification, peptide and IgG-BP include peptides to which a functional group Z is bound. For example, the substituent represented by the formula (PI) may have a functional group Z bound to the end of (Linker). Examples of such substituent include the following substituents.
62) Acetyl-K(Z)-RRRGS-GPDCAYHKGELVWCTFH-NH₂ (SEQ ID NO: 90)
63) Acetyl-K(Z)-EEGGS-GPDCAYHKGELVWCTFH-NH₂ (SEQ ID NO: 91)
64) Acetyl-K(Z)-(PEG)₄-GPDCAYHKGELVWCTFH-NH₂ (SEQ ID NO: 92)

R² and R³ may be substituents in which a maleimide group, a DBCO group, a tetrazine group or a TCO group is bound to the N-terminus or PEG of the following peptides, or substituents in which an NH₂ group is bound to the C-terminus.
65) RRRGS-GPDCAYHKGELVWCTFH (SEQ ID NO: 93)
66) EEGGS-GPDCAYHKGELVWCTFH (SEQ ID NO: 94)
(PEG)₄-GPDCAYHKGELVWCTFH (SEQ ID NO: 92)

Furthermore, the following peptides can be recited as substituents having a functional group Z bound to the end of (Linker).
67) Acetyl-K(Z)RRRGS-DCAYHKGELVWCT-NH₂ (SEQ ID NO: 95)
68) Acetyl-K(Z)EEGGS-DCAYHKGELVWCT-NH₂ (SEQ ID NO: 96)
69) Acetyl-K(Z)-(PEG)₄-DCAYHKGELVWCT-NH₂ (SEQ ID NO: 97)

R² and R³ may be substituents in which a maleimide group, a DBCO group, a tetrazine group or a TCO group is bound to the N-terminus or PEG of the following peptides, or substituents in which an NH₂ group is bound to the C-terminus.
70) RRRGS-DCAYHKGELVWCT (SEQ ID NO: 98)
71) EEGGS-DCAYHKGELVWCT (SEQ ID NO: 99)
(PEG)₄-DCAYHKGELVWCT (SEQ ID NO: 98)
(ii) A substituent containing IgG-BP represented by the following formula (PII), or a substituent containing IgG-BP consisting of an amino acid sequence in which one or several amino acids are added, deleted, and/or substituted at positions other than X⁹ to X¹⁴ in the amino acid sequence of (PII):

X⁹₁₋₂NMQX¹⁰QX¹⁴RFYEALHDPNLNEEQRNAX¹¹IX¹²SIRDDX¹³-(Linker2)-CONH₂ (PII)

[in the formula (PII), (Linker2) is (GSGGS)₁₋₃, (SGSGS)₁₋₃, or (PEG)₂₋₁₀-Lys (preferably, (PEG)₄-Lys), SGSGSK, SRRCR, SRRK(Z)R, SRRCRRCRRC, SRRK(Z)RRK(Z)RRK(Z), or (PEG)₁₋₈-Lys (preferably, (PEG)₄-Lys), or absent,
X⁹₁₋₂ is selected from the group consisting of GF, AF, VF, LF, IF, MF, PF, FF, WF, KF, Orn-F, CF, DF, EF, beta-alanine-F, 2-aminosuberic acid-F, Dpr-F, NH₂-(PEG)ₙ-CO (wherein n=1-50)-F, F, K, Orn, C, D, E, 2-aminosuberic acid, Dpr, and Acetyl-K,
X¹⁰ is C or Q,
X¹¹ and X¹² are each independently selected from the group consisting of R, H, D, E, S, T, N, Q, Y, and C,
X¹³ is C or P, or absent, and
X¹⁴ is R, C, K, or K(Z)].

The end (-NH₂) of the N-terminal amino acid of the formula (PII) may be acetylated to form a (CH₃-C(=O)-NH-) group. In addition, the Cys residue (C) contained in the linker may be bonded to another functional molecule via a maleimide group, where necessary.

IgG-BP contained in the substituent represented by the formula (PII) includes the following peptides.
[21] X⁹ is selected from the group consisting of GF, AF, βAlaF, NH₂-(PEG)ₙ-CO (n=2-10)-F, F, K, Orn, C, Dpr, and Acetyl-K.
[22] X⁹ is selected from the group consisting of GF, F, K, and Acetyl-K.
[23] X¹⁰ is Q.
[24] X¹¹ and X¹² are each independently selected from the group consisting of R, H, and E.
[25] X¹¹ is R.
[26] X¹² is R or K(Z) (preferably, Z is azide).

More specific examples of the substituent represented by the formula (PII) include the following substituents (the Lys residue contained therein may be bound to a functional group as necessary):
72) FNMQCQRRFYEALHDPNLNEEQRNARIRSIRDDC (SEQ ID NO: 100)
73) GFNMQCQRRFYEALHDPNLNEEQRNARIRSIRDDC (SEQ ID NO: 101)
74) KNMQCQRRFYEALHDPNLNEEQRNARIRSIRDDC (SEQ ID NO: 102)
75) GFNMQCQKRFYEALHDPNLNEEQRNARIRSIRDDC (SEQ ID NO: 103)
76) KNMQCQKRFYEALHDPNLNEEQRNARIRSIRDDC (SEQ ID NO: 104)
77) FNMQQQRRFYEALHDPNLNEEQRNARIRSIRDD (SEQ ID NO: 105)
78) GKNMQCQRRFYEALHDPNLNEEQRNARIRSIRDDC (SEQ ID NO: 106)
79) Acetyl-FNMQQQRRFYEALHDPNLNEEQRNARIRSIRDDP-SGSGSK-NH₂ (SEQ ID NO: 107)
80) Acetyl-FNMQCQRRFYEALHDPNLNEEQRNARIRSIRDDC-SGSGSK-NH₂ (SEQ ID NO: 108)
81) Acetyl-FNMQQQRRFYEALHDPNLNEEQRNARIRSIRDDP-(PEG)₄-Lys-NH₂ (SEQ ID NO: 109)
72-2) Acetyl-FNMQCQRRFYEALHDPNLNEEQRNARIRSIRDDC-(PEG)₄-Lys-NH₂ (SEQ ID NO: 100)
82) FNMQQQCRFYEALHDPNLNEEQRNARIRSIRDD-NH₂ (SEQ ID NO: 110)
83) FNMQQQRRFYEALHDPNLNEEQRNARIRSIRDDC-NH₂ (SEQ ID NO: 111)
84) FNMQQQRRFYEALHDPNLNEEQRNARIRSIRDDP-SRRK(Z)R-NH₂ (SEQ ID NO: 112)
85) FNMQQQRRFYEALHDPNLNEEQRNARIRSIRDDP-SRRCR-NH₂ (SEQ ID NO: 113)
86) FNMQQQCRFYEALHDPNLNEEQRNARICSIRDDP-SRRCRRCRRC-NH₂ (SEQ ID NO: 114) 
87) FNMQQQK(Z)RFYEALHDPNLNEEQRNARIK(Z)SIRDDP-
SRRK(Z)RRK(Z)RRK(Z)-NH₂ (SEQ ID NO: 115)
88) Acetyl-KNMQQQCRFYEALHDPNLNEEQRNARIRSIRDD-NH₂ (SEQ ID NO: 116)
89) Acetyl-KNMQQQRRFYEALHDPNLNEEQRNARIRSIRDDC-NH₂ (SEQ ID NO: 117)
90) Acetyl-KNMQQQRRFYEALHDPNLNEEQRNARIRSIRDDP-SRRK(Z)R-NH₂ (SEQ ID NO: 118)
91) Acetyl-KNMQQQRRFYEALHDPNLNEEQRNARIRSIRDDP-SRRCR-NH₂ (SEQ ID NO: 119)
92) Acetyl-KNMQQQCRFYEALHDPNLNEEQRNARICSIRDDP-SRRCRRCRRC-NH₂ (SEQ ID NO: 120) 
93) Acetyl-KNMQQQK(Z)RFYEALHDPNLNEEQRNARIK(Z)SIRDDP-
SRRK(Z)RRK(Z)RRK(Z)-NH2 (SEQ ID NO: 121)
94) GFNMQQQCRFYEALHDPNLNEEQRNARIRSIRDD-NH₂ (SEQ ID NO: 122)
95) GFNMQQQRRFYEALHDPNLNEEQRNARIRSIRDDC-NH₂ (SEQ ID NO: 123)
96) GFNMQQQRRFYEALHDPNLNEEQRNARIRSIRDDP-SRRK(Z)R-NH₂ (SEQ ID NO: 124)
97) GFNMQQQRRFYEALHDPNLNEEQRNARIRSIRDDP-SRRCR-NH₂ (SEQ ID NO: 125)
98) GFNMQQQCRFYEALHDPNLNEEQRNARICSIRDDP-SRRCRRCRRC-NH₂ (SEQ ID NO: 126) 
99) GFNMQQQK(Z)RFYEALHDPNLNEEQRNARIK(Z)SIRDDP-
SRRK(Z)RRK(Z)RRK(Z)-NH2 (SEQ ID NO: 127)
100) FNMQCQZRFYEALHDPNLNEEQRNARIRSIRDDC-NH₂SEQ ID NO: 128)
101) Acetyl-KNMQCQZRFYEALHDPNLNEEQRNARIRSIRDDC-NH₂ (SEQ ID NO: 129)
102) GFNMQCQK(Z)RFYEALHDPNLNEEQRNARIRSIRDDC-SRRK(Z)R-NH₂ (SEQ ID NO: 130)
103) FNMQCQK(Z)RFYEALHDPNLNEEQRNARIRSIRDDC-NH₂ (SEQ ID NO: 131)
104) Acetyl-KNMQCQK(Z)RFYEALHDPNLNEEQRNARIRSIRDDC-SRRK(Z)R-NH₂ (SEQ ID NO: 132) 
105) GFNMQCQK(Z)RFYEALHDPNLNEEQRNARIRSIRDDC-SRRK(Z)RRK(Z)RRK(Z)-
NH2 (SEQ ID NO: 133)
 
106) Acetyl-KNMQCQK(Z)RFYEALHDPNLNEEQRNARIRSIRDDC-
SRRK(Z)RRK(Z)RRK(Z)-NH2 (SEQ ID NO: 134)
 
107) GFNMQCQK(Z)RFYEALHDPNLNEEQRNARIRSIRDDC-SRRK(Z)RRK(Z)RRK(Z)-
NH2 (SEQ ID NO: 135)
(iii) The following peptides:
108) CAWHLGELVWC (SEQ ID NO: 136)
109) DCAWHLGELVWCT (SEQ ID NO: 137)
110) DCAWHLGELVFCT (SEQ ID NO: 138)
111) DCAWHLGELVX¹⁵CT (SEQ ID NO: 139)
   X¹⁵=1-naphtoyl, 2-naphtoyl, benzyl, or benzothiophene
112) CDCAWHLGELVWCTC (SEQ ID NO: 140)
113) CAYHLGELVWC (SEQ ID NO: 141)
114) DCAYHLGELVWCTF(2-Pya) (SEQ ID NO: 142)
115) Acetyl-(Lys[Azide])RRRGSGPDCAYHKGELVWCTFH-CONH₂) (SEQ ID NO: 143)

In consideration of the three-dimensional structure of IgG-BP and IgG, when R³ has IgG-BP, the amino acid sequence of IgG-BP may be any one of the amino acid sequences shown in SEQ ID NOs: 144 to 179 lacking the N-terminal amino acid residue of the amino acid sequences shown in SEQ ID NOs: 100 to 135.

R² may be bonded to the carbon of the carbonyl group of the formula I or a linker via the side chain of a Lys residue contained in IgG-BP, particularly X⁵, and R³ may be bonded to a carbon of the benzene ring in the formula I or a linker via the side chain of Lys residue contained in IgG-BP, particularly the above-mentioned X⁵.

When R² is, for example, the above-mentioned 79), 80), 81), 72-2), 88), 89), 90), 91), 92), 93), 101), 104), or 106), the side chain of any Lys residue may be bonded to the carbon of the carbonyl group or a linker in the formula I. Similarly, when R³ is the above-mentioned 79), 80), 81), 72-2), 88), 89), 90), 91), 92), 93), 101), 104), or 106), the side chain of any Lys residue may be bonded to the carbon of the benzene ring in the formula I or a linker.

In addition, in the aforementioned IgG-BP, any two cysteine residues may form an intramolecular (peptide) bond via a disulfide bond.

R⁴ is H or a substituted or unsubstituted alkyl group having 1 to 8 carbon atoms. R⁴ is, for example, an alkyl group having 1 to 6, 1 to 4, or 1 to 3 carbon atoms, preferably an ethyl group, more preferably a methyl group.

The salt of the cyclic peptide is not particularly limited as long as it is a pharmacologically acceptable salt, and may be either an acidic salt or a basic salt. Examples of the salt include alkali metal salts such as lithium salt, sodium salt, and potassium salt, alkaline earth metal salts such as magnesium salt and calcium salt, inorganic acid salts such as hydrochloride, hydrobromide, sulfate, nitrate, oxalate, and phosphate, and organic acid salts such as acetate, propionate, hexanoate, cyclopentanepropionate, glycolate, pyruvate, lactate, malonate, succinate, malate, fumarate, tartrate, citrate, benzoate, o-(4-hydroxybenzoyl)benzoate, cinnamate, mandelate, methanesulfonate, ethanesulfonate, 1,2-ethanedisulfonate, 2-hydroxyethanesulfonate, benzenesulfonate, p-chlorobenzenesulfonate, 2-naphthalenesulfonate, p-toluenesulfonate, camphorsulfonate, glucoheptanoate, 3-phenylpropionate, trimethylacetate, tert-butylacetate, lauryl sulfate, gluconate, glutamate, hydroxynaphthoate, salicylate, stearate, trifluoroacetate (TFA), maleate, muconate, and the like.

The compounds used in the tCAP method can be synthesized by a known method based on the structure shown in the above-mentioned formula I. For example, in the synthesis of a compound in which R² has IgG-BP, the amino group of the peptide on the resin synthesized by solid-phase peptide synthesis is glutarylated, N-methyl-1,2-phenylenediamine is condensed, and then 4-nitrophenyl chloroformate treatment is performed. For example, in the synthesis of a compound in which R³ has IgG-BP, a MeDbz residue is inserted using Fmoc-MeDbz (3-[(9-Fluorenylmethoxycarbonyl)amino]-4-(methylamino)benzoic acid) during the peptide elongation on the resin, whereby the skeleton shown in the above-mentioned formula I can be constructed at the MeDbz residue site by 4-nitrophenyl chloroformate treatment of the protected peptide resin.

IgG has two symmetrical pairs of partial peptide chains (Fc chains) of the heavy chain constant region in the Fc region, and thus has two sites where IgBP can bind. For this reason, 0 to 2 IgBPs can bind to one antibody molecule. In the present specification, the absence of binding of IgBP to an antibody is sometimes referred to as "zero valent", binding of one IgBP to one antibody molecule is sometimes referred to as "monovalent," and binding of two IgBPs to one antibody molecule is sometimes referred to as "bivalent".

In the amino acid sequence described in the present specification, A is an alanine residue, R is an arginine residue, N is an asparagine residue, D is an aspartic acid residue, C is a cysteine residue, Q is a glutamine residue, E is a glutamic acid residue, G is a glycine residue, H is a histidine residue, I is an isoleucine residue, L is a leucine residue, K is a lysine residue, M is a methionine residue, F is a phenylalanine residue, P is a proline residue, S is a serine residue, T is a threonine residue, W is a tryptophan residue, Y is a tyrosine residue, and V is a valine residue. In addition, Hcy is a homocysteine residue, Dpr is a diaminopropionic acid residue, Orn is an ornithine residue, βAla is a β-alanine residue, Dab is a 2,4-diaminobutyric acid residue, Nle is a norleucine residue, Nva is a norvaline residue, Tle is a tert-leucine residue, Ala(t-Bu) is a tert-butylalanine residue, and Cha is a cyclohexylalanine residue. In addition, the amino group in the residue having an amino group in the side chain (lysine residue, ornithine residue, 2,4-diaminobutyric acid residue) may be acetylated as necessary. In the present specification, the acetylated form of natural and artificial amino acids may be described with the prefix Ac in the amino acid symbols described above. It is understood that the acetylated form may be included even when Ac is not indicated, unless such interpretation is not particularly inconsistent. K(Azide) shows an azide-bound lysine residue.

The conjugate of the present invention can be preferably used for the examination of intestinal tissues and the treatment of intestinal diseases. Therefore, the present invention provides an agent for the examination of intestinal tissues or the treatment of intestinal diseases (hereinafter sometimes referred to as the "agent of the present invention"), which contains the conjugate of the present invention.

The amount of the conjugate of the antibody, etc. of the present invention and a functional molecule contained in the agent of the present invention is not particularly limited, and may generally be 0.001 to 100% by weight relative to the agent of the present invention. The amount may be appropriately set in consideration of the purpose, the subject of application, the application conditions, and the like. The agent of the present invention may contain components other than the antibody, etc. of the present invention. Examples of other components that may be contained in the agent of the present invention include, but are not limited to, surfactants (PEG, Tween, etc.), excipients, antioxidants (ascorbic acid, etc.), colorants, flavorings, preservatives, stabilizers, buffers (phosphoric acid, citric acid, other organic acids, etc.), chelating agents (EDTA, etc.), suspending agents, isotonicity agents, binders, disintegrants, lubricants, flowability enhancers, taste masking agents, and the like. Also, the agent may contain other components such as light anhydrous silicic acid, lactose, crystalline cellulose, mannitol, starch, carmellose calcium, carmellose sodium, hydroxypropyl cellulose, hydroxypropyl methyl cellulose, polyvinyl acetal diethyl amino acetate, polyvinyl pyrrolidone, gelatin, medium chain fatty acid triglyceride, polyoxyethylene hydrogenated castor oil 60, sucrose, carboxymethyl cellulose, corn starch, inorganic salts, and the like. Alternatively, the agent may contain amino acids, proteins such as serum albumin and gelatin, and antibodies and antigen-binding portions thereof other than the antibody, etc. of the present invention. In addition, when the agent of the present invention is prepared as an aqueous solution for injection, the antibody, etc. of the present invention are dissolved in an isotonic solution containing, for example, physiological saline, glucose, or other auxiliary agents. Examples of the auxiliary agent that can be used include, but are not limited to, D-sorbitol, D-mannose, D-mannitol, and sodium chloride. Furthermore, a suitable solubilizing agent, for example, alcohol (ethanol, etc.), polyalcohol (propylene glycol, PEG, etc.), nonionic surfactant (polysorbate 80, HCO-50), and the like may be used in combination.

The dosage form of the agent of the present invention is not particularly limited, and may be appropriately set in consideration of its use, the subject of application, the application conditions, and the like. The dosage form of the agent of the present invention may be, for example, liquid, semi-solid, solid, or powder, preferably liquid.

In preparing the agent of the present invention, the conjugate of the present invention may be formulated according to a conventional method (for example, Remington's Pharmaceutical Science, latest edition, Mark Publishing Company, Easton, U.S.A.).

The agent of the present invention can be administered orally or parenterally, and is preferably administered parenterally. Concretely, it can be administered to the subject by injection or transdermal administration. Examples of the injection dosage form include intravenous injection, intramuscular injection, or subcutaneous injection, which can be administered systemically or locally. It can also be injected locally at or near the target site. Examples of the transdermal dosage form include ointment, gel, cream, poultice, and patch, which can be administered systemically or locally. The administration method and dose can be determined appropriately in consideration of the age, weight, condition of the subject, purpose of administration, and the like.

In another embodiment, the present invention provides a polynucleotide encoding the antibody, etc. of the present invention (hereinafter sometimes referred to as "the polynucleotide of the present invention"). The polynucleotide of the present invention may be DNA or RNA, or DNA/RNA chimera. Also, the polynucleotide of the present invention may be double-stranded or single-stranded. When it is double-stranded, it can be double-stranded DNA, double-stranded RNA, or DNA:RNA hybrid.

The polynucleotide of the present invention includes a polynucleotide containing a nucleotide sequence encoding both the heavy chain variable region and the light chain variable region of the antibody, etc. of the present invention, and a combination of a polynucleotide containing a nucleotide sequence encoding the heavy chain variable region and a polynucleotide containing a nucleotide sequence encoding the light chain variable region.

The polynucleotide of the present invention can be easily produced by utilizing known gene recombination techniques, based on the amino acid sequence information of the antibody, etc. of the present invention, known sequence information, and sequence information described in the Sequence Listing of the present specification. For example, the polynucleotide of the present invention can be produced by designing appropriate primers based on the sequence information, amplifying DNA encoding the constituent elements constituting the antibody of the present invention by PCR reaction, and linking the DNA fragments by using an appropriate enzyme such as ligase. Alternatively, polynucleotide encoding each constituent element may be synthesized using a polynucleotide synthesizer, based on the amino acid sequence information of the antibody of the present invention.

The obtained polynucleotide encoding the antibody, etc. of the present invention can be used as it is, or after digestion with a restriction enzyme or addition of a linker, if desired, depending on the purpose. The polynucleotide may have the translation initiation codon ATG at the 5' end thereof, and the translation stop codon TAA, TGA or TAG at the 3' end thereof. These translation initiation codon and translation stop codons can be added using an appropriate synthetic DNA adapter.

The polynucleotide of the present invention is preferably isolated or purified. The purity of the isolated or purified polynucleotide of the present invention (the ratio of the weight of the polynucleotide of the present invention to the weight of the total polynucleotide) is generally 50% or more, preferably 70% or more, more preferably 90% or more, and most preferably 95% or more (e.g., substantially 100%).

In another embodiment, the present invention provides a vector containing the polynucleotide of the present invention (hereinafter sometimes referred to as the "vector of the present invention").

The vector of the present invention includes a vector containing a polynucleotide containing a nucleotide sequence encoding both the heavy chain variable region and the light chain variable region of the antibody, etc. of the present invention, and a combination of a vector containing a polynucleotide containing a nucleotide sequence encoding the heavy chain variable region and a vector containing a polynucleotide containing a nucleotide sequence encoding the light chain variable region. The vector is preferably isolated or purified. Examples of the vector include expression vector, cloning vector, and the like, and can be selected according to the purpose, with preference given to an expression vector. The expression vector is capable of expressing the antibody of the present invention. The expression vector can be produced by functionally linking the polynucleotide of the present invention downstream of a promoter in an appropriate expression vector. Examples of the type of the vector include plasmid vector, viral vector, and the like, and the vector can be appropriately selected according to the host to be used.

As the host, for example, bacteria of the genus Escherichia (Escherichia coli, etc.), bacteria of the genus Bacillus (Bacillus subtilis, etc.), yeast (Saccharomyces cerevisiae, etc.), insect cells (Spodoptera frugiperda sell; Sf cell, etc.), insects (silkworm larvae, etc.), mammalian cells (human mesenchymal stem cell (MSC), rat nerve cell, monkey cell (COS-7), Chinese hamster cell (CHO cell), etc.) can be used, but the host is not limited to these.

Examples of the plasmid vector include plasmid vectors derived from Escherichia coli (e.g., pBR³22, pBR³25, pUC12, pUC13), plasmid vectors derived from Bacillus subtilis (e.g., pUB110, pTP5, pC194), yeast-derived plasmid vectors (e.g., pSH19, pSH15), and the like, and the plasmid vector can be appropriately selected according to the type of the host to be used and the purpose of use.

The type of virus vector can be selected appropriately according to the type of the host to be used and the purpose of use. For example, when insect cell is used as the host, baculovirus vector and the like can be used, though not limited to these. In addition, when mammalian cell is used as the host, retrovirus vectors such as Moloney murine leukemia virus vector, lentivirus vector, Sindbis virus vector, and the like, adenovirus vector, herpes virus vector, adeno-associated virus vector, parvovirus vector, vaccinia virus vector, Sendai virus vector and the like can be used, though not limited to these.

In addition, a promoter that can initiate transcription in the host can be selected according to the type of the host to be used. For example, when the host is a bacterium of the genus Escherichia, trp promoter, lac promoter, T7 promoter, and the like are preferred. When the host is a bacterium of the genus Bacillus, SPO1 promoter, SPO2 promoter, penP promoter, and the like are preferred. When the host is a yeast, PHO5 promoter, PGK promoter, and the like are preferred. When the host is an insect cell, polyhedrin promoter, P10 promoter, and the like are preferred. When the host is a mammalian cell, subgenomic (26S) promoter, CMV promoter, SRα promoter, and the like are preferred.

The vector of the present invention may contain a signal sequence for antibody secretion. When produced in the periplasm of Escherichia coli, pelB signal sequence (Lei, S. P. et al. J. Bacteriol. (1987) 169, 4379) can be used as the signal sequence for antibody secretion.

When desired, the vector of the present invention may contain an enhancer, a splicing signal, a polyA addition signal, a selection marker, an SV40 replication origin, and the like, each in a functional form. Examples of the selection marker include, but are not limited to, dihydrofolate reductase gene (methotrexate (MTX) resistance), ampicillin resistance gene, neomycin resistance gene, and the like.

A transformant into which the vector of the present invention has been introduced (hereinafter sometimes referred to as the "transformant of the present invention") can be produced by introducing the vector of the present invention into a host according to a gene introduction method known per se (e.g., lipofection method, calcium phosphate method, microinjection method, protoplast fusion method, electroporation method, DEAE dextran method, gene introduction method using Gene Gun, and the like). Using an expression vector as the vector to be introduced, the transformant of the present invention can express the antibody, etc. of the present invention. The transformant of the present invention is useful for producing the antibody, etc. of the present invention.

The antibody, etc. of the present invention can be produced by culturing the transformant of the present invention by a method known per se according to the type of host, and isolating the antibody, etc. of the present invention from the culture A transformant whose host is a bacterium of the genus Escherichia is cultured in an appropriate medium such as LB medium or M9 medium, generally at about 15 to 43°C for about 3 to 24 hr. A transformant whose host is a bacterium of the genus Bacillus is cultured in an appropriate medium generally at about 30 to 40°C for about 6 to 24 hr. A transformant whose host is a yeast is cultured in an appropriate medium such as Burkholder's medium, generally at about 20 to 35°C for about 24 to 72 hr. A transformant whose host is an insect cell or insect is cultured in an appropriate medium, such as Grace's Insect medium supplemented with about 10% bovine serum, generally at about 27°C for about 3 to 5 days. A transformant whose host is an animal cell is cultured in an appropriate medium, such as MEM medium supplemented with about 10% bovine serum, generally at about 30°C to 40°C for about 15 to 60 hr. In each culture, aeration and stirring may be performed as necessary.

For methods for producing an antibodies by genetic engineering, refer to, for example, Co, M. S. et al., J. Immunol. (1994) 152, 2968-2976; Better, M. and Horwitz, A. H., Methods Enzymol. (1989) 178, 476-496; Pluckthun, A. and Skerra, A., Methods Enzymol. (1989) 178, 497-515; Lamoyi, E., Methods Enzymol. (1986) 121, 652-663; Rousseaux, J. et al., Methods Enzymol. (1986) 121, 663-669; Bird, R. E. and Walker, B. W., Trends Biotechnol. (1991) 9, 132-137, and the like.

The antibody, etc. of the present invention can be separated and purified from the culture by the separation and purification methods generally used for the purification of antibodies. For example, the antibodies and antigen-binding portions can be separated and purified by appropriately combining chromatography column, filter, ultrafiltration, salting out, solvent precipitation, solvent extraction, distillation, immunoprecipitation, SDS-polyacrylamide gel electrophoresis, isoelectric focusing, dialysis, recrystallization, and the like.

Examples of chromatography include affinity chromatography, ion exchange chromatography, hydrophobic chromatography, gel filtration, reversed-phase chromatography, and adsorption chromatography (Strategies for Protein Purification and Characterization: A Laboratory Course Manual. Ed Daniel R. Marshak et al., Cold Spring Harbor Laboratory Press, 1996). These chromatographies can be performed using liquid-phase chromatography such as HPLC and FPLC. Examples of the column used for affinity chromatography include protein A column and protein G column. Examples of the column using Protein A include Hyper D, POROS, and Sepharose FF (GE Amersham Biosciences). The present invention also encompasses the antibody, etc. of the present invention with highly purity, which are obtained using these purification methods.

In one embodiment of the transformant in the present invention, the transformant may be a mammalian cell expressing the antibody of the present invention on the cell surface thereof. As the mammalian cell, any mammalian cell can be used as long as it is capable of expressing the antibody of the present invention, on the cell surface thereof. In one embodiment, the mammalian cell may be a somatic cell (e.g., skin cell, blood cell, muscle cell, nerve cell, bone cell, liver cell, intestinal cell, lung cell, pancreatic cell, kidney cell, etc.) or a stem cell (somatic stem cell, ES cell, iPS cell, etc.). In one preferred embodiment, the mammalian cell may be a mesenchymal stem cell.

A method for introducing a nucleic acid sequence encoding an antibody or an antigen-binding portion thereof into a cell by recombinant gene technology and expressing same on the cell surface thereof may be any method known per se. One example is the method used in the Examples described below, but is not limited thereto.

In another embodiment, the present invention provides an agent for promoting regeneration of and/or repairing an intestinal tissue (hereinafter sometimes referred to as the "cell therapeutic agent of the present invention"), containing mammalian cells expressing the antibody of the present invention or a transformant thereof on the cell surface thereof.

Mammalian cells expressing the antibody of the present invention or a transformant thereof on the cell surface thereof, which are the active ingredient of the cell therapy agent of the present invention, have the property of accumulating in tissues containing cells expressing GPA33 (i.e., intestinal tissue). In addition, for example, when a stem cell (somatic liver cell or embryonic stem cell) is used as the mammalian cell, the regeneration and/or repair of tissues expressing GPA33 can be promoted. In a preferred one embodiment of the present invention, the mammalian cell may be a mesenchymal stem cell.

The cell therapeutic agent of the present invention may contain components other than mammalian cells expressing GPA33 on the cell surface thereof. Such components may be pharmaceutically acceptable pharmaceutical additives. Examples of pharmaceutical additives include, but are not limited to, isotonicity agent, buffer, pH adjuster, stabilizer, chelating agent, and the like.

Examples of the isotonicity agent include sodium chloride, potassium chloride, sugars, glycerin, and the like. Examples of the buffer include boric acid, phosphoric acid, acetic acid, citric acid, the corresponding salts thereof (for example, alkali metal salts or alkaline earth metal salts such as sodium salt, potassium salt, calcium salt, magnesium salt, etc.). Examples of the pH adjuster include inorganic acids such as hydrochloric acid, sulfuric acid, phosphoric acid, polyphosphoric acid, boric acid, borax, and the like; organic acids such as acetic acid, propionic acid, oxalic acid, gluconic acid, fumaric acid, lactic acid, citric acid, succinic acid, tartaric acid, malic acid, and the like; inorganic bases such as potassium hydroxide, sodium hydroxide, and the like; organic bases such as monoethanolamine, triethanolamine, diisopropanolamine, triisopropanolamine, and the like; ammonium acetate, sodium lactate, sodium citrate, potassium carbonate, sodium bicarbonate, sodium carbonate, ammonium bicarbonate, dipotassium phosphate, potassium dihydrogen phosphate, sodium hydrogen phosphate, sodium dihydrogen phosphate, calcium lactate, and the like. Examples of the stabilizer include human serum albumin, ordinary L-amino acid, sugars, cellulose derivative, and the like, which can be used alone or in combination with a surfactant, and the like. The above-mentioned L-amino acid may be any of glycine, cysteine, glutamic acid, and the like, though not limited thereto. The sugar may be any of monosaccharides such as glucose, mannose, galactose, fructose, and the like, sugar alcohols such as mannitol, inositol, xylitol, and the like, disaccharides such as sucrose, maltose, lactose, and the like, polysaccharides such as dextran, hydroxypropyl starch, chondroitin sulfate, hyaluronic acid, and the like, and derivatives thereof, and is not limited to these. The cellulose derivative may be any of methylcellulose, ethylcellulose, hydroxyethylcellulose, hydroxypropylcellulose, hydroxypropylmethylcellulose, sodium carboxymethylcellulose, and the like, though not limited thereto. Examples of the chelating agent include sodium edetate, citric acid, and the like.

The shape of the cell therapeutic agent of the present invention is not particularly limited as long as it is a shape permitting transplantation into a living body, and examples of the shape include a liquid form consisting of cells and an appropriate dispersion medium, a sheet form in which cells are fixed on an appropriate biocompatible material, and the like.

The cell therapeutic agent of the present invention can also be paraphrased as a "cell therapeutic agent for preventing and/or treating intestinal diseases" or a "pharmaceutical composition for preventing and/or treating intestinal diseases". Intestinal diseases are as described above.

The present invention is explained in more detail in the following Examples; however, the present invention is not limited in any way by these examples.

### [Example]

### [Example 1] Immunization of complete antibody human antibody-producing animals with human and mouse GPA33

Complete antibody human antibody-producing animals (hereinafter sometimes simply referred to as "TC animals") were immunized with human and mouse GPA33 (C-terminal His tag fusion protein, abcam) as antigens. Freund's complete adjuvant was used as an immunostimulant for the first immunization, and Sigma adjuvant system was used for the booster immunization. Human GPA33 and mouse GPA33 were mixed at 1:1 (molar ratio), and this was further mixed with adjuvant and administered intraperitoneally. Immunization was performed by 250 µg/administration at two-week intervals. For the final immunization, only the antigen was administered via the tail vein of the TC animal. The antibody titers of antibodies against human and mouse GPA33 in the TC animal serum after immunization were evaluated by ELISA, and the results are shown in Fig. 1. As shown in Fig. 1, the antibody titers increased by repeating immunization, indicating that antibodies specific to human and mouse GPA33 were produced.

### [Example 2] Phage library construction

Using RNA iso Plus (Total RNA extraction reagent, TAKARA), total RNA was extracted from the spleen of the TC animal immunized in Example 1, and cDNA was synthesized by reverse transcription with an oligo dT primer using SuperScript^{™} III Reverse Transcriptase (Invitrogen^{™}). Using the synthesized cDNA as a template, a single-chain Fv antibody phage library was constructed according to a method reported previously (PLOS ONE, 2017, 12, e0167860). The diversity size of the constructed phage library was 1.4×10⁷.

### [Example 3] Enrichment of specific antibody from phage library

The flow of the isolation method for GPA33-specific clones from the constructed GPA33 immune scFv antibody phage library is shown in Fig. 2 (left). In brief, tube panning against mGPA33 (abcam) was first performed using the scFv antibody phage library. 1 µg of hGPA33 and mGPA33 were dissolved in 1 mL of PBS, and the antigen solutions were added to plastic tubes and left standing overnight. After washing with PBST, 3% skim milk was added to the tubes and the tubes were left standing at room temperature for 2 hr for blocking. After washing with PBST, a mixture of 0.5 mL (PBS) of antibody phage library (0R, 1.0×10^11 pfu) and 0.5 mL (PBS) of 3% skim milk was added to the tubes and the mixture was reacted at room temperature for 1.5 hr with shaking. After washing with PBST, 1 mL of 100 mM Glycine-HCl (pH 2.2) was added to the tubes and the mixture was incubated at room temperature for 10 min with shaking, after which the solution was collected and neutralized by adding 100 µL of 1 M Tris-HCl (pH 9.0). This solution was mixed with 15 mL of Escherichia coli TG-1 culture medium and left standing at 37°C for 30 min to infect the phage. After centrifugation at 1500 g for 5 min and removal of the supernatant, the pellets were resuspended in 1 mL of 2TY and seeded on a 2TYAG plate and cultured overnight at 30°C. Escherichia coli colonies on the plate were collected and used as library stock (1R).

To perform cell panning, hGPA33-expressing cells were first prepared by the following method. An hGPA33 expression vector (ORIGENE) co-expressing GFP was transfected into CHO-K1 (Chinese Hamster Ovary) strain, and drug screening was repeated while monitoring GFP fluorescence. Cell panning was performed using the hGPA33-expressing cells co-expressing GFP established by this method. That is, 1.0×10^6 cells of hGPA33- expressing cells were suspended in 0.25 mL of 2% BSA/PBS, 0.25 mL (1.0×10^11 pfu) of phage solution at 4.0×10^11 pfu/mL was added to this solution, and after thorough mixing by pipetting, the reaction was performed at 4°C for 1 h with shaking. After centrifugation, the supernatant was removed, and the cells were resuspended in 300 µL of 1xPBS. 5 µL of 7-AAD Viability Dye (Beckman Coulter) was added to the resuspended cells and reacted at 4°C for 15 min to stain dead cells. After centrifugation, the supernatant was removed and the cells were suspended in 1 mL of 1×PBS. After passing through a mesh filter, sorting was performed on a flow cytometer (S3e, BioRad). 7-AAD stained cells were excluded, and GFP high expression areas were collected. 110 µL of 1 M Glycine-HCl (pH 2.2) was added to the collected cells, and after 5 min, the cells were centrifuged, the supernatant was collected, and the phages bound to the cells were collected. The solution was neutralized by adding 110 µL of 1M Tris-HCl (pH 9.0), mixed with 30 mL of Escherichia coli in logarithmic growth phase, and left standing at 37°C for 30 min to infect the phage. After infection, the mixture was centrifuged at 1500 g x 5 min, the supernatant was removed, and Escherichia coli was resuspended in 2 mL of 2TY, plated on a 2TYAG plate, and cultured overnight at 30°C. In this way, library stock (2R) was obtained.

A phage library was prepared from the thus-obtained library stocks of 0R, 1R, and 2R, and the binding activity to hGPA33-expressing cells and mGPA33-expressing cells expressing mouse GPA33 was evaluated by FACS. To be specific, cells were prepared to 2.0×10^6 cells/1 mL (in 1×PBS), and 100 µL of 2.0×10^5 cells were dispensed into 1.5 mL tubes. After centrifugation at 200 g, RT, for 3 min, and removing the supernatant, the cells were washed by being suspended in 500 µL of 2% BSA/PBS. 100 µL of 1.0×10^11 pfu/mL phage solution was added to 100 µL of the cell solution suspended in 1×PBS, and the mixture was thoroughly mixed by pipetting, and then reacted at 4°C for 30 min with shaking. After washing twice with 500 µL of 2% BSA/PBS, 1 µL of 0.1 mg/mL Anti M13 Ab-biotin (abcam)/50 µL of PBS (0.2 µg, final concentration: 2 µg/mL) was added to 50 µL of a cell solution suspended in PBS (total 100 µL), and incubated at 4°C for 30 min, followed by washing once with 500 µL of 2% BSA/PBS. Then, 0.5 µL of 1 mg/mL SA-PE (Vector Laboratories)/50 µL of PBS (0.5 µg, final concentration: 5 µg/mL) was added to 50 µL of a cell solution suspended in PBS (total 100 µL), and the mixture was incubated at 4°C for 30 min, followed by washing once with 500 µL of 2% BSA/PBS. The cells were suspended in 500 µL of 1×PBS, passed through a mesh filter, and then subjected to FACS analysis using a flow cytometer (S3e, BioRad).

The results are shown in Fig. 2 (right). As shown in Fig. 2 (right), in the control cells, a shift in the horizontal axis did not occur due to the absence of expression of GFP derived from the vector, and no shift in the vertical axis indicating phage binding was observed in the library stocks of 0R, 1R, and 2R . On the other hand, in the hGPA33-expressing cells and mGPA33-expressing cells, GFP expression was confirmed by a shift in the horizontal axis. Although not observed in the library stock (0R), in the library stock (1R) and library stock (2R), a shift in the vertical axis, i.e., phage binding, was observed in the cells in the region with high GFP expression. From the above results, it was found that phages with binding activity to human and mouse GPA33 were enriched by two rounds of biopanning.

### [Isolation of specific antibodies from the phage library]

32 phages were randomly picked up from library stock 2R and cloned, and the binding activity to human and mouse GPA33 (C-terminal His tag fusion protein, abcam) recombinant proteins was analyzed by ELISA. The results are shown in Fig. 3. As shown in Fig. 3, 9 out of the 32 monoclonal phages showed binding activity to hGPA33 and mGPA33. DNA sequence analysis was performed for these 9 monoclonal phages, and the sequences revealed that they contained 5 types of monoclonal phages.

Then, the binding activity to GPA33 of the obtained 5 types of cloned phages was evaluated by FACS. The results are shown in Fig. 4.

As shown in Fig. 4, among the 5 clones, 2RC16 and 2RC24 did not show any shift in the vertical axis and did not show binding activity. On the other hand, three clones of 2RC9, 2RC17, and 2RC28, were found to have cross-binding activity to both human and mouse GPA33. From these three clones, the amino acid sequences of single-chain Fv antibodies that bind to human and mouse GPA33 were determined (Fig. 5).

### [Example 4] Identification of specific antibody from phage library by comprehensive sequence analysis

As described above, three types of single-chain Fvs with cross-reactivity to both human and mouse GPA33 were successfully identified by biopanning. In order to search for further candidate sequences, in vivo panning was performed using the library stocks (1R and 2R) obtained by tube panning and cell panning in Fig. 2 and library stock 0R and 0R library, and comprehensive sequence analysis was performed on the obtained library stocks by using a next-generation sequencer. The in vivo panning method is as follows: The 0R phage library stock used for in vivo panning was passed through an Endo Trap HD (LIONEX) column before administration to mice to remove endotoxins (Hietala V, et al., (2019) Front. Microbiol. 10:1674). 100 µL of the 0R phage library (6.0×10^10 pfu) was administered from the tail vein of four mice (Balb/c: 8ws), and autopsy was performed 2 hr, 24 hr, 48 hr, or 72 hr after administration. For autopsy procedure, the abdomen was first opened while the mouse was inhaling the anesthetic isoflurane (Pfizer), and the mouse was perfused with 1% heparin PBS from the left ventricle. The timing of the end of perfusion was determined by the change in color of the liver. The intestine was excised and the inside of the intestine was washed with 1% heparin PBS to remove unbound phages. The intestine was then homogenized in 5 mL of PBS on ice. 1 mL of 1 M Glycine-HCl (pH 2.2) was added to the homogenized solution, which was then centrifuged after 5 min and the supernatant was collected. 1 mL of 1 M Tris-HCl (pH 9.0) was added to neutralize the solution, which was then mixed with 30 mL of Escherichia coli in the logarithmic growth phase and allowed to stand at 37°C for 30 min to infect the phage. After infection, the mixture was centrifuged at 1500 g x 5 min, the supernatant was removed, and the Escherichia coli was resuspended in 2 mL of 2TY, seeded on a 2TYAG plate, and cultured overnight at 30°C. Colonies were collected after culture and stored as Escherichia coli library stocks.

The phagemid DNAs prepared from the library stocks of in vivo panning 2 hours, 24 hours, 48 hours, and 72 hours (2h, 24h, 48h, and 72h) and the library stocks of 0R, 1R, and 2R were used to perform comprehensive sequence analysis of the VH sequences contained therein by using the MiSeq system (Illumina, Inc., San Diego). The DNA sequencing sample (10 ng) was amplified by PCR using two types of primers containing complementary sequences upstream and downstream of the VH gene on the phagemid DNA. The MiSeq library for DNA sequencing was prepared using the QIAseq 1-step Amplicon Library kit (QIAseq, Inc.) according to the protocol provided by the manufacturer. The final loading concentration was adjusted to 15 pM according to the MiSeq loading protocol. For long paired-end (2×300 bp) sequence analysis, MiSeq reagent kit v3 (Illumina) was used. The quality of MiSeq analysis was monitored according to the standard Illumina procedures explained by the service provider. The sequence data after analysis was subjected to USEARCH Ver.8.0 (Edgar, RC) to merge paired-end nucleotide sequences, filtering, alignment, and trimming were performed, and low-quality and meaningless short sequences were removed. The DNA sequence of the finally obtained VH was converted to an amino acid sequence, and an analysis program called SOPRA (Sequence Ordering Program Ranked by Amplification Factor), written in Perl script, was used to perform alignment, clustering, and counting of the number of each sequence in the VH amino acid sequence data. The number of DNA reads by this next-generation sequencer and the number of amino acid sequences used for analysis by SOPRA after processing with USEARCH Ver.8.0 (Edgar, RC) are summarized in Table 1.

**[Table 1]**

| GPA33 VH Library | OR | h1R | m1R | mh2RC | 2h | 24h | 48h | 72h |
|---|---|---|---|---|---|---|---|---|
| **total read number (DNA sequence)** | 731247 | 522746 | 627062 | 587100 | 631967 | 610897 | 624881 | 730302 |
| **analysis target pair read number (DNA sequence)** | 166145 (100%) | 228693 (100%) | 193672 (100%) | 217212 (100%) | 172524 (100%) | 188266 (100%) | 195631 (100%) | 219461 (100%) |
| **cluster number (unique sequence) (amino acid sequence)** | 27521 (16.6%) | 70598 (30.9%) | 40271 (20.8%) | 25971 (12.0%) | 57305 (33.2%) | 58438 (31.0%) | 64920 (33.2%) | 41862 (19.1%) |
| **cluster number of member number 1 (amino acid sequence)** | 20872 (12.5%) | 50514 (22.1%) | 27110 (14.0%) | 18190 (8.4%) | 46893 (27.2%) | 47336 (25.1%) | 53676 (27.4%) | 26739 (12.2%) |
| **Top 1 member number (amino acid sequence)** | 7306 (4.4%) | 3700 (1.6%) | 7137 (3.7%) | 7871 (3.6%) | 6908 (4.0%) | 7930 (42%) | 10729 (5.5%) | 8515 (3.9%) |

Using the thus-obtained amino acid sequence data of VH, the ratio of the frequency of occurrence of a certain VH sequence after each panning (the percentage of the VH sequence among the total number of VH sequences after each panning) to the frequency of occurrence of the VH sequence at 0R (the percentage (%) of the VH sequence among the total number of VH sequences) was calculated as the amplification rate using the SOPRA program. The top 40 sequences with the highest amplification rate were extracted from each library stock, and alignment was performed using a program on CLC Genomics Workbench 9 (QIAGEN Bioinformatics), and a phylogenetic tree was created by the neighbor-joining method. The results are shown in Fig. 6.

As shown in Fig. 6, clusters of groups A to K with high homology are listed. The characteristics of each cluster were inferred as shown in Table 2.

**[Table 2]**

| group | library stock | H-CDR3 |
|---|---|---|
| A | 2h and 72h are predominant. The highest affinity is shown by mH2R-4 (AF = 126). | Similar sequences with about two mutations in CDR3. |
| B | Diverse, including m1Rm, mh2R, mH2RC, 2h, 72h and the like. The highest affinity is shown by h1R-11 (AF = 72). | CDR3s are divided into about three groups with large differences. |
| C | mh2R, 2h, 24h, 48h are predominant. The highest affinity is shown by mh2R-21 (AF = 90). | CDR3s are divided into four groups with large differences. |
| D | m1R is predominant. The highest affinity is shown by mm1R-1 (AF = 147). | CDR3s are widely different. |
| E | From almost all library stocks. | CDR3s are divided into about two groups with large differences. |
| F | Consists of only 1hR. | Almost one type of CDR3. |
| G | mh2R is predominant. The highest affinity is shown by mh2R-1 (AF = 136) . | Almost one type of CDR3. |
| H | mh2RC is predominant. Including clone GPA33-2RC28. The highest affinity is shown by h1R-16 (AF = 65). | Half of CDR3s are the same as clone GPA33-2RC28. |
| I | From almost all library stocks. The highest affinity is shown by mh2R-3 (AF = 80). | Only one type of CDR3. |
| J | mh2RC alone. Including clone GPA33-2RC9, 17. The highest affinity is shown by mh2RC-1 (AF = 68). | Only one type of CDR3 has 1 or 2 mutations. |
| K | From almost all library stocks. The highest affinity is shown by m1R-2 (AF = 81). | Mainly divided into two groups. |

From the above results, in particular, groups H and J on the molecular phylogenetic tree are mainly obtained from the library stock of mh2RC. Based on the panning technique, this mh2RC library stock is considered to contain antibodies that bind to human and mouse GPA33 and can recognize GPA33 on the cell surface. In fact, these groups included GPA33-2RC9, GPA33-2RC17, and GPA33-2RC28 cloned in Example 3, which supports this assumption. From the above, VHs having amino acid sequences included in groups H and J can be used as antibodies targeting intestinal tissue.

In addition, the VH sequences of the phages obtained after the step "2R: Cell panning (hGPA33-expressing CHO-K1 cells)" in the "Biopanning flow" in Fig. 2 were comprehensively analyzed, the sequences with the top 50 amplification rates were determined, and a phylogenetic tree was created (Fig. 26). These 50 types of VHs are also considered to have high likelihood of specifically binding to human and mouse GPA33. The sequences of CDR1-3 and full length of the 50 types of VH determined are shown in Table 3-1 to Table 3-5 below.

**[Table 3-1]**

| Clone Name | CDR1 | SEQ ID NO | CDR2 | SEQ ID NO | CDR3 | SEQ ID NO |
|---|---|---|---|---|---|---|
| GPA33-2RmhC-1 | GFTFSSYN | 8 | ISSRGDYI | 197 | AREAVGTPFDY | 217 |
| GPA33-2RmhC-2 | GYTFTGYY | 180 | INPNSGGT | 198 | ARVESGAFDI | 218 |
| OPA33-2RmhC-3 | GFTFSNYN | 3 | IDRSSTYI | 199 | AREAVGTPFDY | 217 |
| GPA33-2RmhC-4 | GFTFSNYN | 3 | ISSSGDYI | 200 | AREAIGTPFDY | 219 |
| GPA33-2RmhC-5 | GFTFSTYN | 181 | ISSSGDYI | 200 | AREAIGTPFDY | 219 |
| GPA33-2RmhC-6 | GFTFSSYN | 8 | ISSRGDYI | 197 | AREAVGTPFDY | 217 |
| GPA33-2RmhC-7 | GFTFSSYN | 8 | IDRSSTYI | 199 | AREGPGTPFDY | 5 |
| GPA33-2RmhC-8 | GFTFSNYN | 3 | IVSSGDYI | 201 | AREAVGTPFDY | 217 |
| GPA33-2RmhC-9 | GFTFSSYN | 8 | IDRSSTYI | 199 | AREAVGTPFDY | 217 |
| GPA33-2RmhC-10 | GFTFSTYN | 181 | ISSSGDYI | 200 | AREGPGTPFDY | 5 |
| GPA33-2RmhC-11 | GFTFSSYA | 182 | ISGSGGST | 202 | AKGDIVVVPAALFDY | 220 |
| GPA33-2RmhC-12 | GFTFSTYN | 181 | ISSSGDYI | 200 | AREAIGTPFDY | 219 |
| GPA33-2RmhC-13 | GFTFSSYN | 8 | ISSRGDYI | 197 | AREAVGTPFDY | 217 |
| GPA33-2RmhC-14 | GGTFSSYA | 183 | IIPIFGTA | 203 | ARGQQQLAYYQFYGMDV | 221 |
| GPA33-2RmhC-15 | GFTFSSYN | 8 | ISSSGDFM | 4 | AREGPGTPFDY | 5 |
| GPA33-2RmhC-16 | GFTFSNYN | 3 | ISSSGDFM | 4 | AREAIGTPFDY | 219 |
| GPA33-2RmhC-17 | GFTFSTYS | 181 | ISRDSAYI | 204 | VREGVAGAFDI | 222 |
| GPA33-2RmhC-18 | GFTFSTYS | 181 | ISRDSAYI | 204 | VREGVAGAFDI | 222 |
| GPA33-2RmhC-19 | GFTFSTYS | 181 | ISRDSAYI | 204 | VREGVAGAFGI | 223 |
| GPA33-2RmhC-20 | GGTFSSYA | 183 | IIPIFGTA | 203 | ARAYGGSAYYYNYGMDV | 224 |
| GPA33-2RmhC-21 | GFTFDDYA | 184 | ISWNSGSI | 205 | AKDRWDTAMITPDAFDI | 225 |
| GPA33-2RmhC-22 | GFTFSTYS | 1.81 | ISRDSAYI | 204 | VREGVAGAFDI | 222 |
| GPA33-2RmhC-23 | GFTFGDYG | 185 | ISWNSGRK | 206 | AKDMKIIRMEAAGPLEF | 226 |
| GPA33-2RmhC-24 | GFTFSTYS | 181 | ISRDSAYI | 204 | VREGVAGAFDI | 222 |
| GPA33-2RmhC-25 | GFTFSSYN | 8 | ISSSGDFM | 4 | AREGPGTPFDY | 5 |

**[Table 3-2]**

| Clone Name | CDR1 | | CDR2 | | CDR3 | |
|---|---|---|---|---|---|---|
| GPA33-2RmhC-26 | GFTFSTYS | 181 | ISRDSAYI | 204 | VREGVAGAFDI | 222 |
| GPA33-2RmhC-27 | GGTFSSYA | 183 | IIPIFGTA | 203 | ARAYGGSAYYHNYGMDV | 227 |
| GPA33-2RmhC-28 | GYTFTNYG | 186 | ISAYNGNT | 207 | ARDSPLNWGPDAFDI | 228 |
| GPA33-2RmhC-29 | GGSVSSTSYY | 187 | IYYSGST | 208 | ARDRGSGWYRDAFDI | 229 |
| GPA33-2RmhC-30 | GFTFSSYN | 8 | ISSSGDFM | 4 | AREGPGTPFDY | 5 |
| GPA33-2RmhC-31 | GFTFSSYN | 8 | ISSSGDFM | 4 | AREGPGTPFDY | 5 |
| GPA33-2RmhC-32 | GFTFDDYA | 184 | ISWSSGSI | 209 | AKDITDSSGYYGMDA | 230 |
| GPA33-2RmhC-33 | GFTFSSYN | 8 | ISSSGDFM | 4 | AREGPGTPFDY | 5 |
| GPA33-2RmhC-34 | GFTFSNYN | 3 | ISSSGDFM | 4 | AREGPGTPFDY | 5 |
| GPA33-2RmhC-35 | GFTFSSYN | 8 | ISSSGDFM | 4 | AREGPGTPLDY | 231 |
| GPA33-2RmhC-36 | GFTFSNFN | 188 | ISSSGDFM | 4 | AREGPGTPFDY | 5 |
| GPA33-2RmhC-37 | GGSISSYY | 189 | IYHSGST | 210 | ARSRQQLLRDYYYYGMDV | 232 |
| GPA33-2RmhC-38 | GFTFSSYN | 8 | ISSSGDFM | 4 | AREGPGTPFDY | 5 |
| GPA33-2RmhC-39 | GFTFSSYN | 8 | ISSSGDYM | 211 | AREGPGTPFDY | 5 |
| GPA33-2RmhC-40 | GFTFSNYA | 190 | ISGSGGST | 202 | AKGSGSYYKPVYYYYGMDV | 233 |
| GPA33-2RmhC-41 | GFTFSNYN | 3 | ISSSGDFM | 4 | AREGPGTPFDY | 5 |
| GPA33-2RmhC-42 | GFTFDDYA | 184 | ISWNSGSI | 205 | AKCDGSGSYGAFDI | 234 |
| GPA33-2RmhC-43 | GGTFSSYA | 182 | IIPIFGTA | 203 | ARAPDILTSSGYYYYGMDV | 235 |
| GPA33⁻2RmhC-44 | GYTLTELS | 191 | FDPEDGET | 212 | ATEAAAGTPFFDY | 236 |
| GPA33-2RmhC-45 | GGSVTSTSYY | 192 | IYYSGST | 208 | ARDRGSGWYRDAFDI | 229 |
| GPA33-2RmhC-46 | GFTFDDYA | 184 | ISWNSGSI | 205 | AKDIGAVAGYGMDV | 237 |
| GPA33-2RmhC-47 | GDSYSSNSAA | 193 | TYYRSKWYN | 213 | AREKTGLYWYFDL | 238 |
| GPA33-2RmhC-48 | GGTFNTYA | 194 | IIPIFGSA | 214 | ARAYGGSAHYYSYGMDV | 239 |
| GPA33-2RmhC-49 | GFTFSSYG | 195 | ISTSSSSI | 215 | ARDGAYCGGDCYPYWNFDL | 240 |
| GPA33-2RmhC-50 | GYSFTDSW | 196 | IYPGDSDT | 216 | ARPYRDYSFDY | 241 |

**[Table 3-3]**

| SEQ ID NO | Code name | VH Sequence |
|---|---|---|
| 242 | GPA33-2RmhC-1 | QVQLQESGGGLVKPGGSLRLSCAASGFTFSSYNMNWVRQAPGKGLEWV SSISSRGDYIYYADSVKGRFTISRDNAKNSLYLQMSSLRAEDTAVYYCAR EAVGTPFDYWGQGTLVTVSS |
| 243 | GPA33-2RmhC-2 | QMQLVQSGAEVKKPGASVKVSCKASGYTFTGYYMHWVRQAPGRGLEW MGRINPNSGGTNYAQKFQGRVTMTRDTSISTAYMELSRLRSDDTVVYYC ARVESGAFDIWGQGTMVTVSS |
| 244 | GPA33-2RmhC-3 | QVQLVQSGGGLVKPGGSLRLSCAASGFTFSNYNMNWVRQAPGKGLEW VSSIDRSSTYIYYADSVKGRFTISRDNAKNSLYLQMNSLRAEDTAVYYCA REAVGTPFDYWGQGTLVTVSS |
| 245 | GPA33-2RmhC-4 | QVQLVQSGGGLVKPGGSLRLSCAASGFTFSNYNMNWVRQAPGKGLEW VSSISSSGDYIYYADSVKGRFTISRDDAKNSLYLQMNSLRAEDTAVYYCA REAIGTPFDYWGQGTLVTVSS |
| 246 | GPA33-2RmhC-5 | EVQLVESGGGLVKPGGSLRLSCAASGFTFSTYNMNWVRQAPGKGLEWV SSISSSGDYIYYADSVKGRFTISRDDAKNSLYLQMNSLRAEDTAVYYCAR EAIGTPFDYWGQGTLVTVSS |
| 247 | GPA33-2RmhC-6 | QVQLVESGGGLVKPGGSLRLSCAASGFTFSSYNMNWVRQAPGKGLEWV SSISSRGDYIYYADSVKGRFIISRDNAKNSLYLQMSSLRAEDTAVYYCARE AVGTPFDYWGQGTLVTVSS |
| 248 | GPA33-2RrrihC-7 | QVQLVQSGGGLVKPGGSLRLSCAASGFTFSSYNMNWVRQAPGKGLEWV SSIDRSSTYIYYADSVKGRFTISRDNAKNSLYLQMNSLRAEDTAVYYCAR EGPGTPFDYWGQGTLVTVSS |
| 249 | GPA33-2RmhC-8 | EVQLVESGGGLVKPGGSLRLSCAASGFTFSNYNMNWVRQAPGKGLEWV SSIVSSGDYIYYADSVKGRFTTSRDNAKNSLYLQMSSLRAEDTAVYYCA REAVGTPFDYWGQGTLVTVSS |
| 250 | GPA33-2RmhC-9 | QVQLVQSGGGLVKPGGSLRLSCAASGFTFSSYNMNWVRQAPGKGLEWV SSIDRSSTYIYYADSVKGRFTISRDNAKNSLYLQMNSLRAEDTAVYYCAR EAVGTPFDYWGQGTLVTVSS |
| 251 | GPA33-2RmhC-10 | QVQLVQSGGGLVKPGGSLRLSCAASGFTFSTYNMNWVRQAPGKGLEWV SSISSSGDYIYYADSVKGRFTISRDDAKNSLYLQMNSLRAEDTAVYYCAR EGPGTPFDYWGQGTLVTVSS |
| 252 | GPA33-2RmhC⁻11 | QVQLVESGGGLVQPGGSLRLSCAASGFTFSSYAMSWVRQAPGKGLEWV SAISGSGGSTYYADSVKGRFTISRDDSKNTLYLQMNSLRAEDTAVYYCA KGDIVVVPAALFDYWGQGTLVTVSS |
| 253 | GPA33-2RmhC-12 | QVQLVQSGGGLVKPGGSLRLSCAASGFTFSTYNMNWVRQAPGKGLEWV SSISSSGDYIYYADSVKGRFTISRDDAKNSLYLQMNSLRAEDTAVYYCAR EAIGTPFDYWGQGTLVTVSS |
| 254 | GPA33-2RmhC-13 | QMQLVQSRGGLVKPGGSLRLSCAASGFTFSSYNMNWVRQAPGKGLEW VSSISSRGDYIYYADSVKGRFTISRDNAKNSLYLQMSSLRAEDTAVYYCA REAVGTPFDYWGQGTLVTVSS |
| 255 | GPA33-2RmhC-14 | QVQLVQSGAEVKKPGSSVKVSCKASGGTFSSYAINWVRQAPGQGLEWM GGIIPIFGTADYAQKFRGRVTITADESTSTAYMELSSLRSEDTAVYYCAR GQQQLAYYQFYGMDVWGQGTTVTVSS |
| 256 | GPA33-2RmhC-15 | QMQLVQSEGGLVKPGGSLRLSCAASGFTFSSYNMNWVRQAPGKGLEWV SSISSSGDFMYYADSVKGRFTISRDNAKNSLYLQMSSLRAEDTAVYYCA REGPGTPFDYWGQGTLVTVSS |
| 257 | GPA33-2RmhC-16 | QVQLVQSGGGLVKPGGSLRLSCAASGFTFSNYNMNWVRQAPGKGLEW VSSISSSGDFMYYADSVKGRFTISRDDAKNSLYLQMNSLRAEDTAVYYC AREAIGTPFDYWGQGTLVTVSS |
| 258 | GPA33-2RmhC-17 | QVQLVQSGGGLVKPGGSLRLSCAASGFTFSTYSMNWVRQAPGKGLEWV SSISRDSAYIYYADSVKGRFTISRDNAKNSLYLQMNSLRAEDTAVYYCVR EGVAGAFDIWGQGTMVTVSS |

**[Table 3-4]**

| SEQ ID NO | Code name | VH Sequence |
|---|---|---|
| 259 | GPA33-2RmhC-18 | EVQLVESGGGLVKPGGSLRLSCAASGFTFSTYSMNWVRQAPGKGLEWV SSISRDSAYIYYADSVKGRFTISRDNAKNSLYLQMNSLRAEDTAVYYCVR EGVAGAFDIWGQGTMVTVSS |
| 260 | GPA33-2RmhC-19 | QMQLVQSGGGLVKPGGSLRLSCAASGFTFSTYSMNWVRQAPGKGLEWV SSISRDSAYIYYADSVKDRFTISRDNAKNSLYLQMNSLRAEDTAVYYCVR EGVAGAFGIWGQGTMVTASS |
| 261 | GPA33-2RmhC-20 | QVQLVQSGAEVKKPGSSVKVSCKASGGTFSSYAFNWVRQAPGQGLEW MGGIIPIFGTANYAQKFQGRVTITADESTSTAYMELSSLRSEDTAVYYCA RAYGGSAYYYNYGMDVWGQGTTVTVSS |
| 262 | GPA33-2RmhC-21 | QVQLVESGGGLVQPGRSLRLSCAASGFTFDDYAMHWVRQAPGKGLEW VSGISWNSGSIGYADSVKGRFTISRDNAKNSLYLQMNSLRAEDTALYYCA KDRWDTAMITPDAFDIWGQGTMVTVSS |
| 263 | GPA33⁻2RmhC-22 | HVTLKESGGGLVKPGGSLRLSCAASGFTFSTYSMNWVRQAPGKGLEWV SSISRDSAYIYYADSVKGRFTISRDNAKNSLYLQMNSLGAEDTAVYYCVR EGVAGAFDIWGQGTMVTVSS |
| 264 | GPA33-2RmhG-23 | QMQLVQSGGGLVQPGRSLRLSCTASGFTFGDYGMHWVRQVPGKGLEW VSGISWNSGRKGYADSVKGRFTISRDNAKNSLYLQMDSLRAEDTALYYC AKDMKIIRMEAAGPLEFWGQGTLVTVSS |
| 265 | GPA33-2RnihC-24 | QMQLVQSGGGLVKPGGSLRLSCAASGFTFSTYSMNWVRQAPGKGLEWV SSISRDSAYIYYADSVKGRFTISRDNAKNSLYLQMNSLRAEDTAVYYCVR EGVAGAFDIWGQGTMVTVSS |
| 266 | GPA33-2RmhC-25 | QMQLVQSGGGLVKPGGSLRLPCAASGFTFSSYNMNWVRQAPGKGLEW VSSISSSGDFMYYADSVKGRFTISRDNAKNSLYLQMSSLRAEDTAVYYC AREGPGTPFDYWGQGTLVTVSS |
| 267 | GPA33-2RmhC-26 | QVTLKESGGGLVKPGGSLRLSCAASGFTFSTYSMNWVRQAPGKGLEWV SSISRDSAYIYYADSVKGRFTISRDNAKNSLYLQMNSLRAEDTAVYYCVR EGVAGAFDIWGQGTMVTVSS |
| 268 | GPA33-2RmhC-27 | QVQLVQSGAEVKKPGSSVKVSCKASGGTFSSYAFSWVRQAPGQGLEW MGGIIPIFGTANYAQKFQDRVTITADESTSTAYMELSSLRSEDTAVYYCA RAYGGSAYYHNYGMDVWGQGTTVTVSS |
| 269 | GPA33-2RmhC-28 | QVQLVQSGAEVKKPGASVKVSCKGSGYTFTNYGISWVRQAPGQGLEWM GWISAYNGNTNYAQKLQGRVTMTTDTSTSTVYMELRSLRSDDTAVYYC ARDSPLNWGPDAFDIWGQGTMVTVSS |
| 270 | GPA33-2RmhC-29 | EVQLLESGPGLVKPSETLSLTCTVSGGSVSSTSYYWSWIRQPPGKGLEWI GYIYYSGSTNYNPSLKSRVTISVDTSKNQFSLKLSSMTAADTAVYYCARD RGSGWYRDAFDIWGQGTMVTVSS |
| 271 | GPA33-2RmhC-30 | QMQLVQSGGGLVKPGGSLRPSCAASGFTFSSYNMNWVRQAPGKGLEW VSSISSSGDFMYYADSVKGRFTISRDNAKNSLYLQMSSLRAEDTAVYYC AREGPGTPFDYWGQGTLVTVSS |
| 272 | GPA33-2RnihC-31 | QMQLVQSGGGLVKPGGSLRLSCAASGFTFSSYNMNWVRQAPGKGLEW VSSISSSGDFMYYADSVKGRFTISRDDAKNSLYLQMNSLRAEDTAVYYC AREGPGTPFDYWGQGTLVTVSS |
| 273 | GPA33-2Rmbd-32 | EVQLVESGGGLVQPGRSLRLSCAASGFTFDDYAMHWVRQAPGKGLEW VSGISWSSGSIGYADSVKGRFTISRDNAKSSLYLQMNSLRAEDTALYYCA KDITDSSGYYGMDAWGQGTLVTVSS |
| 274 | GPA33-2RmhC-33 | QMQLVQSGGGLVKPGGSLRLSCAAPGFTFSSYNMNWVRQAPGKGLEW VSSISSSGDFMYYADSVKGRFTISRDNAKNSLYLQMSSLRAEDTAVYYC AREGPGTPFDYWGQGTLVTVSS |
| 275 | GPA33-2RmhC-34 | QVQLVQSGGGLVKSGGSLRLSCAASGFTFSNYNMNWVRQAPGKGLEW VSSISSSGDFMYYADSVKGRFTISRDDAKNSLYLQMNSLRAEDTAVYYC AREGPGTPFDYWGQGTLVTVSS |

**[Table 3-5]**

| SEQ ID NO | Code name | VH Sequence |
|---|---|---|
| 276 | GPA33-2RmhC-35 | QMQLVQSGGGLVKPGGSLRLSCAASGFTFSSYNMNWVRQAPGKGLEW VSSISSSGDFMYYADSVKGRFTISRDNAKNSLYLQMSSLRAEDTAVYYC AREGPGTPLDYWGQGTLVTVSS |
| 277 | GPA33-2RmhC-36 | QVQLQESGGGLVKPGGSLRLSCAASGFTFSNFNMNWVRQAPGKGLEWV SSISSSGDFMYYADSVKGRFTISRDNAKNSLYLQMNSLRAEDTAVYYCA REGPGTPFDYWGQGTLVTVSS |
| 278 | GPA33-2RmhC-37 | QVQLVESGPGLVKPSETLSLTCTVSGGSISSYYWSWIRQPPGKGLEWIGYI YHSGSTNYNPSLKSRVTISVDTSKNQFSLKLSSVTAADTAVYYYARSRQ QLLRDYYYYGMDVWGQGTTVTVST |
| 279 | GPA33-2RmhC-38 | QMQLVQSGGGLVKPGGSLRLSCAASGFTFSSYNMNWVRQAPGKGLEW VSSISSSGDFMYYADSVKGRFTISRGNAKNSLYLQMSSLRAEDTAVYYC AREGPGTPFDYWGQGTLVTVSS |
| 280 | GPA33-2RmhC-39 | QMQLVQSGGGLVKPGGSLRLSCAASGFTFSSYNMNWVRQAPGKGLEW VSSISSSGDYMYYADSVKGRFTISRDNAKNSLYLQMNSLRAEDTAVYYC AREGPGTPFDYWGQGTLVTVSS |
| 281 | GPA33-2RmhC-40 | EVQLVESGGGLAQPGGSLRLSCAASGFTFSNYAMSWVRQAPGKGLEWV SAISGSGGSTYYADSVKGRFTISRDNSKNTLYLQMNSLRAEDTAVYYCA KGSGSYYKPVYYYYGMDVWGQGTTVTVSS |
| 282 | GPA33-2RmhC-41 | QVQLVQSGGGLVKPGGSLRLSCAASGFTFSNYNMNWVRQAPGKGLEW VSSISSSGDFMYYADSVKGRFTISRDDAKNSLYLQMNSLRAEDTAVYYC AREGPGTPFDYWGQGTLVIVSS |
| 283 | GPA33-2RmhC-42 | EVQLVESGGGLVQPGRSLRLSCAASGFTFDDYAMHWVRQAPGKGLEW VSGISWNSGSIGYADSVKGRFTISRDNAKNSLYLQMNSLRAEDTALYYCA KCDGSGSYGAFDIWGQGTMVTVSS |
| 284 | GPA33-2RmhC-43 | QVQLVQSGAEVKKPGSSVKVSCKASGGTFSSYAISWVRQAPGQGLEWM GGIIPIFGTANYAQKFQGRVTITADESTSTAYMELSSLRSEDTAVYYCAR APDILTSSGYYYYGMDVWGQGTTVTASS |
| 285 | GPA33-2RmhC-44 | QVQLVQSGAEVKKPGASVKVSCKVSGYTLTELSMHWVRQAPGKGLEWM GGFDPEDGETIYAQKFQGRVTMTEDTSTDTAYMELSSLRSEDTAVYYCA TEAAAGTPFFDYWGQGTLVTASS |
| 286 | GPA33-2RmhC-45 | QLQLQESGPGLVKPSETLSLTCTVSGGSVTSTSYYWSWIRQPPGKGLEWI GYIYYSGSTNYNPSLKSRVTISVDTSKNQFSLKLSSVTAADTAVYYCARD RGSGWYRDAFDIWGQGTMVTVSS |
| 287 | GPA33-2RmhC-46 | QVTLKESGGGLVQPGRSLRLSCAASGFTFDDYAMHWVRQAPGKGLEWV SGISWNSGSIGYADSVKGRFTISRDNAKNSLYLQMNSLRAEDTALYYCAK DIGAVAGYGMDVWGQGTTVTVSS |
| 288 | GPA33-2RmhC-47 | QVQLQESGPGLVKPSQTLSFTCAISGDSVSSNSAAWNWIRQSPSRGLEW LGRTYYRSKWYNDYALSVKSRITINPDTSKNQFSLHLNSVTPEDTAVYYC AREKTGLYWYFDLWGRGTLVTVSS |
| 289 | GPA33-2RmhC-48 | QVQLVQSGAEVKKPGSSVKVSCKASGGTFNTYAINWVRQAPGQGLEWM GGIIPIFGSANYAQKFQDRVTITADESTSTAYMELSSLRSEDTAVYYCAR AYGGSAHYYSYGMDVWGQGTLVTVSS |
| 290 | GPA33-2RmhC-49 | EVQLVESGGGLVQPGGSLRLSCAASGFTFSSYGMNWVRQAPGKGLEWV SYISTSSSSIYYADSVKGRFTISRDNAKNSLYLQMNSLRDEDTAVYYCAR DGAYCGGDCYPYWNFDLWGHGTLVTVSS |
| 291 | GPA33-2RmhC-50 | EVQLVESGAEVKKPGESLKISCKGSGYSFTDSWIGWVRQMPGKGLEWMG IIYPGDSDTRYSPSFQGQVTTSADKSISTAYLQWSSLKASDTAMYYCARP YRDYSFDYWGQGPWSPSR |

### [Example 5] Evaluation of binding activity of anti-GPA33 single chain Fv-His

After infecting Escherichia coli HB2151 with the three cloned anti-GPA33 antibody phages, periplasmic expression was performed by IPTG induction. The amino acid sequence of the scFv expressed in Escherichia coli is shown in Fig. 7. For fluorescent labeling, a sequence containing Cys (boxed portion 241-246) was introduced near the C-terminus of the scFv sequence, and a His tag for purification and a Myc tag sequence for detection (boxed portion 256-265) were added. In the periplasmic expression, of the three clones GPA33-2RC9, 17, and 28, the expression of GPA33-2RC17 was extremely low, and thus the remaining two clones were purified using a His tag column.

In order to evaluate the binding activity of the obtained two types of scFvs, FACS analysis was performed using the CHO-K1 cell strain expressing human and mouse GPA33 used in Fig. 4. That is, cells were prepared in PBS to a concentration of 2.0x10^6 cells/1 mL, and 100 µL was dispensed into 1.5 mL tubes. After centrifugation at 200xg and RT (room temperature) for 3 min and removing the supernatant, 500 µL of PBS containing 2% BSA was added to each tube to suspend the cells, and the cells were washed by centrifuging again and removing the supernatant. 100 µL of PBS was added to suspend the cells, and 100 µL of PBS containing 0.1 µg of scFv was added thereto, and the cells were thoroughly mixed by pipetting, and then reacted at 4°C for 30 min with shaking. After washing twice with 500 µL of PBS containing 2% BSA, 50 µL of PBS containing 0.1 µL of 1 mg/mL Anti-His-tag-Biotin antibody (MBL) was added to the cell solution suspended in 50 µL of PBS, and after incubation at 4°C for 30 min, the cells were washed once with 500 µL of PBS containing 2% BSA. After 50 µL of PBS containing 0.1 uL of 1 mg/mL SA-PE (VECTOR LABORATORIES) was added to the cell solution suspended in 50 µL of PBS, and after reaction at 4°C for 30 min, the cells were washed once with 500 µL of PBS containing 2% BSA. After centrifugation, the cells were suspended in 500 µL of PBS, passed through a mesh filter, and subjected to FACS analysis using a flow cytometer (S3e, BioRad). The results are shown in Fig. 8.

As shown in Fig. 8, when 2RC9 and 2RC28 scFvs were added, in cells with high GFP fluorescence on the horizontal axis (considered to have high GPA33 expression), a shift in PE fluorescence on the vertical axis due to binding of single-chain Fv was observed compared to the control (black: anti-His tag antibody, SA-PE not added). On the other hand, no shift was observed when only anti-His tag antibody and SA-PE were added without adding scFv antibody. From the above, it was shown that the two types of scFv have binding activity to human and mouse GPA33.

### [Example 6] Verification of intestinal migration of anti-GPA33 scFv-His antibody

To verify the intestinal migration of anti-GPA33scFv-His antibody, fluorescent labeling was performed using Cys introduced near the C-terminus. That is, 6.72 µL of 10 mM TCEP aqueous solution (67.2 nmol) was added to 300 µL of 112 µM scFv in PBS (33.6 nmol), and the mixture was reacted at room temperature for 30 min (molar ratio 1:2). Then, 9.9 µL of 109.6 µM Bromoacetamido-dPEG (registered trademark)₄-amido-DBCO (Quanta BioDesign) DMSO solution (99 nmol) was added, and the mixture was reacted at room temperature for 4 hr (molar ratio 1:3). After confirmation of the DBCO modification of scFv by LC-MS, 162 µL of 788 µM IR800-Azide (LI-COR) aqueous solution (127.8 nmol) was added, and the mixture was reacted at room temperature for 30 min (molar ratio 1:4). The process of fluorescent labeling of scFv is shown in Fig. 9. Fig. 9(A) shows an outline of the reaction from reduced scFv to DBCO labeling and further to IR-800 fluorescent labeling by click reaction. Fig. 9(B) shows the results of LC-MS analysis of the reaction products at each step using 2CR9 scFv (mass peak of each scFv is split into two because part of the N-terminal Gln is pyroglutamylated and a dehydration reaction has occurred). These results show that each step proceeds almost quantitatively. Finally, to remove unreacted fluorescent reagents, etc., the fluorescence-labeled scFv was purified by size exclusion chromatography using NAP DNA Purification Columns (Sephadex G-25, Cytiva) equilibrated with PBS.

As described above, fluorescence-labeled scFv was used to perform in vivo imaging of mice. That is, 50 µg of the prepared fluorescence-labeled scFv was administered to two mice from the tail vein, and after 1 or 2 hr, the mice were anesthetized and euthanized, perfused, and the organs were excised and washed, and fluorescent images of the intestines were taken using a multi-imaging system (FUSION SOLO, MS Equipment). As a negative control, 4D5 scFv which was fluorescently labeled similarly was used (4D5 is an scFv in which VH and VL of Trastuzumab are linked). The results are shown in Fig. 10(A).

As shown in Fig. 10(A), the anti-GPA33-2RC9 and anti-GPA33-2RC28 clones clearly accumulated in the intestine compared to the 4D5 scFv used as a control. The results of quantification of the accumulated amount of fluorescence are shown in Fig. 10(B). As shown in Fig. 10(B), the GPA33-2RC9 and 2RC28 clones had 1.7-fold and 2.3-fold higher fluorescence intensity accumulated in the intestine than 4D5 at 1 hr after administration, and 1.9-fold and 2.8-fold higher fluorescence intensity accumulated in the intestine at 2 hr after administration, respectively. These results indicate that the anti-GPA33 scFv antibody obtained at this time shows high intestinal migration.

### [Example 7] Evaluation 1 of binding activity of anti-GPA33 single-chain Fv-Fc

Based on the amino acid sequence of the anti-GPA33 single-chain Fv antibody shown in Fig. 5, single-chain Fv-Fc linking single-chain Fv and human IgG1-Fc was prepared using an animal cell expression system using HEK293. The amino acid sequences of the three types of single-chain Fv-Fc (GPA2RC9-Fc, GPA2RC17-Fc, GPA2RC28-Fc) prepared are shown in Fig. 11.

The binding activity of the prepared three types of anti-GPA33 single-chain Fv-Fc against hGPA33-expressing cells and mGPA33-expressing cells was evaluated by FACS. Concretely, 2.0×10^5 cells were washed once with 500 µL of 2% BSA/PBS, suspended in 50 µL of 2% BSA/PBS, and 50 µL of a solution of anti-GPA33 single chain Fv-Fc dissolved at 20 µg/mL in 2% BSA/PBS was added (final concentration: 10 µg/mL), and the mixture was reacted at 4°C for 1 hr with shaking. After washing twice with 500 µL of 2% BSA/PBS, 50 µL of a solution of Alexa FluorR 594-conjugated AffiniPure Goat Anti-Human IgG, Fcy Fragment Specific (Jackson Immuno Research Laboratories) dissolved at 10 µg/mL was added to 50 µL of the cell solution suspended again in 2% BSA/PBS, mixed, and reacted at 4°C for 30 min. The cells were then washed once with 500 µL of 2% BSA/PBS. The washed cells were suspended in 500 µL of 1×PBS and passed through a mesh filter, and FACS was performed using a flow cytometer (S3e, BioRad). The results are shown in Fig. 12.

As shown in Fig. 12, the tendency of Alexa FluorR 594 on the vertical axis, which indicates the binding of single-chain Fv-Fc, increased with increasing GFP intensity on the horizontal axis. That is, it was confirmed that all three types of single-chain Fv-Fc (GPA2RC9-Fc, GPA2RC17-Fc, GPA2RC28-Fc) retained the binding ability to human and mouse GPA33.

### [Example 8] Evaluation 2 of binding activity of anti-GPA33 single-chain Fv-Fc

The affinity of three types of anti-GPA33 single-chain Fv-Fc was analyzed by surface plasmon resonance. Concretely, the analysis was performed at 25°C using a Biacore T200 (GE Healthcare). According to the BIAcore protocol, hGPA33 and mGPA33 (recombinant proteins with a C-terminal His tag) were immobilized on Series S Sensor Chip CM5 (Cytiva) by the amine coupling method to RU = 126.2 and RU = 82.5, respectively. Single-chain Fv-Fc solutions (concentration series of 100 nM, 50 nM, 25 nM, 12.5 nM, 6.25 nM, or 20 nM, 10 nM, 5 nM, 2.5 nM, 1.25 nM) prepared using HBS-EP buffer (pH 7.4) (0.01 M HEPES, 0.15 M NaCl, 3 mM EDTA, 0.005% tween) were run at a flow rate of 50 µL/min for 180 sec to measure the binding reaction. Thereafter, HBS-EP buffer (pH 7.4) was run for 600 sec to measure the dissociation reaction. The obtained sensorgram was analyzed using the 1:1 binding model of BIA evaluation software, and the binding parameter was calculated. The results are shown in Table 4.

**[Table 4]**

| | | | | |
|---|---|---|---|---|
| 2RC9 scFv-Fc | hGPA33 | *k*ₐ = 2.4×10⁻6 nM | *k*_{d} = 8.6×10⁻-3 nM | K_{D} = 3.6 nM |
| | mGPA33 | *k*ₐ = 3.0×10⁻5 nM | *k*_{d} = 4,3×10⁻-3 nM | K_{D} = 14.3 nM |
| 2RC17 scFv-Fc | hGPA33 | *k*ₐ = 5.5×10⁻6 nM | *k*_{d} = 7.7×10⁻-3 nM | K_{D} = 1.4 nM |
| | mGPA33 | *k*ₐ *=* 1.7×10⁻7 nM | *k*_{d} = 6.6×10⁻-2 nM | K_{D} = 3.8 nM |
| 2RC28 scFv-Fc | hGPA33 | *k*ₐ = 6.6×10⁻5 nM | *k*_{d} = 3.8×10⁻-3 nM | K_{D}= 5.7 nM |
| | mGPA33 | *k*ₐ = 5,4×10⁻5 nM | *k*_{d} = 3.7×10⁻-3 nM | K_{D} = 6.9 nM |

As shown in Table 4, the three types of anti-GPA33 single-chain Fv-Fc showed relatively high binding affinity to human GPA33 and mouse GPA33 (Kd: 1.4-5.7 nM (human GPA33), 3.6-15 nM (mouse GPA33)). Among them, 2RC17-scFv-Fc showed the strongest binding activity to both human GPA33 and mouse GPA33.

### [Example 9] Verification of intestinal migration of anti-GPA33 scFv-Fc antibody

To verify the intestinal migration ability of the three types of anti-GPA33 scFv-Fc antibodies prepared, these antibodies were fluorescently labeled with IRDye (registered trademark) 800CW using the CCAP method. Then, the obtained fluorescence-labeled antibodies were administered from the tail vein of mice, and the migration of these antibodies to the intestine was verified by fluorescent imaging. The details are explained below.

First, the fluorescent labeling of the anti-GPA33 single-chain Fv-Fc antibody was performed using the "CCAP method" developed by the present inventors (see Kishimoto, S. et al. (2019) Bioconjugate Chemistry, 30, 698-702 and JP-B-6872181). In this method, two types of products are generated in which the IgG-binding peptide is monovalently or bivalently bound to Lys248 on Fc of human IgG. The divalent product is not suitable for use as an antibody drug because it impairs the binding ability of the antibody to FcRn and has an extremely short half-life in blood. Therefore, only the monovalent products were obtained by a method to remove divalent products and purify only monovalent products by using an affinity peptide column in which an IgG-binding peptide is linked to a column resin, which was separately developed by the present inventors (see WO2021/080008). An outline of the fluorescent labeling process of anti-GPA33 single-chain Fv-Fc by the CCPA method and the reaction confirmation results of the fluorescence-labeled anti-GPA33 single-chain Fv-Fc are shown in Fig. 13.

In the reaction between the CCAP reagent of the azidized IgG binding peptide having an azide group added to the end and the anti-GPA33 single-chain Fv-Fc (2RC17), the mass of the molecular species with a mass of 105192 Da shown in Fig. 13(A) (molecular species with a larger mass are molecular species generated by the diversity of N-glycans added to Fc) increased to 108054 Da and 11923 Da. These were found to correspond to the molecular species in which the azidized IgG binding peptide was monovalently or divalently bound. Furthermore, the mass spectrometry results of the monovalent product purified from this mixture by using an affinity peptide column in which the IgG binding peptide was linked to the column resin are shown in Fig. 13(C), from which it can be seen that only the monovalent species was purified cleanly. Finally, this monovalent azidized anti-GPA33 single-chain Fv-Fc was mixed with IRDye (registered trademark) 800CW DBCO (LICOR) and linked by click reaction to prepare IRDye (registered trademark) 800CW-labeled anti-GPA33 single-chain Fv-Fc (2RC17). The results of mass spectrometry are shown in Fig. 13(D). Since an increase in mass of 1262 (theoretical value: 1259) was observed, it was confirmed that the antibody was quantitatively fluorescently labeled in a monovalent form. Using a similar method, IRDye (registered trademark) 800CW-labeled anti-GPA33 single-chain Fv-Fc (2RC9) and monovalently modified fluorescence-labeled Trastuzumab (Tmab) were prepared as a control antibody.

Using the prepared fluorescence-labeled antibodies (fluorescence-labeled 2RC9 scFV-Fc, fluorescence-labeled 2RC9 scFV-Fc, fluorescence-labeled Trastuzumab), fluorescence imaging was performed in mice. That is, fluorescence-labeled scFv-Fc was administered to four mice per antibody from the tail vein (50 µg/mouse), and after 2, 5, 24, or 48 hr, the mice were perfused under anesthesia, and the abdomen was opened. Fluorescence images of the internal organs in the open state were obtained using a multi-imaging system (FUSION SOLO S, Vilber GmbH). The results are shown in Fig. 14.

In Fig. 14, the upper panel of the images of the mouse laparotomy after administration of each antibody shows actual images, and the lower panel shows fluorescent images. In the case of 2RC9 single-chain Fv-Fc, increased fluorescence was observed in the intestine after 24 hr, and the intensity decreased after 48 hr. In the case of 2RC17 single-chain Fv-Fc, accumulation in the intestine was observed from 2 hr after administration, which increased from 5 hr to 24 hr, and retention in the intestine was observed even after 48 hr. On the other hand, in the case of the control antibody, accumulation in the internal organs was not observed at 2 and 5 hr, accumulation in the liver was observed at 24 hr, and the fluorescence decreased after 48 hr. From the above, it was suggested that both types of anti-GPA33 single-chain Fv-Fc antibodies (2RC9 scFV-Fc antibody and 2RC9 scFV-Fc antibody) have the ability to accumulate in the intestine; in particular, the 2RC17 scFv-Fc antibody has a significantly stronger ability to accumulate in the intestine than the control antibody.

The results shown in Fig. 14 include fluorescence-labeled antibodies in the contents (feces) of the intestinal tract, and do not necessarily indicate only antibodies selectively accumulated in the intestine. Therefore, in order to more accurately examine the intestinal accumulation ability of each fluorescence-labeled antibody, the mouse intestine was excised, the intestinal contents were washed and removed, and fluorescent images of the intestinal tissue were obtained. The results are shown in Fig. 15.

As shown in Fig. 15 (the upper panel is the actual image, and the lower panel is the fluorescent image), the accumulation of the 2RC9 scFv-Fc antibody and the 2RC17 scFv-Fc antibody in the intestine clearly and significantly increased compared to the control antibody (Tmab). Furthermore, the accumulation in the intestine of both antibodies reached maximum at 24 hr after administration. The results of confirmation of the accumulation of antibodies in organs other than the intestine are shown in Fig. 16 ((1) brain, (2) heart, (3) kidney, (4) liver from the left).

As shown in Fig. 16, none of the fluorescence-labeled antibodies accumulated in organs other than the liver. While the 2RC9 scFv-Fc antibody and the 2RC17 scFv-Fc antibody were confirmed to accumulate in the liver, the amount thereof was relatively small. On the other hand, the control antibody (Tmab) accumulated in large amounts in the liver.

In order to quantitatively evaluate the accumulation of the anti-GPA33 single-chain Fv-Fc antibody in the intestine, the fluorescence intensity of the entire intestine was quantified by integrating the fluorescence intensity by area, using the quantification tool attached to the multi-imaging system (FUSION SOLO S), based on the image data in Fig. 15. The results are shown in Fig. 17.

As shown in Fig. 17, the accumulation of both the 2RC9 scFv-Fc antibody and the 2RC17 scFv-Fc antibody in the intestine reached a peak at 24 hr after administration. Furthermore, when compared at 24 hr after administration, the 2RC9 scFv-Fc antibody accumulated in the intestine 7.3-fold more and the 2RC17 scFv-Fc antibody accumulated in the intestine 24-fold more, than the control antibody Tmab.

### [Example 10] Construction of expression vectors encoding various Fc fusions (anti-GPA33 scFv-Fc, human R-spondin 1 CRD fragment-Fc, and human R-spondin 4 CRD fragment-Fc)

Six types of DNA fragments (below), in which three types of anti-GPA33 scFv (2RC9, 2RC17, 2RC28), a control antibody scFv (Cont), human R-spondin 1 CRD fragment (R1CRD), and human R-spondin 4 CRD fragment (R4CRD) were each linked to nucleic acids encoding human IgG1-derived Fc (mutants with reduced ADCC and CDC activity) were obtained by outsourcing the production to Thermo Scientific. An expression vector was constructed by inserting the six types of the synthetic DNA fragments between the CMV promoter and bovine growth hormone polyA site of the pcDNA3 vector (Thermo Fisher). In addition, the production of expression vectors for Fc fusions of full-length human R-spondin 1 (Rspo1FL) (below) and preparation of recombinants were outsourced to TechnoPro Co., Ltd. to obtain them.
(1) Cont-scFv-Fc (SEQ ID NO: 292)
(2) R1CRD-Fc (SEQ ID NO: 293)
(3) R4CRD-Fc (SEQ ID NO: 294)
(4) GPA2RC9-scFv-Fc (SEQ ID NO: 295)
(5) GPA2RC17-scFv-Fc (SEQ ID NO: 296)
(6) GPA2RC28-scFv-Fc (SEQ ID NO: 297)
(7) Rspo1FL-Fc (SEQ ID NO: 298)

### [Example 11] Co-expression of each anti-GPA33 scFv-Fc and human R-spondin 1 CRD fragment-Fc in cultured cells

In order to express heterodimers of each anti-GPA33 scFv-Fc and human R-spondin 1 CRD fragment-Fc, co-expression of anti-GPA33 scFv-Fc and human R-spondin 1 CRD fragment-Fc in cultured cells was performed using the expression vector obtained in Example 10. Assuming that each Fc fusion is expressed at the same level, the expression ratio of heterodimer: GPA33 scFv-Fc homodimer: human R-spondin 1 CRD fragment-Fc homodimer in the culture supernatant is 2:1:1. In practice, since the expression level of each Fc fusion varies, the proportion of heterodimers in all Fc fusions expressed in the supernatant is less than the theoretical value. Fig. 18 shows the structures of the anti-GPA33 scFv-Fc and R-spondin 1 CRD fragment-Fc heterodimer, the R-spondin 1 CRD fragment-Fc homodimer, and the GPA33 scFv-Fc homodimer.

The expression vectors for three types of anti-GPA33 scFv-Fc (GPA2RC9-scFv-Fc, GPA22RC17-scFv-Fc, GPA22RC28-scFv-Fc), control scFv-Fc (Cont-scFv-Fc), and human R-spondin 1 CRD fragment-Fc (R1CRD-Fc) were introduced into HEK293T (RIKEN CELL BANK: RCB2202) cells by the electric pulse method using NEPA21 (NEPA GENE, 150 mV, 2.5 msec). The following five combinations were used:
(i) GPA2RC9- scFv-Fc×R1CRD-Fc,
(ii) GPA2RC17- scFv-Fc×R1CRD-Fc,
(iii) GPA2RC28- scFv-Fc×R1CRD-Fc,
(iv) Cont-scFv-Fc×R1CRD-Fc,
(v) R1CRD-Fc alone.

HEK293T cells introduced with the expression vectors were seeded on a 6 cm dish and cultured in DMEM medium (FUJIFILM Wako Pure Chemical) containing 10% FBS (Nichirei), and the culture supernatant was collected after 96 hr. The collected culture supernatant was sterilized with a PVDF filter (Sigma-Aldrich) and then subjected to concentration measurement by ELISA method.

The ELISA method was performed as follows:
(1) Anti-human IgG antibody (SIGMA) was coated on an assay plate (NUNC) .
(2) The plate was washed and blocked after removing the antibody solution.
(3) The blocking solution was removed, and after washing, culture supernatant of unknown concentration was added to the plate and incubated.
(4) Culture supernatant was removed, and after washing, enzyme-labeled secondary antibody (SIGMA) was added to the plate and incubated.
(5) Peroxidase colorimetric substrate (Sumitomo Bakelite) was added to develop color.
(6) Color development was measured with a NIVO plate reader (PerkinElmer), and the concentration of total Fc fusions including heterodimeric Fc fusions in each culture supernatant was determined based on a calibration curve prepared using human IgG of known concentration.

### [Example 12] Measurement of Wnt/β-catenin signal promoting activity of culture supernatant containing heterodimer of anti-GPA33 antibody scFv-Fc and human R-spondin 1 CRD fragment-Fc

The Wnt/β-catenin signal promoting activity of culture supernatant containing heterodimer was measured as shown below. ONE-Glo^{™} + Tox Luciferase Reporter and Cell Viability Assay (Promega) was used for the measurement. EGFP-GPA33 expression vector (ORIGENE: RG210225) or Mock vector was introduced into HEK293STF cells (ATCC: CRL-3249) by the electric pulse method using NEPA21 (NEPA GENE, 150V, 2.5 msec). Cells introduced with each vector were seeded on a 6 cm dish and cultured in DMEM medium containing 10% FBS (Nichirei), and after 48 hr, the cells were collected and seeded on a 96-well plate. After 24 hr, 100 µL of (1) control medium or (2) culture supernatant containing heterodimer (adjusted such that the total Fc fusion concentration was 100 ng/mL based on the results of ELISA analysis; final concentration 50 ng/mL), or (3) recombinant Rspo1FL-Fc (100 ng/mL, final concentration 50 ng/mL) prepared using the recombinant obtained in Example 10 was added to each well. Furthermore, Recombinant Afamin/Wnt3a (MBL) was added at that time to a final concentration of 100 ng/mL. After 18 hr from the addition, 100 µL of the supernatant in the well was removed, and 100 µL of Cell Titer-Flour^{™} Reagent was added. After incubation at 37°C for 30 min, the mixture was stirred and further incubated at 37°C for 15 min. Then, 100 µL of the supernatant was transferred to a CulturPlate^{™}96 (PerkinElmer) and the fluorescence value was measured using a Microplate Reader SH-9000 (CORONA). 100 µL of ONE-Glo^{™} Reagent was added to the plate and the mixture was incubated at room temperature for 5 min, after which the luminescence value was measured using a NIVO plate reader (PerkinElmer). The signal intensity was calculated as the luminescence/fluorescence value, and the signal intensity of each supernatant was quantified relative to the value of the control medium containing recombinant RspolFL at a final concentration of 50 ng/mL, which was set to 1. The measurement results are shown in Fig. 19.

As shown in Fig. 19, in GPA33-expressing cells, the culture supernatants containing the anti-GPA33 scFv-Fc and R1CRD-Fc heterodimers all showed higher values than the culture supernatant containing the R1CRD-Fc homodimer. On the other hand, in cells not expressing GPA33, the activity of all the supernatants was low, and there was no difference between the activity of the heterodimers and the R1CRD-Fc homodimer. That is, it was revealed that the heterodimers of anti-GPA33 scFv-Fc and R1CRD-Fc show high activity in a GPA33 antigen-dependent manner.

### [Example 13] Co-expression of each anti-GPA33 scFv-Fc and human R-spondin4 CRD fragment-Fc in cultured cells

To express heterodimers of each anti-GPA33 scFv-Fc and human R-spondin4 CRD fragment-Fc, co-expression of each anti-GPA33 scFv-Fc and human R-spondin4 CRD fragment-Fc in cultured cells was performed in the same manner as in Example 11.

The expression vectors for each anti-GPA33 scFv-Fc (GPA2RC9-scFv-Fc, GPA22RC17-scFv-Fc, GPA22RC28-scFv-Fc), control scFv-Fc (Cont-scFv-Fc), and human R-spondin4 CRD fragment-Fc fusion (R4CRD-Fc) were introduced into HEK293T cells by the electric pulse method using NEPA21 (NEPA GENE, 150 mV, 2.5 msec). The following five combinations were used:
(i) GPA2RC9-scFv-Fc×R4CRD-Fc,
(ii) GPA2RC17-scFv-Fc×R4CRD-Fc,
(iii) GPA2RC28-scFv-Fc×R4CRD-Fc,
(iv) Cont-scFv-Fc×R4CRD-Fc,
(v) R4CRD-Fc.

HEK293T cells introduced with the expression vector were seeded on a 6 cm dish and cultured in DMEM medium (FUJIFILM Wako Pure Chemical) containing 10% FBS (Nichirei), and the culture supernatant was collected after 96 hr. The collected culture supernatant was sterilized with a PVDF filter (Sigma-Aldrich) and then subjected to concentration measurement by ELISA method. The concentration of the total Fc fusions, including the heterodimer Fc fusions, in each culture supernatant was determined by ELISA method in the same manner as in Example 11. The structures of the heterodimer of anti-GPA33 scFv-Fc and R-spondin4 CRD fragment-Fc, the R-spondin4 CRD fragment-Fc homodimer, and the GPA33 scFv-Fc homodimer are shown in Fig. 20.

### [Example 14] Measurement of Wnt/β-catenin signal promoting activity of culture supernatant containing heterodimer of anti-GPA33 scFv-Fc and human R-spondin4 CRD fragment-Fc

The Wnt/β-catenin signal promoting activity of culture supernatant containing the heterodimer was measured by the same method as in Example 12. The measurement results are shown in Fig. 21.

As shown in Fig. 21, in GPA33-expressing cells, the culture supernatants containing the anti-GPA33 antibody scFv-Fc and R4CRD-Fc heterodimers all showed higher activity than the R4CRD-Fc homodimer. On the other hand, in cells not expressing GPA33, the activity of all the supernatants was low, and there was no difference in activity between the heterodimer and the R4CRD-Fc homodimer. That is, it was revealed that the anti-GPA33 scFv-Fc and R4CRD-Fc heterodimers showed high activity in a GPA33 antigen-dependent manner.

### [Example 15] Construction of Knobs into Holes type vector for heterodimeric expression of anti-GPA33 scFv-Fc and human R-spondin 1 CRD fragment-Fc

For each of the three types of anti-GPA33 scFv (2RC12, 2RC17, 2RC28), a control antibody (Cont), and the human R-spondin 1 CRD fragment (R1CRD), 10 types (below) of DNA fragments to which Knob-type and Hole-type mutations of human IgG1-derived Fc (mutants with reduced ADCC and CDC activity) are linked, respectively were obtained by outsourcing the production to Thermo Scientific.
(1) Cont-scFv-Fc-Knob (SEQ ID NO: 299)
(2) Cont-scFv-Fc-Hole (SEQ ID NO: 300)
(3) Rspo1CRO-scFv-Fc-Knob (SEQ ID NO: 301)
(4) Rspo1CRO-scFv-Fc-Hole (SEQ ID NO: 302)
(5) GPARC12-scFv-Fc-Knob (SEQ ID NO: 303)
(6) GPARC12-scFv-Fc-Hole (SEQ ID NO: 304)
(7) GPARC17-scFv-Fc-Knob (SEQ ID NO: 305)
(8) GPARC17-scFv-Fc-Hole (SEQ ID NO: 306)
(9) GPARC28-scFv-Fc-Knob (SEQ ID NO: 307)
(10) GPARC28-scFv-Fc-Hole (SEQ ID NO: 308)

The above 10 types of synthetic DNA fragments were inserted between the CMV promoter and bovine growth hormone polyA site of the pcDNA3 vector (Thermo Fisher) to construct an expression vector.

### [Example 16] Co-expression of each anti-GPA33 scFv-Fc (Knob type, Hole type) and human R-spondin 1 CRD fragment-Fc (Knob type, Hole type) in cultured cells

To express heterodimers of each anti-GPA33 scFv-Fc and human R-spondin 1 CRD fragment-Fc, co-expression of each anti-GPA33 scFv-Fc (Knob type, Hole type) and human R-spondin 1 CRD fragment-Fc (Knob type, Hole type) in cultured cells was performed using the expression vector obtained in Example 15. When anti-GPA33 scFv-Fc Knob type and human R-spondin 1 CRD fragment-Fc Hole type, or anti-GPA33 scFv-Fc Hole type and human R-spondin 1 CRD fragment-Fc Knob type are co-expressed, it is considered that only heterodimers are expressed in the culture supernatant.

Various combinations of anti-Knob type x Hole type expression vectors were introduced into HEK293T cells by the electric pulse method using NEPA21 (NEPA GENE, 150 mV, 2.5 msec). The following seven combinations were used:
(i) GPA2RC12-scFv-Fc-Knob×R1CRD-Fc-Hole,
(ii) GPA2RC12-scFv-Fc-Hole×R1CRD-Fc-Knob,
(iii) GPA2RC17-scFv-Fc-Knob×R1CRD-Fc-Hole,
(iv) GPA2RC17-scFv-Fc-Hole×R1CRD-Fc-Knob,
(v) GPA2RC28-scFv-Fc-Knob×R1CRD-Fc-Hole,
(vi) GPA2RC28-scFv-Fc-Hole×R1CRD-Fc-Knob,
(vii) Cont-scFv-Fc-Hole×R1CRD-Fc-Knob.

HEK293T cells introduced with the expression vector were seeded on a 6 cm dish and cultured in DMEM medium (FUJIFILM Wako Pure Chemical) containing 10% FBS (Nichirei), and the culture supernatant was collected after 96 hr. The collected culture supernatant was sterilized with a PVDF filter (Sigma-Aldrich) and then subjected to concentration measurement by ELISA method. The concentration of the total Fc fusions, including the heterodimer Fc fusions, in each culture supernatant was determined by ELISA method in the same manner as in Example 11. The structures of anti-GPA33-scFv-Fc-Knob×R1CRD-Fc-Hole and anti-GPA33-scFv-Fc-Hole×R1CRD-Fc-Knob are shown in Fig. 22.

### [Example 17] Measurement of Wnt/β-catenin signal promoting activity of culture supernatant containing Knobs into Hole type anti-GPA33 scFv-Fc/human R-spondin 1 CRD fragment-Fc heterodimer

The Wnt/β-catenin signal promoting activity of culture supernatant containing Knobs into Hole type heterodimer was measured in the same manner as in Example 11. The signal intensity was calculated as the luminescence/fluorescence value, and the signal intensity of each supernatant was quantified relative to the value of the control medium containing recombinant Rspo1FL at a final concentration of 50 ng/mL, which was set to 1. The measurement results are shown in Fig. 23.

As shown in Fig. 23, in GPA33-expressing cells, the culture supernatants containing anti-GPA33 scFv-Fc and R1CRD-Fc Knobs into Holes heterodimers all showed higher activity than the R1CRD-Fc homodimer. On the other hand, in cells not expressing GPA33, the activity of all the supernatants was low, and there was no difference between the activity of the Knobs into Holes heterodimer and the R1CRD-Fc homodimer. That is, it was revealed that anti-GPA33 scFv-Fc and R1CRD-Fc Knobs into Holes heterodimers showed high activity in a GPA33 antigen-dependent manner.

### [Example 18] Design of anti-GPA33 antibody scFv-Rspo fragment fusion and preparation of recombinant protein

Recombinant proteins of fusions obtained by combining the anti-GPA33 human antibody scFv fragments G9 (2R_C9) and G17 (2R_C17) obtained in Example 10, the control antibody scFv fragment 4D5 specific to human HER2 ((1) Carter et al., 1992, DOI: 10.1073/pnas.89.10.4285), various R-spondin ((2) Rspo; Park et al., 2018, DOI: 10.1074/jbc.RA118.002743)-derived fragments, mutant human IgG1-derived Fc fragments (Fcm), and His tag fragments (His), as shown in Table 5, were obtained by outsourcing the production to Biointron (Shanghai, China). The sequence numbers of each fusion and each fragment are shown in Table 5. The human IL-10 secretory signal sequence (IL-10ss, SEQ ID NO: 342) was used as the signal sequence for secretion and expression in HEK293 cells used in recombinant protein production. R1CRD: R-spondin 1 cysteine rich domain (active domain); R2CRD: R-spondin 2 cysteine rich domain (active domain); R4CRD: R-spondin 4 cysteine rich domain (active domain); R1FU1: the first half of the R-spondin 1 active domain; R2FU1: the first half of the R-spondin 2 active domain; R1CRDCys10: the R-spondin 1 active domain with the Cys residue at the most C-terminus deleted.

**[Table 5]**

| fusion name (SEQ ID NO) | scFv fragment name (SEQ ID NO) | R-spo fragment name (SEQ ID NO) | Fcm fragment/His fragment (SEQ ID NO) |
|---|---|---|---|
| G17-R1CRD-His(309) | G17(330) | R1CRD(333) | His(339) |
| G9-R1CRD-His(310) | G9(331) | R1CRD(333) | His(339) |
| 4D5-R1CRD-His(311) | 4D5 (332) | R1GRD(333) | His(339) |
| R1GRD-Fcm (312) | - | R1CRD(333) | Fcm(340) |
| R1CRD-GS3-Fcm(313) | - | R1CRD(333) | GS3 linker-Fcm (341) |
| R4CRD-GS3-Fcm(314) | - | R40RD(334) | GS3 linker-Fcm (341) |
| G17-R4CRD-His(315) | G17(330) | R4CRD (334) | His(339) |
| G9-R4CRD-His(316) | G9(331) | R4GRD(334) | His(339) |
| R1CRD-G17-His(317) | G17(330) | R1CRD(333) | His(339) |
| R1CRD-G9-His(318) | G9(331) | R1GRD(333) | His(339) |
| G17-R1CRD-GS3-Fcm (319) | G17(330) | R1CRD(333) | GS3 linker-Fcm (341) |
| G9-R1CRD-GS3-Fcm(320) | G9(331) | R1CRD(333) | GS3 linker-Fcm (341) |
| 4D5-R1CRD-GS3-Fcm(321) | 4D5(332) | R1CRD(333) | GS3 linker-Fcmi (341) |
| G17-R2CRD-His(322) | G17(330) | R2CRD (335) | His(339) |
| 4D5-R2CRD-His(323) | 4D5(332) | R2CRD(335) | His(339) |
| G9-R4CRD-GS3-Fcm(324) | G9(331) | R4CRD(334) | GS3 linker-Fcm(341) |
| 4D5-R4CRD-GS3-Fcm(325) | 4D5(332) | R4CRD(334) | GS3 linker-Fcm (341) |
| G9-R1FU1-His(326) | G9(331) | R1FU1(336) | His(339) |
| G9-R1CRDCys10-His(327) | G9(331) | R1CRDCys10(337) | His(339) |
| G9-R2FU1-His (328) | G9 (331) | R2FU1(338) | His(339) |
| G9_R2CRD-His(329) | G9(331) | R2CRD(335) | His(339) |

### [Example 19] Preparation of conjugate of R1CRD-Fcm and anti-GPA33 antibody scFv-Rspo fragment by tCAP

### (1) tCAP modification of R1CRD-Fcm (SEQ ID NO: 312)

A PBS solution of R1CRD-Fcm (87.2 µM, 2000 µL) prepared in Example 18 was mixed with a DMSO solution of tCAP(3N) (code. ZY-562 Lot.752-211101) (10 mM, 69.8 uL) (1:4 molar ratio, pH 7.4, room temperature). After lapse of 10 min, 231 µL of 1 M NaHCO3 (pH 8.9) was added (final concentration 100 mM) and incubation was performed for 1.5 hr. Thereafter, dialysis was performed under the following conditions to remove excess tCAP reagent.
dialysis conditions:
dialysis tube: Spectra/Por(R)6Dialysis Membranes, MWCO8000 (Spectrum(R) Laboratories)
First dialysis: 0.1 M CH3COOH/CH3COONa pH 4 (300 mL), 4°C 2 hours Second dialysis: 0.1 M CH3COOH/CH3COONa pH 4 (300 mL), 4°C 2 hours
Third dialysis: 1xPBS pH:7.4 (300 mL), 4°C 2 hours
Fourth dialysis: 1xPBS pH:7.4 (300 mL), 4°C O/N

In the above-mentioned experiment, it was assumed that azidoLys-Gly-Gly was bivalently bound to R1CRD-Fcm by the tCAP reagent.

### (2) DBCO modification of anti-GPA33 antibody scFv fragment

The anti-GPA33 antibody scFv-Rspo fragments G17: GPA33_2R_C17_scFv-His (SEQ ID NO: 343; abbreviated as G17), G9: GPA33_2R_C9_scFv-His (SEQ ID NO: 344; abbreviated as G9), and 4D5_scFv-His (SEQ ID NO: 345; abbreviated as 4D5) were expressed in CHO cells as the host and purified at Biointron (Shanghai, China). The results of SDS-PAGE under reduced and non-reduced conditions are shown in Fig. 27 and Fig. 28. In both cases, dimers were present, which is considered to be due to dimer formation via Cys introduced near the C-terminus for DBCO modification. Thus, 3.95 µL of 500 mM TCEP-HCl (biovision, cat: 1202-10G, lot: 9D031202) was added to 3 mL of PBS solution of G17 (164 nM) such that the molar ratio was 4-fold (final concentration 656 µM), and the mixture was reduced at room temperature for 30 min. The analysis results by LC-MS (BioAccord, Waters) before and after the reduction are shown in the upper panel and middle panel of Fig. 29. Mass peaks observed after reduction were 27826, 28482, and 28774. The theoretical value calculated from the G17 sequence was 27846.93 (all in reduced state). However, assuming that the two Cys in VH and VL retain SS bonds and that the N-terminal Gln is pyroglutamylated by dehydration, the mass would decrease by 18+4=22, resulting in 27824.93, which is almost identical to the smallest mass peak. The observed 28482 and 28774 were determined to be due to the addition of glycans.

19.73 µL of 100 mM Bromoacetamido-PEG4-DBCO (QUANTA BIODESIGN, product no.: 11221) (final concentration 656 µM) was added to the reduced scFv fragment such that the molar ratio was 4-fold, and 1 M NaHCO₃ was added to a final concentration of 100 mM, and the reaction was performed for 1 hr. The results of the LC-MS analysis at that time are shown in the lower panel of Fig. 29. The mass peaks of 27826, 28482, and 28774 observed after reduction changed to 28389, 29046, and 29337, and an increase in mass of 563 or 564 was observed. Since the value of the expected increase in mass due to the modification of thiols with Bromoacetamido-PEG4-DBCO was almost consistent with 563.65, it was confirmed that the DBCO modification was almost complete. After that, dialysis was performed under the following conditions to remove the reagent.
dialysis conditions:
dialysis tube: Spectra/Por (registered trademark) 6Dialysis Membranes, MWCO8000 (Spectrum (registered trademark) Laboratories)
First dialysis: 1xPBS pH:7.4 (400 mL), 4°C 2 hours
Second dialysis: 1xPBS pH:7.4 (400 mL), 4°C 2 hours

The same experimental procedure as for G17 described above was also applied to G9. The results of LC-MS analysis before and after reduction are shown in the upper panel and middle panel of Fig. 30, and the results of LC-MS analysis after modification of the reduced scFv fragment with Bromoacetamido-PEG4-DBCO are shown in the lower panel of Fig. 30. From the results in the lower panel of Fig. 30, it was confirmed that DBCO modification was also almost complete for G9.

### (3) Linking reaction between R1CRD-Fcm and G17 or G9 by click reaction

To 1.81 mL of PBS solution of 53.9 µM azidized R1CRD-Fcm prepared in (1) was added 3.0 mL of PBS solution of 163.0 µM DBCO-G17 or G9 prepared in (2) such that the molar ratio was 5-fold, and they were mixed. After 24 hr, the reactivity was confirmed by SDS-PAGE under reduced conditions. The results are shown in Fig. 31. Regarding G17, the band of DBCO-G17 in lane 1 (about 25 kDa) was shifted to a band of about 100 kDa shown in lane 4 by linking with azidized R1CRD-Fcm (about 50 kDa) in lane 3 by click reaction. In lane 4, although a band corresponding to the original azidized R1CRD-Fcm was observed, it was determined that more than 70% had formed conjugates.Lanes 2 and 5 show, respectively, DBCO-4D5 which was used as a control scFv fragment, and the reaction product of its conjugate with azidized R1CRD-Fcm. Like G17, conjugates were found to be produced. Similar results were also obtained for G9 (lane 7: G9 scFV-His+DBCO, lane 8: R1CRD-hFcm-tCAP, lane 9: R1CRD-hFcm-G9 scFv dialysis). The conjugates obtained by the tCAP reaction (R1CRD-hFcm-G17 scFv, R1CRD-hFcm-G9 scFv) were used in the subsequent experiments.

Fig. 32 shows an outline of the creation of a conjugate between R1CRD-Fcm and DBCO-anti-GPA33 antibody scFv fragment by the tCAP method. First, in step 1), a divalent azide group is introduced into R1CRD-Fcm by using the tCAP reagent. Then, in step 2), the newly introduced Cys on the C-terminus side of the anti-GPA33 antibody scFv fragment is reduced, and the resulting thiol is modified with a DBCO-modification reagent to perform the DBCO-modification of the anti-GPA33 antibody scFv fragment. Finally, in step 3), the azidized R1CRD-Fcm and the DBCO-anti-GPA33 antibody scFv fragment are mixed, and a conjugate is created by a click reaction.

### [Example 20] Measurement of Wnt/β-catenin signal promoting activity of anti-GPA33 antibody scFv-Rspo fragment fusion

The Wnt/β-catenin signal promoting activity of the anti-GPA33 antibody scFv-Rspo fragment fusion recombinant prepared in Example 18 was measured as follows. ONE-Glo^{™} + Tox Luciferase Reporter and Cell Viability Assay (Promega) was used for the measurement. EGFP-human GPA33 expression vector (ORIGENE: RG210225) or MOG (Myelin Oligodendrocyte Glycoprotein) expression vector (ORIGENE: SC308258) was introduced into HEK293STF cells (ATCC: CRL-3249) that do not express GPA33 molecules by the electric pulse method using NEPA21 (NEPA GENE, 150V, 2.5 msec). The cells into which each vector was introduced were cultured for 2 days in DMEM medium containing 10% FBS (Nichirei), and then the cells were collected and seeded at 100 µL/well on a CulturPlate-96 (PerkinElmer). The next day, 100 µL of (1) control medium or (2) medium containing recombinant Rspo1FL-Fc or various anti-GPA33 antibody scFv-Rspo fragment fusion recombinant proteins prepared in Example 10 (10-fold serial dilution between 10000 and 0.001 ng/mL, final concentration 5000 to 0.005 ng/mL) was added to each well. Furthermore, Recombinant Afamin/Wnt3a (MBL) was added at that time to a final concentration of 883 ng/mL. At 18 hr after addition, 100 µL of the supernatant in the well was removed, and 20 µL of 5XCell Titer-Flour (trademark) Reagent (Promega) was added and stirred. After incubation at 37°C for 30 min, the mixture was stirred again and incubated at 37°C for 15 min. The fluorescence value was measured using a Microplate Reader SH-9000 (CORONA). 100 µL of ONE-Glo (trademark) Reagent (Promega) was added to the plate and incubated at room temperature for 3 min, after which the luminescence value was measured using a NIVO microplate reader (PerkinElmer). After calculating reporter activity as the luminescence/fluorescence value, a calibration curve (four-parameter logistic curve) was fitted using JMP Pro 16 to determine the 50% effective concentration: EC50 (nM) value. The results are shown in Table 6. The values in the column marked EC50 (GPA33) in the table are the EC50 values (nM) in 293STF cells expressing GPA33, and the column marked EC50 (MOG) shows the EC50 value (nM) in 293STF cells expressing MOG. Samples with low activity for which the EC50 value could not be determined were marked NC. EC50(MOG)/EC50(GPA33) when NC was included was calculated assuming NC to be>30 nM. Samples for which no measurement was performed are indicated as ND.

**[Table 6]**

| fusion name (SEQ ID NO) | EC50(GPA33) (nM) | EC50(MOG) (nM) | EC50(MOG)/ EC50(GPA33) |
|---|---|---|---|
| G17-R10RD-His(309) | 0.031 | NC | >967.7 |
| G9-R1CRD-His(310) | 0.008 | 25.9 | 3238 |
| R1CRD-hFcm(312) | 2.41 | 3.29 | 1.365 |
| R1CRD-GS3-Fcm(313) | ND | 3.24 | - |
| R4CRD-GS3-Fcm(314) | ND | 0.39 | - |
| G17-R4CRD-His(315) | 0.008 | 2.78 | 347.5 |
| G9-R4CRD-His(316) | 0.011 | 1.67 | 151.8 |
| R1CRD-G17-His(317) | 0.027 | 8.28 | 306.7 |
| R1CRD-G9-His(318) | 0.028 | 11.6 | 414.3 |
| G17-R1CRD-GS3-Fcm(319) | 0.054 | NC | >555.6 |
| G9-R1CRD-GS3-Fcm(320) | 0.04 | NC | >750 |
| G17-R2CRD-His(321) | 0.021 | 0.32 | 15.2 |
| G9-R4CRD-GS3-Fcm(323) | 0.016 | 0.58 | 36.3 |
| Rspo1FL-Fc | 0.95 | 0.96 | 1.011 |

The EC50 value of the anti-GPA33 antibody scFv-Rspo fragment fusion in 293STF cells expressing GPA33 was lower than that in 293STF cells expressing MOG, that is, it showed higher Wnt/β-catenin signal promoting activity. The ratio (EC50(MOG)/EC50(GPA33)) reached a maximum of 3000-fold or more. On the other hand, the EC50(MOG) and EC50(GPA33) values of Rspo1FL-Fc (containing wild-type R-spondin 1) and R1CRD-hFcm (containing the R-spondin1 active domain), which do not contain the anti-GPA33 antibody scFv fragment, were almost the same. Furthermore, a comparison of the EC50(GPA33) of G9-R1CRD-His with that of Rspo1FL-Fc shows that G9-R1CRD-His had 118.8 times higher Wnt/β-catenin signaling promotion activity. In addition, a comparison of the EC50(MOG) of the anti-GPA33 antibody scFv-Rspo (containing R1CRD) fragment fusion with that of Rspo1FL-Fc shows that the value of the anti-GPA33 antibody scFv-Rspo (containing R1CRD) fragment fusion was higher, namely, that the Wnt/β-catenin signaling promotion activity was low.

These results indicate that the anti-GPA33 antibody scFv-Rspo fragment fusion has high activity specific to cells expressing GPA33. The Wnt/β-catenin signal promoting activity to GPA33-expressing cells (intestinal tissue) is higher than that of wild-type R-spondin, while the activity to cells not expressing GPA33 (tissues other than the intestinal tissue) is lower than that of wild-type R-spondin. Such property is desirable as an agent for repairing and regenerating intestinal tissues.

### [Example 21] Measurement of Wnt/β-catenin signal promoting activity of anti-GPA33 antibody scFv-Rspo fragment-Fc conjugate

The Wnt/β-catenin signal promoting activity of the anti-GPA33 antibody scFv-Rspo fragment-Fc conjugate R1CRD-hFcm-tCAP-G17 prepared in Example 19 was measured as shown in Example 20. The results are shown in Table 7. The values in the column marked EC50(GPA33) in the table are the EC50 values (nM) in 293STF cells expressing GPA33, and the column marked EC50(MOG) shows the EC50 values (nM) in 293STF cells expressing MOG.

**[Table 7]**

| **fusion name (SEQ ID NO)** | EC50(GPA33) (nM) | EC50(MOG) (nM) | EC50(MOG)/ EC50(GPA33) |
|---|---|---|---|
| R1CRD-hFcm(312) | 2.41 | 3.29 | 1.365 |
| R1CRD-hFcm-tCAP-G17 | 0.085 | 4.04 | 47.53 |
| R1CRD-hFcm-tCAP-G9 | 0.13 | NC | >230.1 |
| Rspo1FL-Fc | 0.95 | 0.96 | 1.011 |

The EC50 value of R1CRD-hFcm-tCAP-G17 in 293STF cells expressing GPA33 was lower than the EC50 value in 293STF cells expressing MOG, indicating a higher Wnt/β-catenin signal promoting activity. On the other hand, the EC50(MOG) value and the EC50(GPA33) value of Rspo1FL-Fc (containing wild-type R-spondin 1) and R1CRD-hFcm (containing R-spondin 1 active domain), which do not contain anti-GPA33 antibody scFv fragment, were almost the same. Furthermore, a comparison of the EC50(GPA33) of R1CRD-hFcm-tCAP-G17 with that of Rspo1FL-Fc shows that R1CRD-hFcm-tCAP-G17 had higher Wnt/β-catenin signaling activity. Also, a comparison of the EC50(MOG) of R1CRD-hFcm-tCAP-G17 with that of Rspo1FL-Fc shows that R1CRD-hFcm-tCAP-G17 had lower Wnt/β-catenin signaling activity.

These results indicate that the anti-GPA33 antibody scFv-Rspo fragment-Fc conjugate has high activity specific to cells expressing GPA33. The Wnt/β-catenin signal promoting activity to GPA33-expressing cells (intestinal tissue) is higher than that of wild-type R-spondin, while the activity to cells not expressing GPA33 (tissues other than the intestinal tract) is lower than that of wild-type R-spondin. Such property is desirable as an agent for repairing and regenerating intestinal tissues.

### [Example 22] Mouse small intestinal organoid culture using anti-GPA33 antibody scFv-Rspo fragment fusion and anti-GPA33 antibody scFv-Rspo fragment-Fc conjugate

Mouse small intestinal organoid culture using anti-GPA33 antibody scFv-Rspo fragment fusion (Example 18) and anti-GPA33 antibody scFv-Rspo fragment-Fc conjugate (Example 19) was performed as shown below. Mouse small intestinal organoids (VERITAS ST-70931) were maintenance cultured in ENR medium obtained by adding 2 µg/mL of the recombinant Rspo1FL-Fc prepared in Example 10 to EN medium (DMEM/F-12 containing penicillin/streptomycin, 10 mM HEPES, GlutaMAX, 1xN2, 1xB27 (all Gibco), 1 mM N-acetylcysteine (FUJIFILM Wako Pure Chemical), 50 ng/mL mouse EGF (Gibco), and 100 ng/mL mouse Noggin (PEPROTECH)), or ENR+WA medium (DMEM/F-12 containing penicillin/streptomycin, 10 mM HEPES, GlutaMAX, 1xN2, 1xB27 (all Gibco), 2 µM N-acetylcysteine (FUJIFILM Wako Pure Chemical), 50 ng/mL mouse EGF (Gibco), and 100 ng/mL mouse Noggin (PEPROTECH), 10% Afamin/Wnt3a CM (MBL), and 500 nM A-83-01 (FUJIFILM Wako Pure Chemical)). Mouse small intestinal organoids were collected with Gentle Cell Dissociation Reagent (STEMCELL), resuspended in EN medium and Matrigel (Corning), and seeded onto 48-well plates. 250 µL of (1) EN medium, (2) EN medium containing recombinant Rspo1FL-Fc (20 ng/mL or 1 µg/mL) prepared in Example 10, or (3) EN medium containing anti-GPA33 antibody scFv-Rspo fragment fusion or anti-GPA33 antibody scFv-Rspo fragment-Fc conjugate (0.02 ng/mL, 0.08 ng/mL, 1.4 ng/mL, 20 ng/mL, 100 ng/ml, 200 ng/ml, or 1 µg/mL) was added to each well. The medium was changed once every three days, and the cells were cultured for six days.

The results are shown in Table 8. Comparing organoids on the first day (Day 1) and the fifth day (Day 5) after seeding, those in which clear proliferation was observed are indicated with ○, those in which proliferation was observed but to a weak extent are indicated with △, and those in which no proliferation was observed are indicated with ×. Typical micrographs with marks of ○, △, and × are shown in Fig. 33. Samples that were not evaluated are indicated with ND.

**[Table 8]**

| **anti-GPA33 antibody scFv-Rspo fragment fusion name (SEQ ID NO)** | 1000 (ng/ml) | 200 (ng/ml) | 20 (ng/ml) | 2 (ng/ml) | 1.4 (ng/ml) | 0.8 (ng/ml) | 0.2 (ng/ml) |
|---|---|---|---|---|---|---|---|
| G17-R1CRD-His(309) | ○ | ○ | ○ | ○ | ○ | ○ | × |
| G9-R1CRD-His(310) | ○ | ○ | ○ | ○ | ○ | Δ | × |
| 4D5-R1CRD-His (311) | ○ | Δ | × | ND | ND | ND | ND |
| Rspo1FL-Fc | ○ | ×* | × | ND | ND | ND | ND |
| G17-R4CRD-His(315) | ○ | ○ | ○ | ○ | ○ | ○ | × |
| G17-R2CRD-His(322) | ○ | ○ | ○ | ○ | ○ | ○ | × |
| 4D5-R2CRD-His(323) | ○ | ND | × | × | × | × | × |
| 4D5-R1CRD-GS3-Fcm(321) | Δ | × * | × | × | × | × | × |
| G17-R1CRD-GS3-Fcm(319) | ○ | ○* | ○ | × | × | × | × |
| G9-R1 CRD-GS3-Fcm(320) | ○ | ○* | ○ | × | × | × | × |

| **anti-GPA33 antibody scFv-Rspo fragment-Fc conjugate name (SEQ ID NO)** | 1000 (ng/ml) | 100 (ng/ml) | 20 (ng/ml) | 2 (ng/ml) | 1.4 (ng/ml) | 0.8 (ng/ml) | 0.2 (ng/ml) |
|---|---|---|---|---|---|---|---|
| R1CRD-hFcm(312) | ○ | × | × | × | × | × | × |
| R1CRD-hFcm-tGAP-G17 | ○ | ○ | Δ | × | × | × | × |

| | | | | | | | |
|---|---|---|---|---|---|---|---|
| *:100 ng/ml | | | | | | | |

It is shown that the anti-GPA33 antibody scFv-Rspo fragment fusion and the anti-GPA33 antibody scFv-Rspo fragment-Fc conjugate were shown to have organoid proliferation activity at lower concentrations compared to the control antibody scFv-Rspo fragment fusion, Rspo1FL-Fc (containing wild-type R-spondin 1), and R1CRD-hFcm (containing the R-spondin 1 activity domain).

### [Example 23] Therapeutic effect of anti-GPA33 antibody scFv-Rspo fragment fusion on dextran sulfate sodium (DSS)-induced inflammatory bowel disease (IBD) model

Using the recombinant proteins of anti-GPA33 antibody scFv-Rspo fragment fusion G9-R1CRD-His (SEQ ID NO: 310) and control antibody scFv-Rspo fragment fusion 4D5-R1CRD-His (SEQ ID NO: 311) prepared in Example 18, the therapeutic effect on dextran sulfate sodium (DSS)-induced inflammatory bowel disease (IBD) model was examined.

Seven-week-old female Balb/c mice (CLEA Japan, Inc.) were housed in a ventilated cage and acclimated to a 12-hr light-dark cycle for one week. The animals were fed 4% (w/v) DSS [MP Biomedicals, DEXTRAN SULFATE SODIUM SALT, COLITIS GRADE (36,000 - 50,000), USA] drinking solution ad libitum for 7 days (Day 0-7). On Day 7, the 4% (w/v) DSS drinking solution was replaced with 1% (w/v) DSS drinking solution. From Day 0 to Day 14, the body weight of each animal was recorded, and the degree of body weight loss, fecal consistency, and rectal bleeding were scored according to the criteria shown in Fig. 34 to calculate the Disease Activity Index (DAI). On Day 5, animals with matched and equivalent DAI scores were selected and divided into groups of 5 animals. The treatments in each group (n=5) were as follows.

Water group: Normal drinking water throughout the experiment, no administration.

Saline group: DSS (4% → 1%), 5 times from day 5 (Day 5) to day 9 (Day 9), equal volume (same amount as other groups) of physiological saline (saline) was injected into the tail vein.

G9 group: DSS (4% → 1%), 5 times from day 5 (Day 5) to day 9 (Day 9), 80.0 µg (4.0 mg/kg) of G9-R1CRD-His (SEQ ID NO: 310) dissolved in physiological saline was injected into the tail vein.

4D5 group: DSS (4% → 1%), 5 times from day 5 (Day 5) to day 9 (Day 9), 80.0 µg (4.0 mg/kg) of 4D5-R1CRD-His (SEQ ID NO: 311) dissolved in physiological saline was injected into the tail vein.

The average DAI score and body weight change of each animal are shown in Fig. 35 and Fig. 36, respectively.

On the 15th day (Day15), the animals were subjected to abdominal incision, blood was collected from the heart, and they were sacrificed. The stomach to anal region including the small intestine and large intestine was excised, and fixed in 10% neutral buffered formalin for histological analysis. Paraffin blocks were prepared for the small intestine (duodenum, jejunum, and ileum) and large intestine (colon and rectum) and thin-sectioned. Hematoxylin-eosin staining (H&E staining) was then performed for histopathological analysis of the intestinal mucosa. In addition, the degree of inflammation in the large intestine (rectum), the depth of injury (cell infiltration), and the damage to the crypts were scored according to the criteria in Fig. 37, with reference to J Immunol. 2012; 188: 6309-18. The histological scores for each item were calculated, and the sums were averaged for each group. The histological scores for each group are shown in Fig. 38. A typical micrograph of large intestinal tissue is shown in Fig. 39.

The DAI score of the G9 group was lower than that of the saline group on all days from day 6 onwards. It also tended to be lower than that of the 4D5 group (Fig. 35). Similarly, the G9 group showed the most remarkable degree of weight recovery in terms of the weight change rate (Fig. 36).

The histological score of the G9 group was clearly lower than that of the saline group. On the other hand, the difference between the 4D5 group and the saline group was not clear (Fig. 38). In Fig. 39, while significant inflammatory cell infiltration and crypt damage were observed in the saline group and 4D5 group, the G9 group showed a healthy intestinal epithelial tissue image similar to that of the water group.

The above results indicate that the anti-GPA33 antibody scFv-Rspo fragment fusion has the effect of alleviating the symptoms of the DSS-induced inflammatory bowel disease model, and that the high therapeutic effect thereof depends on the addition of the anti-GPA33 antibody scFv fragment. That is, it was shown that the anti-GPA33 antibody scFv-Rspo fragment fusion has high efficacy and can be a therapeutic agent for inflammatory bowel disease with little risk of side effects.

### [Example 24] Preparation of human mesenchymal stem cells expressing anti-GPA33 antibody scFv-Fc on the membrane surface

Mesenchymal stem cell (MSC) with immune control ability is expected to be a cell medicine, especially for the treatment of inflammatory bowel disease, and many clinical trials have been conducted. On the other hand, the therapeutic effect is often insufficient, which is attributed to the low efficiency of MSC delivery to the damaged intestinal mucosa (Ko et al. 2021, DOI: 10.3390/biom11010082). To solve this problem, attempts have been made to present tissue-specific antibodies on the cell membrane surface as a method for accumulating MSC in target tissues and enhancing therapeutic effects (Ko et al. 2010, DOI: 10.1038/mt.2010.54; Golinelli et al. 2020, DOI: 10.1038/s41417-018-0062-x; Komarova et al. 2010, DOI: 10.1186/1757-2215-3-12). That is, it is expected that the presentation of the anti-GPA33 antibody of the present invention on the MSC cell surface will improve the delivery efficiency to the intestinal mucosa and provide a higher therapeutic effect against inflammatory bowel disease. To express the anti-GPA33scfv fragment on the cell membrane surface, a fusion protein was designed in which the anti-GPA33scfv fragment (G9 or G17) was linked to the N-terminus of the wild-type human IgG1-derived Fc fragment (Fcwt), mutant human IgG1-derived Fc fragment (Fcm), or mouse IgG2a-derived Fc fragment (mG2aFc) and the PDGF receptor membrane-binding domain (PDGFTM, SEQ ID NO: 352) was linked to the C-terminus, and the DNA sequence was synthesized by Thermo Scientific. The sequence numbers of each protein and fragment are shown in Table 9.

**[Table 9]**

| **fusion protein name (SEQ ID NO)** | **scFv fragment name (SEQ ID NO)** | **Fc fragment (SEQ ID NO)** |
|---|---|---|
| G17-mG2aFc-PDGFTM(346) | G17(330) | mG2aFc(350) |
| G17-Fcwt-PDGFTM(347) | G17(330) | Fcwt(351) |
| G9-Fcm-PDGFTM(348) | G9(331) | Fcm(350) |
| 4D5-Fcm-PDGFTM(349) | 4D5(332) | Fcm(350) |

The human IL-2 signal sequence (SEQ ID NO: 353) was used as the signal sequence for expression on the membrane surface. Reversibly immortalized MSCs (WO2022097716), into which an immortalization gene was introduced using a chromosomally non-integrating RNA viral vector, were used as gene recipient cells. For gene transfer into MSCs, mRNA was used as reported by Li et al. (2021, DOI: 10.1016/j.omtn.2021.07.009). G17-mG2aFc-PDGFTM mRNA (1 µg) was synthesized using the IVTpro (trademark) mRNA Synthesis Kit (Takara) and introduced into the MSCs by the electric pulse method according to the method reported by Li et al. (2021). At 48 hr after G17-mG2aFc-PDGFTM mRNA introduction, the gene-transfected MSCs were stained with a labeled anti-mIgG2a antibody (Biolegend, Alexa Fluor 647 anti-mouse IgG2a Antibody, 407116) and FACS analysis was performed. As a result, G17-mG2aFc was detected on the surface of most MSCs (Fig. 40).

### [Industrial Applicability]

The present invention enables drug delivery targeting intestinal tissue, and can realize treatment of intestinal disease with high therapeutic effect and reduced side effects. Therefore, the present invention is extremely useful in the medical field and the drug discovery field.

This application is based on a patent application No. 2022-186944 filed in Japan (filing date: November 22, 2022), the contents of which are incorporated in full herein.

## Claims

1. A human antibody or an antigen-binding portion thereof that specifically binds to human GPA33 and mouse GPA33.

2. The antibody or an antigen-binding portion thereof according to claim 1, which comprises a light chain variable region and a heavy chain variable region, wherein
(1) the light chain variable region comprises CDR1 comprising the amino acid sequence shown in SEQ ID NO: 1, CDR2 comprising the amino acid sequence alanine-alanine-serine, and the amino acid sequence shown in SEQ ID NO: 2, and the heavy chain variable region comprises CDR1 comprising the amino acid sequence shown in SEQ ID NO: 3, CDR2 comprising the amino acid sequence shown in SEQ ID NO: 4, and CDR3 comprising the amino acid sequence shown in SEQ ID NO: 5;
(2) the light chain variable region comprises CDR1 comprising the amino acid sequence shown in SEQ ID NO: 6, CDR2 comprising the amino acid sequence valine-alanine-serine, and the amino acid sequence shown in SEQ ID NO: 7, and the heavy chain variable region comprises CDR1 comprising the amino acid sequence shown in SEQ ID NO: 8, CDR2 comprising the amino acid sequence shown in SEQ ID NO: 4, and CDR3 comprising the amino acid sequence shown in SEQ ID NO: 5; or,
(3) the light chain variable region comprises CDR1 comprising the amino acid sequence shown in SEQ ID NO: 1, CDR2 comprising the amino acid sequence isoleucine-alanine-serine, and the amino acid sequence shown in SEQ ID NO: 9, and the heavy chain variable region comprises CDR1 comprising the amino acid sequence shown in SEQ ID NO: 10, CDR2 comprising the amino acid sequence shown in SEQ ID NO: 11, and CDR3 comprising the amino acid sequence shown in SEQ ID NO: 12.

3. The antibody or an antigen-binding portion thereof according to claim 1, which comprises a light chain variable region and a heavy chain variable region, wherein
(4) the light chain variable region comprises the amino acid sequence shown in SEQ ID NO: 13, and the heavy chain variable region comprises the amino acid sequence shown in SEQ ID NO: 14;
(5) the light chain variable region comprises the amino acid sequence shown in SEQ ID NO: 15, and the heavy chain variable region comprises the amino acid sequence shown in SEQ ID NO: 16; or
(6) the light chain variable region comprises the amino acid sequence shown in SEQ ID NO: 17, and the heavy chain variable region comprises the amino acid sequence shown in SEQ ID NO: 18.

4. The antibody or an antigen-binding portion thereof according to claim 1, wherein the heavy chain variable region (VH) is selected from the group consisting of the following (a1) to (a38):
(a1) VH in which the amino acid sequences of CDR1 to CDR3 respectively comprise the amino acid sequences shown in SEQ ID NOs: 8, 197 and 217
(a2) VH in which the amino acid sequences of CDR1 to CDR3 respectively comprise the amino acid sequences shown in SEQ ID NOs: 180, 198 and 218
(a3) VH in which the amino acid sequences of CDR1 to CDR3 respectively comprise the amino acid sequences shown in SEQ ID NOs: 3, 199 and 217
(a4) VH in which the amino acid sequences of CDR1 to CDR3 respectively comprise the amino acid sequences shown in SEQ ID NOs: 3, 200 and 219
(a5) VH in which the amino acid sequences of CDR1 to CDR3 respectively comprise the amino acid sequences shown in SEQ ID NOs: 181, 200 and 219
(a6) VH in which the amino acid sequences of CDR1 to CDR3 respectively comprise the amino acid sequences shown in SEQ ID NOs: 8, 199 and 5
(a7) VH in which the amino acid sequences of CDR1 to CDR3 respectively comprise the amino acid sequences shown in SEQ ID NOs: 3, 201 and 217
(a8) VH in which the amino acid sequences of CDR1 to CDR3 respectively comprise the amino acid sequences shown in SEQ ID NOs: 8, 199 and 217
(a9) VH in which the amino acid sequences of CDR1 to CDR3 respectively comprise the amino acid sequences shown in SEQ ID NOs: 181, 200 and 5
(a10) VH in which the amino acid sequences of CDR1 to CDR3 respectively comprise the amino acid sequences shown in SEQ ID NOs: 182, 202 and 220
(a11) VH in which the amino acid sequences of CDR1 to CDR3 respectively comprise the amino acid sequences shown in SEQ ID NOs: 183, 203 and 221
(a12) VH in which the amino acid sequences of CDR1 to CDR3 respectively comprise the amino acid sequences shown in SEQ ID NOs: 8, 4 and 5
(a13) VH in which the amino acid sequences of CDR1 to CDR3 respectively comprise the amino acid sequences shown in SEQ ID NOs: 3, 4 and 219
(a14) VH in which the amino acid sequences of CDR1 to CDR3 respectively comprise the amino acid sequences shown in SEQ ID NOs: 181, 204 and 222
(a15) VH in which the amino acid sequences of CDR1 to CDR3 respectively comprise the amino acid sequences shown in SEQ ID NOs: 181, 204 and 223
(a16) VH in which the amino acid sequences of CDR1 to CDR3 respectively comprise the amino acid sequences shown in SEQ ID NOs: 183, 203 and 224
(a17) VH in which the amino acid sequences of CDR1 to CDR3 respectively comprise the amino acid sequences shown in SEQ ID NOs: 184, 205 and 225
(a18) VH in which the amino acid sequences of CDR1 to CDR3 respectively comprise the amino acid sequences shown in SEQ ID NOs: 185, 206 and 226
(a19) VH in which the amino acid sequences of CDR1 to CDR3 respectively comprise the amino acid sequences shown in SEQ ID NOs: 183, 203 and 227
(a20) VH in which the amino acid sequences of CDR1 to CDR3 respectively comprise the amino acid sequences shown in SEQ ID NOs: 186, 207 and 228
(a21) VH in which the amino acid sequences of CDR1 to CDR3 respectively comprise the amino acid sequences shown in SEQ ID NOs: 187, 208 and 229
(a22) VH in which the amino acid sequences of CDR1 to CDR3 respectively comprise the amino acid sequences shown in SEQ ID NOs: 184, 209 and 230
(a23) VH in which the amino acid sequences of CDR1 to CDR3 respectively comprise the amino acid sequences shown in SEQ ID NOs: 3, 4 and 5
(a24) VH in which the amino acid sequences of CDR1 to CDR3 respectively comprise the amino acid sequences shown in SEQ ID NOs: 8, 4 and 231
(a25) VH in which the amino acid sequences of CDR1 to CDR3 respectively comprise the amino acid sequences shown in SEQ ID NOs: 188, 4 and 5
(a26) VH in which the amino acid sequences of CDR1 to CDR3 respectively comprise the amino acid sequences shown in SEQ ID NOs: 189, 210 and 232
(a27) VH in which the amino acid sequences of CDR1 to CDR3 respectively comprise the amino acid sequences shown in SEQ ID NOs: 8, 211 and 5
(a28) VH in which the amino acid sequences of CDR1 to CDR3 respectively comprise the amino acid sequences shown in SEQ ID NOs: 190, 202 and 233
(a29) VH in which the amino acid sequences of CDR1 to CDR3 respectively comprise the amino acid sequences shown in SEQ ID NOs: 184, 205 and 234
(a30) VH in which the amino acid sequences of CDR1 to CDR3 respectively comprise the amino acid sequences shown in SEQ ID NOs: 182, 203 and 235
(a31) VH in which the amino acid sequences of CDR1 to CDR3 respectively comprise the amino acid sequences shown in SEQ ID NOs: 191, 212 and 236
(a32) VH in which the amino acid sequences of CDR1 to CDR3 respectively comprise the amino acid sequences shown in SEQ ID NOs: 192, 208 and 229
(a33) VH in which the amino acid sequences of CDR1 to CDR3 respectively comprise the amino acid sequences shown in SEQ ID NOs: 184, 205 and 237
(a34) VH in which the amino acid sequences of CDR1 to CDR3 respectively comprise the amino acid sequences shown in SEQ ID NOs: 193, 213 and 238
(a35) VH in which the amino acid sequences of CDR1 to CDR3 respectively comprise the amino acid sequences shown in SEQ ID NOs: 194, 214 and 239
(a36) VH in which the amino acid sequences of CDR1 to CDR3 respectively comprise the amino acid sequences shown in SEQ ID NOs: 195, 215 and 240
(a37) VH in which the amino acid sequences of CDR1 to CDR3 respectively comprise the amino acid sequences shown in SEQ ID NOs: 196, 216 and 241
(a38) VH in which the amino acid sequences of CDR1 to CDR3 respectively comprise the amino acid sequences shown in SEQ ID NOs: 10, 11 and 12.

5. The antigen-binding portion according to claim 4, which is a VHH.

6. A conjugate of the antibody or an antigen-binding portion thereof according to any one of claims 1 to 5 and a functional molecule.

7. The conjugate according to claim 6, wherein the functional molecule is a drug for examination or for treating diseases.

8. The conjugate according to claim 7, wherein the drug is fused to the antibody or an antigen-binding portion thereof, or bound to a constant region thereof.

9. The conjugate according to claim 8, wherein the drug is R-spondin 1, 2, 3 or 4, or a partial fragment thereof.

10. An intestinal tissue targeting agent comprising the antibody or an antigen-binding portion thereof according to any one of claims 1 to 5.

11. An agent for examining an intestinal tissue or treating a bowel disease, comprising the conjugate according to claim 6.

12. A polynucleotide encoding the antibody or an antigen-binding portion thereof according to any one of claims 1 to 5.

13. A vector comprising the polynucleotide according to claim 11.

14. A transformant comprising the vector according to claim 12.

15. The transformant according to claim 14, which is a mammalian cell that expresses the antibody or an antigen-binding portion thereof according to any one of claims 1 to 5 on a cell surface thereof.

16. An agent for promoting regeneration of and/or repairing an intestinal tissue, comprising the transformant according to claim 15.
